# EUROPEAN PATENT APPLICATION

(11) **EP 4 578 417 A1**
(43) Date of publication of application: **02.07.2025**
(21) Application number: 24223144.7
(22) Date of filing: 24.12.2024
(51) Int. Cl.: A61B 18/14, A61B 5/287, A61B 18/00, A61B 34/20

(54) **ENCAPSULATED CATHETERS**

(30) Priority: 28.12.2023 US 202363615534 P; 28.12.2023 US 202363615556 P; 28.12.2023 US 202363615574 P; 28.12.2023 US 202363615600 P; 29.12.2023 US 202363615902 P; 29.12.2023 US 202363615947 P; 07.11.2024 US 202418939766
(71) Applicant: Biosense Webster (Israel) Ltd., 2066717 Yokneam (IL)
(72) Inventor: EBRAHIMI, Babak, Irvine, 92618 (US); RODRIGUEZ SOTO, Juan, Irvine, 92618 (US); VAN NIEKERK, Pieter Emmelius, Irvine, 92618 (US); BASU, Shubhayu, Irvine, 92618 (US); ABBAS, Mohammad, Irvine, 92618 (US); GOO, Audrey Claire, Irvine, 92618 (US); PATEL, Amar, Irvine, 92618 (US); GUTA, Alexandru, Irvine, 92618 (US)
(74) Representative: Carpmaels & Ransford LLP

(57) **Abstract**

An end effector for a catheter including a flexible circuit including a plurality of electrodes is herein disclosed. The flexible circuit is positioned at least partially within an insulative material. In some examples, the end effector includes a framework spaced from the flexible circuit and one or more position sensing loops.

## Description

### CROSS-REFERENCE TO RELATED APPLICATIONS

The application claims benefit of priority to prior filed U.S. Provisional Patent Application No. 63/615,600 filed December 28, 2023 (Attorney Docket No.: BI06917USPSP1 - 253757.000438), prior filed U.S. Provisional Patent Application No. 63/615,902 filed December 29, 2023 (Attorney Docket No. BIO6926USPSP1-253757.000471), prior filed U.S. Provisional Patent Application No. 63/615,534 filed December 28, 2023 (Attorney Docket No. BIO6925USPSP1 -- 253757.000472), prior filed U.S. Provisional Patent Application No. 63/615,556 filed December 28, 2023 (Attorney Docket No. BIO6924USPSP1 -- 253757.000473), prior filed U.S. Provisional Patent Application No. 63/615,574 filed December 28, 2023 (Attorney Docket No. BIO6923USPSP1 -- 253757.000474), and prior filed U.S. Provisional Patent Application No. 63/615,947 filed December 29, 2023 (Attorney Docket No. BIO6922USPSP1-253757.000475), all of which are hereby incorporated by reference as if set forth in full herein.

### FIELD

The present disclosure relates generally to minimally invasive medical devices, and in particular cardiac mapping catheters with flexible end effectors.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Many times, electrode contact surfaces of these end effectors must be exposed through an encapsulating material via laser cutting or mechanical removal, both of which increase labor time and production costs. Additionally, the location of these catheters as they travel through the heart are not easily tracked using conventional methods. Moreover, the design of the stiff internal structural members have many shortcomings, such as easy breakage of flexible circuit interconnections and unpredictable collapse patterns. Accordingly, there is a need for an improved end effector of a catheter that addresses these problems.

Moreover, for prevention of unwanted damage to regions surrounding the target anatomy, it may be desirable to provide an end effector with as small as footprint as possible. It may also be desirable to provide a large number of electrodes on the end effector to collect larger amounts of data signals and/or make the most use of the available surface area provided by the end effector. However, a larger number of electrodes increases the number of corresponding electrical traces required for accurate transmission of electrical signals. Providing a large number of electrical traces may be difficult given the small footprint of the end effector. Accordingly, there is a need for an improved end effector of a catheter that addresses these problems.

### SUMMARY

The following disclosure presents solutions to the foregoing problems.

To enable the time- and cost-efficient manufacture of end effectors that enhance the function of mapping and ablation catheters mentioned in the background section, the present disclosure relates to easily manufacturable end effectors having enhanced aspects of mapping and ablation catheter performance, including, but not limited to: mapping resolution, electrode contact with the target anatomy, delivery of the end effector to the target anatomy, biocompatibility, end effector stiffness, and atraumaticity.

There is provided, in accordance with the disclosed technology, an end effector. The end effector can include an insulative material having a first outer surface and a second outer surface. The end effector can include a framework disposed in the insulative material. The end effector can include a first flexible circuit comprising a first plurality of electrodes. The first flexible circuit is disposed longitudinally along and at least partially within the insulative material and being positioned such that a first portion of the first flexible circuit is contiguous to a first plane and a second portion of the first flexible circuit is contiguous to a second plane. The first plane is a first distance from the framework and the second plane is a second distance from the framework. The second distance is less than the first distance.

There is provided, in accordance with the disclosed technology, an end effector. The end effector can include an insulative material having a first outer surface and a second outer surface. The end effector can include a framework disposed in the insulative material. The end effector can include a first flexible circuit comprising a first plurality of electrodes. The first flexible circuit is disposed on or within the insulative material and each electrode of the first plurality of electrodes includes a first contact surface. The insulative material is contiguous to the first contact surfaces so that only the first contact surfaces of at least a portion of the first plurality of electrodes are exposed to an ambient environment. A first support layer is within the insulative material between the framework and the first flexible circuit.

There is provided, in accordance with the disclosed technology, an end effector. The end effector can include an insulative material having a first outer surface and a second outer surface. The end effector can include a framework disposed in the insulative material. The end effector can include a first flexible circuit having a corrugate profile disposed in the insulative material. The first flexible circuit includes a first portion contiguous to the insulative material and exposed to ambient environment. The first flexible circuit is spaced apart from the framework.

There is provided, in accordance with the disclosed technology, an end effector for a catheter is provided, including a first flexible circuit extending along a longitudinal axis from a proximal portion to a distal portion of the end effector, and a second flexible circuit extending along the longitudinal axis from the proximal portion to the distal portion of the end effector, the second flexible circuit being substantially coplanar to the first flexible circuit in the distal portion of the end effector and disposed on top of the first flexible circuit in the proximal portion of the end effector. The first flexible circuit can include one or more first electrodes disposed on a distal portion of the first flexible circuit. A proximal portion of the first flexible circuit can comprise one or more first electrical contacts corresponding to the one or more first electrodes. The first flexible circuit can comprise one or more first traces connecting the one or more first electrical contacts to the one or more first electrodes. The one or more first electrodes can comprise a plurality of pairs of first electrodes disposed on the distal portion of the first flexible circuit. Each first electrode of each pair of first electrodes can be spaced apart a first predetermined longitudinal distance and each pair of first electrodes can be spaced apart from adjacent pairs of first electrodes a second predetermined longitudinal distance, the second predetermined longitudinal distance can greater than the first predetermined longitudinal distance. The first predetermined longitudinal distance at which electrodes of a pair are spaced apart can be about 100 microns. Each of the electrodes can comprise a length of about 500 microns and a width of approximately 500 microns. The second flexible circuit can include one or more second electrodes disposed on a distal portion of the second flexible circuit. A proximal portion of the second flexible circuit can include one or more second electrical contacts corresponding to the one or more second electrodes. The second flexible circuit can comprise one or more second traces connecting the one or more second electrical contacts to the one or more second electrodes. The one or more second electrodes can comprise a plurality of pairs of second electrodes disposed on the distal portion of the second flexible circuit. It is noted that the design provided herein overcomes many shortcomings of existing mapping catheters. Specifically, commercially available ECG electrodes in mapping or diagnostic catheters are usually cylindrical shape with partial contact with tissue and partial contact with the blood. While this allows the collection of the ECG signal through the myocardial tissue, other electrical signals (e.g., far-field signals) propagating through the blood and tissue would be collected with traditional cylindrical electrodes. Consequently, flat electrodes on opposite sides with one electrode in contact with tissues with the other flat electrodes in contact with blood (but not tissues) allows for cancellation of the far-field signals. In other words, one of the biggest advantages of this new design is the concept of reference electrode (i.e., the electrode not in physical contact with tissues) which is very critical for the unipolar signal concept. Traditionally, the reference signal for unipolar signal mapping has been WCT, or a single electrode with no tissue contact at one location away from the tissue contacting electrode(s). Thus, the cylindrical electrodes and the single discrete reference electrode have their own deficiencies since the reference signal location is different for different electrodes. These shortcomings are addressed with the new designs provided here where there is a dedicated reference (non-tissue contacting) electrode for each tissue contacting electrode at virtually the same location.

The end effector can further include a frame, and the distal portion of the first flexible circuit can disposed on the frame and the distal portion of the second flexible circuit can be disposed on the frame, and a proximal portion of the first flexible circuit can be disposed on the frame and a proximal portion of the second flexible circuit can disposed on a portion of the proximal portion of the first flexible circuit. The frame can comprise a first, a second, a third, and a fourth spine. The distal portion of the first flexible circuit can include a first loop disposed over the first spine and the second spine; and the distal portion of the second flexible circuit comprising a second loop disposed over the third spine and the fourth spine. The frame can further include at least a partial gap between the first, the second, the third, and the fourth spines. The frame can comprise nitinol. The distal portion of the end effector can comprise a width of about 9 millimeters and a length of about 20 millimeters.

There is provided, in accordance with the disclosed technology, an end effector for a catheter including a frame comprising extending along a longitudinal axis, the frame having a first side and a second side; a first flexible circuit disposed on the first side of the frame and extending along the longitudinal axis from a proximal portion to a distal portion of the frame, and a second flexible circuit extending along the longitudinal axis from the proximal portion to the distal portion of the frame, the second flexible circuit disposed on the first side of the frame at the distal portion of the frame and disposed on top of the first flexible circuit at the proximal portion of the frame. The first flexible circuit can include one or more first electrodes disposed on a distal portion of the first flexible circuit, and the second flexible circuit comprises one or more second electrodes disposed on a distal portion of the second flexible circuit. The first electrodes and the second electrodes can be arranged in pairs of electrodes. Each electrode of a pair of electrodes can be spaced apart a first predetermined longitudinal distance and each pair of electrodes can be spaced apart from adjacent pairs of electrodes a second predetermined longitudinal distance. The second predetermined longitudinal distance can be greater than the first predetermined longitudinal distance. The first predetermined longitudinal distance corresponding to the distance between electrodes of a pair can be about 100 microns.

The end effector can further include a third flexible circuit disposed on the second side of the frame and extending along the longitudinal axis from the proximal portion to the distal portion of the frame, and a fourth flexible circuit extending along the longitudinal axis from the proximal portion to the distal portion of the frame, the fourth flexible circuit can be disposed on the second side of the frame at the distal portion of the frame and disposed on top of the third flexible circuit at the proximal portion of the frame. The third flexible circuit can include disposed on a distal portion of the third flexible circuit, and the fourth flexible circuit can include electrodes disposed on a distal portion of the second flexible circuit.

There is provided, in accordance with the disclosed technology, an end effector for a catheter including a first flexible circuit extending along a longitudinal axis from a proximal portion to a distal portion of the end effector, and a second flexible circuit extending along the longitudinal axis from the proximal portion to the distal portion of the end effector, the second flexible circuit being substantially coplanar to the first flexible circuit in the distal portion of the end effector and disposed on top of the first flexible circuit in the proximal portion of the end effector. The first flexible circuit can include a first set of electrodes disposed on one side of the first flexible circuit and a second set of electrodes on an opposite side of the first flexible circuit, the second flexible circuit including a first set of electrodes disposed on one side of the second flexible circuit and a second set of electrodes disposed on an opposite side of the second flexible circuit. The end effector can further include a substrate disposed between the first and second flexible circuits at the proximal portion and coplanar with the first and second flexible circuits at the distal portion.

There is provided, in accordance with the disclosed technology, an end effector for a catheter. The end effector comprises an insulative material, a framework, and location sensing loops. The framework is disposed in the insulative material and is substantially planar along a longitudinal axis. The location sensing loops are spaced apart from the framework and are coupled to the insulative material. The location sensing loops comprise a center loop and a pair of side loops. The center loop is disposed over the longitudinal axis over an area near a distal portion of the insulative material. The pair of side loops are disposed generally symmetrical about the longitudinal axis, with each side loop extending from a proximal portion to a distal portion of the insulative material along the longitudinal axis.

There is provided, in accordance with the disclosed technology, an end effector for a catheter. The end effector comprises an insulative material, a framework, and location sensing loops. The framework is disposed in the insulative material and is substantially planar along a longitudinal axis. The location sensing loops are disposed generally parallel to the framework and are separated from the framework by the insulative material. The location sensing loops comprise a center loop, a first side loop, and a second side loop. The center loop extends along the longitudinal axis and comprises a cumulative center loop surface area. The first side loop extends along the longitudinal axis and comprises a first cumulative side loop surface area. The second side loop extends along the longitudinal axis and comprises a cumulative second side loop surface area. The cumulative center loop surface area, the cumulative first side loop surface area, and the cumulative second side loop surface area each are within a range of about one-hundred to three-hundred millimeters squared.

There is provided, in accordance with the disclosed technology, a framework for an end effector of a medical device. The framework comprises a base and a first spine loop. The base is configured to connect with an elongated shaft of the medical device and extends along a longitudinal axis towards in a distal direction. The first spine loop extends from the base along the longitudinal axis and comprises first through seventh segments. The curvilinear first segment is connected to the base and extends therefrom in a distal direction along the longitudinal axis. The second segment has an arcuate configuration connected to the curvilinear first segment and extends therefrom. The third segment is connected to the second segment and extends therefrom in a proximal direction along the longitudinal axis. The curvilinear fourth segment is connected to the base and extends therefrom in the distal direction along the longitudinal axis. The arcuate fifth segment is connected to the fourth segment and extends therefrom. The sixth segment is connected to the fifth segment and extends therefrom in the proximal direction along the longitudinal axis. The arcuate seventh segment connects the third segment and the sixth segment.

There is further provided, in accordance with the disclosed technology, another framework for an end effector of a medical device. The framework comprises a base, a first spine loop, a second spine loop, a third spine loop, and a finger shaped opening. The base is configured to connect with an elongated shaft of the medical device and extends along a longitudinal axis. The first spine loop extends from the base along the longitudinal axis and comprises a first segment, a second segment, and a third segment. The first segment is connected to the base and extends therefrom in a distal direction along the longitudinal axis. The second segment is connected to the first segment and extends therefrom inwardly towards the longitudinal axis. The third segment is connected to the second segment and extends therefrom in the distal direction along the longitudinal axis. The second spine loop extends from the base along the longitudinal axis and comprises a first segment, a second segment, and a third segment. The first segment is connected to the base and extends therefrom in the distal direction along the longitudinal axis. The second segment is connected to the first segment and extends therefrom inwardly towards the longitudinal axis. The third segment is connected to the second segment and extends therefrom in the distal direction along the longitudinal axis. The third spine loop connects the third segment of the first spine loop and the third segment of the second spine loop. The finger shaped opening is defined by the base, the first spine loop, the second spine loop, and the third spine loop.

There is further provided, in accordance with the disclosed technology, an end effector for a medical device. The end effector comprises a framework, an insulative material disposed on the framework, and a flexible circuit. The framework comprises a base, a first spine loop, a second spine loop, a third spine loop, and a finger shaped opening. The base is configured to connect with an elongated shaft of the medical device and extends along a longitudinal axis. The first spine loop extends from the base along the longitudinal axis. The second spine loop extends from the base along the longitudinal axis. The third spine loop connects the first spine loop and the second spine loop. The finger shaped opening is defined by the base, the first spine loop, the second spine loop, and the third spine loop. The flexible circuit comprises a first section and a second section. The first section is vertically spaced from the framework along a vertical axis by the insulative material. The second section is offset from the first section along the vertical axis and extending within the finger shaped opening.

There is further provided, in accordance with the disclosed technology, yet another framework for an end effector of a medical device. The framework extends along a longitudinal axis that is coaxial with a longitudinal center of the framework and comprises a base, a first spine loop, and a second spine loop. The base is configured to connect with an elongated shaft of the medical device and extends along the longitudinal axis. The first spine loop extends from the base on a first side of the longitudinal axis and comprises a first distal end and a second distal end. The first distal end is connected to the base at a first longitudinal location along the longitudinal axis. The second distal end is connected to the base at a second longitudinal location along the longitudinal axis. The second spine loop extends from the base on a second side of the longitudinal axis. The second spine loop comprises a first distal end and a second distal end. The first distal end is connected to the base at a third longitudinal location along the longitudinal axis. The second distal end is connected to the base at a fourth longitudinal location along the longitudinal axis. The first longitudinal location, the second longitudinal location, the third longitudinal location, and the fourth longitudinal location are respectively disposed along the longitudinal axis such that the framework is asymmetric relative to the longitudinal axis.

There is further provided, in accordance with the disclosed technology, an end effector for a medical device. The end effector comprises a framework that is substantially planar along a longitudinal axis, a flexible circuit vertically spaced from the framework along a vertical axis, and an insulative material within which the framework and the flexible circuit are disposed. The insulative material defines a first outer edge of the end effector. The first outer edge comprises a treatment configured to facilitate collapsing of the end effector into a sheath. The treatment comprises at least one of: a rounded cut extending along at least a portion of the first outer edge, a tapered cut extending along at least a portion of the first outer edge, a plurality of incised cuts defined along at least a portion of the first outer edge, or a lubricious coating extending along at least a portion of the first outer edge.

There is further provided, in accordance with the disclosed technology, a method of using a medical device. The method comprises retracting, from a deployed configuration, an end effector into a sheath along a longitudinal axis, the end effector comprising a substantially planar shape in the deployed configuration. The method comprises collapsing the end effector into a retracted configuration such that outer edges of the end effector slip past one another. The end effector comprises one of a substantially cylindrical shape or a spiral shape in the retracted configuration. The first outer edge of the outer edges comprises a treatment comprising at least one of: a rounded cut extending along at least a portion of the first outer edge, a tapered cut extending along at least a portion of the first outer edge, a plurality of incised cuts defined along at least a portion of the first outer edge, or a lubricious coating extending along at least a portion of the first outer edge.

There is further provided, in accordance with the disclosed technology, a method of manufacturing an end effector for a medical device. The method comprises forming a framework that is substantially planar along a longitudinal axis. The method comprises disposing a flexible circuit over the framework. The method comprises heating an insulative material. The method comprises reflowing the insulative material such that the insulative material envelopes the framework and the flexible circuit. The method comprises treating an outer edge of the insulative material such that the outer edge comprises at least one of a reduced stiffness or a reduced coefficient of friction relative to a coefficient of friction or a stiffness of the outer edge prior to the treatment.

To enable the time- and cost-efficient manufacture of end effectors that enhance the function of mapping and ablation catheters mentioned in the background section, the present disclosure also relates to easily manufacturable end effectors having enhanced aspects of mapping and ablation catheter performance, including, but not limited to: mapping resolution, electrode contact with the target anatomy, delivery of the end effector to the target anatomy, biocompatibility, end effector stiffness, and atraumaticity.

There is provided, in accordance with the disclosed technology, an end effector for medical instrument. The end effector includes a framework defining a neutral plane. The end effector includes a first insulative material disposed on at least one side of the framework. The end effector includes a first flexible circuit disposed in the first insulative material and spaced apart from the framework along a vertical axis orthogonal to the neutral plane. The first flexible circuit includes a planar transition zone located proximate a proximal end of the first flexible circuit. The first flexible circuit transitions in the planar transition zone from a first plane closer to the neutral plane to a second plane farther from the neutral plane.

There is provided, in accordance with the disclosed technology, an end effector. The end effector includes a framework having a first strut extending along a longitudinal axis. The end effector includes a flexible circuit aligned with the first strut and comprising a substrate supporting an electrical trace, the flexible circuit comprising a plurality of bends such that the flexible circuit comprises a serpentine shape as it extends along the longitudinal axis of the strut. The first strut of the framework includes alternating wide sections and narrow sections extending along the longitudinal axis of the strut. The narrow sections can be positioned proximate respective apexes of the plurality of bends.

There is provided, in accordance with the disclosed technology, an end effector. The end effector includes a framework including a first strut extending along a longitudinal axis. The end effector includes a flexible circuit aligned with the first strut and including a substrate supporting an electrical trace. The flexible circuit can include a plurality of bends such that the flexible circuit has a serpentine shape as it extends along the longitudinal axis of the strut. The electrical trace includes a rolled annealed metal. The serpentine shape can be formed from the rolled annealed metal along a direction of individual grains of the rolled annealed metal such that the individual grains run parallel to the longitudinal axis of the strut.

There is provided, in accordance with the disclosed technology, a method of manufacturing an end effector for a medical catheter. The method includes forming a strut for a framework of the end effector and providing alternating wide sections and narrow sections extending along a longitudinal axis of the strut. The method includes disposing an electrical trace on a substrate. The method includes forming a plurality of bends in the substrate and the electrical trace. The method includes aligning the substrate and the electrical trace with the strut such that apexes of the plurality of bends are positioned at corresponding locations of the narrow sections of the strut.

### BRIEF DESCRIPTION OF THE DRAWINGS

FIG. 1 is a schematic pictorial illustration of a medical system including a planar catheter, in accordance with the disclosed technology;
FIG. 2 is an illustration of a perspective view of an end effector, in accordance with the disclosed technology;
FIG. 3 is an illustration of an exploded perspective view of the end effector of FIG. 2, in accordance with the disclosed technology;
FIG. 4A is an illustration of a cross section of an end effector, in accordance with the disclosed technology;
FIG. 4B is an illustration of a cross section of an end effector, in accordance with the disclosed technology;
FIG. 4C is a variation of FIG. 4B in which a framework is not encapsulated into the insulative material but is contiguous therewith, in accordance with the disclosed technology;
FIG. 4D is an illustration of a cross section of an end effector, in accordance with the disclosed technology;
FIG. 5A is an illustration of an exploded perspective view of an end effector with support layers, in accordance with the disclosed technology;
FIG. 5B is an illustration of a cross section of an end effector with two support layers, in accordance with the disclosed technology;
FIG. 5C is an illustration of a cross section of an end effector with a single support layer, in accordance with the disclosed technology;
FIG. 5D is an illustration of a cross section of an end effector with a single support layer and only one surface with electrodes, in accordance with the disclosed technology;
FIG. 5E is an illustration of a cross section of an end effector with a single support layer and only one surface with electrodes, in accordance with the disclosed technology;
FIG. 6A is a cross sectional view of an end effector with flexible circuits that shift in planes, in accordance with the disclosed technology;
FIG. 6B is a cross sectional view of an end effector with flexible circuits that shift in planes, the circuits including a medial planar section, in accordance with the disclosed technology;
FIG. 6C is a cross sectional view of an end effector with flexible circuits that shift in planes, the circuits including a medial planar section that touches a central framework, in accordance with the disclosed technology;
FIG. 6D is a cross sectional view of an end effector with flexible circuits that shift in planes, the circuits including a medial planar section and a superficial planar section, in accordance with the disclosed technology;
FIG. 6E is a cross sectional view of an end effector with flexible circuits with corrugate profiles, the circuits including a medial planar section and a superficial planar section, in in accordance with the disclosed technology;
FIG. 6F is a cross sectional view of an end effector with flexible circuits with corrugate profiles, the circuits including a medial planar section and a superficial planar section, in accordance with the disclosed technology;
FIG. 7 is a cross sectional view of an end effector with two support layers and flexible circuits that shift in planes, in accordance with the disclosed technology;
FIG. 8 is an illustration of a catheter assembly, in accordance with the disclosed technology;
FIG. 9 is a schematic pictorial illustration of a medical system including a planar catheter, in accordance with the disclosed technology;
FIG. 10A is a schematic pictorial illustration of an end effector, in accordance with the disclosed technology;
FIG. 10B is a schematic pictorial illustration showing a detail view of an electrode arrangement of FIG. 10A, in accordance with the disclosed technology;
FIG. 10C is a schematic pictorial illustration of an end effector, in accordance with the disclosed technology;
FIG. 11A is a schematic pictorial illustration of an end effector, in accordance with the disclosed technology;
FIG. 11B is a schematic pictorial illustration showing a right-side view of the end effector of FIG. 11A, in accordance with the disclosed technology;
FIG. 12A is a schematic pictorial illustration of an end effector, in accordance with the disclosed technology;
FIG. 12B is a schematic pictorial illustration showing a right-side view of the end effector of FIG. 12A, in accordance with the disclosed technology;
FIG. 13 is a schematic pictorial illustration of a medical system including a planar catheter, in accordance with the disclosed technology;
FIG. 14 is a schematic pictorial illustration showing an exploded view of a portion of an end effector, in accordance with the disclosed technology;
FIG. 15A is a schematic pictorial illustration showing a center loop of the end effector of FIG. 14, in accordance with the disclosed technology;
FIG. 15B is a schematic pictorial illustration showing two side loops of the end effector of FIG. 14, in accordance with the disclosed technology;
FIG. 16 is a schematic pictorial illustration showing the center loop and two side loops of the end effector of FIG. 14 disposed on an insulative material and a spine framework, in accordance with the disclosed technology;
FIG. 17 is a schematic pictorial illustration showing an exploded view of a portion of a modified configuration of the end effector of FIG. 14, in accordance with the disclosed technology;
FIG. 18 is a schematic pictorial illustration showing two modified side loops of the end effector of FIG. 14, in accordance with the disclosed technology;
FIG. 19 is a schematic pictorial illustration showing the center loop of FIG. 15A and the two side loops of FIG. 18 disposed on an insulative material and a spine framework, in accordance with the disclosed technology;
FIG. 20 is an illustration of a catheter assembly, in accordance with the disclosed technology;
FIG. 21 is a schematic pictorial illustration of a medical system including a catheter, in accordance with the disclosed technology;
FIG. 22 is a schematic pictorial illustration showing an exploded view of a portion of an end effector, in accordance with the disclosed technology;
FIG. 23 is a schematic pictorial illustration showing a framework of the end effector of FIG. 22 embedded in insulative material, in accordance with the disclosed technology;
FIG. 24 is a schematic pictorial illustration showing a view, in the proximal direction, of the end effector of FIG. 23 with a force applied thereto, in accordance with the disclosed technology;
FIG. 25 is a schematic pictorial illustration showing a detail view of a spine of the framework of FIG. 23, in accordance with the disclosed technology;
FIG. 26 is a schematic pictorial illustration showing a modified framework, similar to the framework of FIG. 23, embedded in insulative material, in accordance with the disclosed technology;
FIG. 27 is a schematic pictorial illustration showing a view, in the proximal direction, of the end effector of FIG. 26 with a force applied thereto, in accordance with the disclosed technology;
FIG. 28 is a schematic pictorial illustration showing a detail view of a spine of the framework of FIG. 26, in accordance with the disclosed technology;
FIG. 29 is a schematic pictorial illustration showing another framework of another end effector, in accordance with the disclosed technology;
FIG. 30 is a schematic pictorial illustration showing the framework of FIG. 29 embedded in insulative material and with a flexible circuit assembled therewith, in accordance with the disclosed technology;
FIG. 31 is a schematic pictorial illustration showing a cross sectional view cut along line 31-31 in FIG. 30, in accordance with the disclosed technology;
FIG. 32 is a schematic pictorial illustration showing a cross sectional view cut along line 32-32 in FIG. 30, in accordance with the disclosed technology;
FIG. 33 is a schematic pictorial illustration showing yet another framework embedded in insulative material, in accordance with the disclosed technology;
FIG. 34 is a schematic pictorial illustration showing yet another modified framework, similar to the framework of FIG. 33, embedded in insulative material, in accordance with the disclosed technology;
FIG. 35 is a schematic pictorial illustration showing an end effector, that uses the framework of FIG. 33 or FIG. 34, being inserted into an introducer, in accordance with the disclosed technology;
FIG. 36 is a schematic pictorial illustration showing the end effector of FIG. 35 inserted into the introducer, in accordance with the disclosed technology;
FIG. 37A is a schematic pictorial illustration showing a top view of an end effector with a first edge treatment, in accordance with the disclosed technology;
FIG. 37B is a schematic pictorial illustration showing the end effector of FIG. 37A inserted into an introducer, in accordance with the disclosed technology;
FIG. 38A is a schematic pictorial illustration showing a top view of an end effector with a second edge treatment, in accordance with the disclosed technology;
FIG. 38B is a schematic pictorial illustration showing the end effector of FIG. 38A inserted into an introducer, in accordance with the disclosed technology;
FIG. 39A is a schematic pictorial illustration showing a top view of an end effector with a third edge treatment, in accordance with the disclosed technology;
FIG. 39B is a schematic pictorial illustration showing the end effector of FIG. 39A inserted into an introducer, in accordance with the disclosed technology;
FIG. 40A is a schematic pictorial illustration showing a top view of an end effector with a fourth edge treatment, in accordance with the disclosed technology;
FIG. 40B is a schematic pictorial illustration showing the end effector of FIG. 41A inserted into an introducer, in accordance with the disclosed technology;
FIG. 41 is a flowchart showing a method of using an end effector for a medical catheter, in accordance with the disclosed technology;
FIG. 42 is a flowchart showing a method of manufacturing an end effector for a medical catheter, in accordance with the disclosed technology;
FIG. 43 is a schematic pictorial illustration of a medical system including a planar catheter, in accordance with the disclosed technology;
FIG. 44 is an illustration of a perspective view of an end effector, in accordance with the disclosed technology;
FIG. 45 is an illustration of an exploded perspective view of the end effector of FIG. 2, in accordance with the disclosed technology;
FIG. 46A is an illustration of a cross section of an end effector, in accordance with the disclosed technology;
FIG. 46B is an illustration of a cross section of an end effector, in accordance with the disclosed technology;
FIG. 46C is a variation of FIG. 46B in which a framework is not encapsulated into the insulative material but is contiguous therewith, in accordance with the disclosed technology;
FIG. 46D is an illustration of a cross section of an end effector, in accordance with the disclosed technology;
FIG. 47 is an illustration of a perspective view of an end effector with a planar transition zone proximally, in accordance with the disclosed technology;
FIG. 48A is a top plan view of an end effector with a planar transition zone proximally, only one surface of the end effector comprising electrodes, in accordance with the disclosed technology;
FIG. 48B is a bottom plan view of the end effector shown in FIG. 48A, in accordance with the disclosed technology;
FIG. 48C is a side view of the end effector shown in FIGs. 48A and 48B, in accordance with the disclosed technology;
FIG. 49A is a top plan view of an end effector with a planar transition zone proximally, a first surface of the end effector having electrodes and a second surface having an electromagnetic coil, in accordance with the disclosed technology;
FIG. 49B is a bottom plan view of the end effector shown in FIG. 49A, in accordance with the disclosed technology;
FIG. 49C is a side view of the end effector shown in FIGs. 49A and 49B, in accordance with the disclosed technology;
FIG. 50A is an illustration of a perspective, exploded view of an end effector with a planar transition zone proximally and alternating levels layers of insulative sheets, in accordance with the disclosed technology;
FIG. 50B is an illustration of a perspective, exploded view of an end effector with a planar transition zone proximally and alternating levels layers of insulative sheets and flexible circuits on both sides of the end effector, in accordance with the disclosed technology;
FIG. 51 is an illustration of a catheter assembly, in accordance with the disclosed technology;
FIG. 52 is a schematic pictorial illustration of a medical system including a planar catheter, in accordance with the disclosed technology;
FIG. 53 is an illustration of a perspective view of an end effector, in accordance with the disclosed technology;
FIG. 54A is a detailed view of an electrical trace showing sections of alternating grain lengths in their crystal structure, in accordance with the disclosed technology;
FIG. 54B is a detailed view of rolled annealed metal, in accordance with the disclosed technology;
FIG. 55 is a top plan view of a serpentine flexible circuit having electrical traces as shown in FIG. 54B applied to a substrate, in accordance with the disclosed technology;
FIG. 56 is a top plan view of the flexible circuit of FIG. 55 applied to a support framework, in accordance with the disclosed technology;
FIG. 57 is an illustration of a catheter assembly, in accordance with the disclosed technology; and
FIG. 58 is a flowchart showing a method of manufacturing an end effector for a medical catheter, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 70.1% or 71% to 109.9% or 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode catheter for the treatment of drug refractory atrial fibrillation to a subject.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal apoptosis inducing technology that can be used for ablation of atrial arrhythmias. To ablate using IRE/PEF, biphasic voltage pulses are applied to disrupt cellular structures of myocardium leading to apoptosis. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis leaving little or no scarring. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

The following description relates to various exemplary configurations of medical probes (and/or portions there), systems associated therewith, and methods of use and/or manufacture thereof.

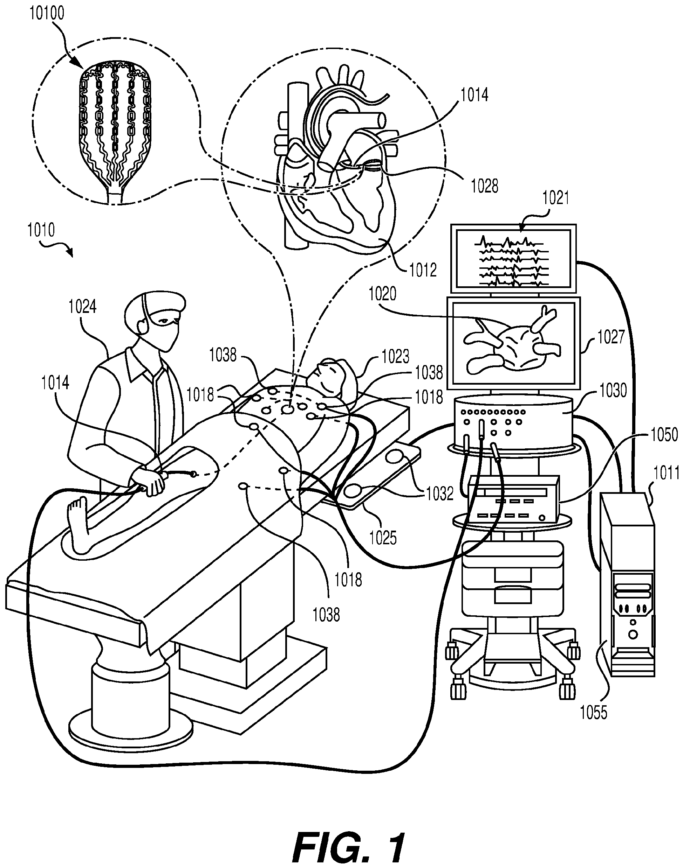

### Encapsulated Planar Catheters with Planar Shifts and Support Layers (FIGs. 1-8)

Reference is made to FIG. 1 showing an example catheter-based electrophysiology mapping and ablation system 1010. System 1010 includes multiple catheters, which are percutaneously inserted by physician 24 through the patient's 1023 vascular system into a chamber or vascular structure of a heart 12. Typically, a delivery sheath catheter is inserted into the left or right atrium near a desired location in heart 1012. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 1014 that is configured for sensing IEGM is illustrated herein. Physician 1024 brings a catheter shaft 1090 with distal tip of catheter 1014 (i.e., multilayered end effector 10100) into contact with the heart wall for sensing a target site in heart 1012. For ablation, physician 1024 would similarly bring a distal end of an ablation catheter to a target site for ablating.

Catheter 1014 is an exemplary catheter that includes one and preferably multiple electrodes 1026 optionally distributed over end effector 10100 coupled to a catheter shaft 1090 and configured to sense the IEGM signals as described in more detail below. Catheter 14 may additionally include a position sensor embedded in or near end effector 10100 for tracking position and orientation of end effector 10100. Optionally and preferably, position sensor is a magnetic based position sensor including multiple magnetic coils for sensing three-dimensional (3D) position and orientation.

Magnetic based position sensor may be operated together with a location pad 1025 including a plurality of magnetic coils 1032 configured to generate magnetic fields in a predefined working volume. Real time position of end effector 10100 of catheter 1014 may be tracked based on magnetic fields generated with location pad 1025 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 1010 includes one or more electrode patches 1038 positioned for skin contact on patient 1023 to establish location reference for location pad 1025 as well as impedance-based tracking of electrodes 1026. For impedance-based tracking, electrical current is directed toward electrodes 1026 and sensed at electrode skin patches 1038 so that the location of each electrode can be triangulated via the electrode patches 1038. Details of the impedance-based location tracking technology are described in US Patent Nos. 7,536,218; 7,756,576; 7,848,787; 7,869,865; and 8,456,182, each of which are incorporated herein by reference.

A recorder 1011 displays electrograms 1021 captured with body surface ECG electrodes 1018 and intracardiac electrograms (IEGM) captured with electrodes 1026 of catheter 1014. Recorder 1011 may include pacing capability for pacing the heart rhythm and/or may be electrically connected to a standalone pacer.

System 1010 may include an ablation energy generator 1050 that is adapted to conduct ablative energy to one or more of electrodes 1026 at a distal tip of a catheter configured for ablating. Energy produced by ablation energy generator 1050 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 1030 is an interface configured to establish electrical communication between catheters, electrophysiological equipment, power supply and a workstation 1055 for controlling operation of system 1010. Electrophysiological equipment of system 1010 may include for example, multiple catheters, location pad 1025, body surface ECG electrodes 1018, electrode patches 1038, ablation energy generator 1050, and recorder 1011. Optionally and preferably, PIU 1030 additionally includes processing capability for implementing real-time computations of location of the catheters and for performing ECG calculations.

Workstation 1055 includes memory, processor unit with memory or storage with appropriate operating software loaded therein, and user interface capability. Workstation 1055 may provide multiple functions, optionally including (1) modeling the endocardial anatomy in three-dimensions (3D) and rendering the model or anatomical map 1020 for display on a display device 1027, (2) displaying on display device 1027 activation sequences (or other data) compiled from recorded electrograms 1021 in representative visual indicia or imagery superimposed on the rendered anatomical map 20, (3) displaying real-time location and orientation of multiple catheters within the heart chamber, and (4) displaying on display device 1027 sites of interest such as places where ablation energy has been applied. One commercial product embodying elements of the system 1010 is available as the CARTO^{™} 3 System in conjunction with an energy generator TruPulse^{™}, available from Biosense Webster, Inc., 31 Technology Drive, Suite 200, Irvine, CA 92618.

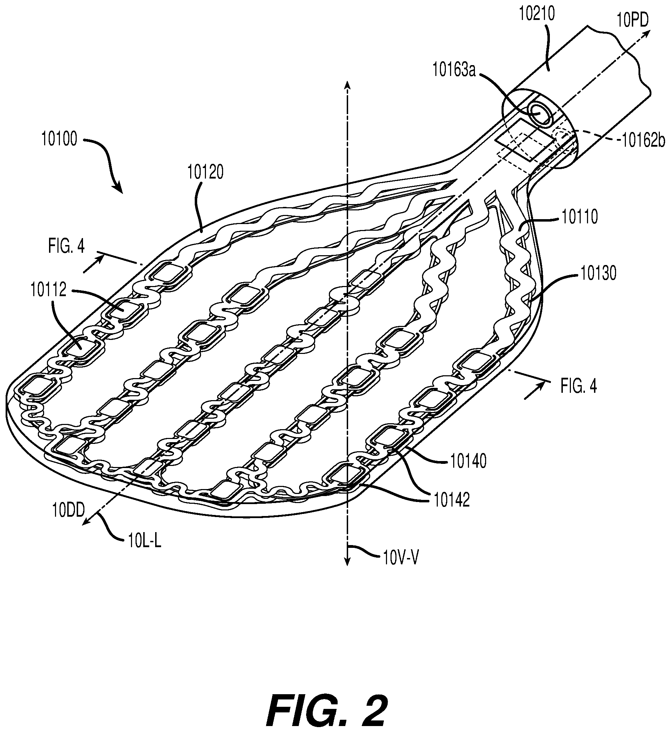

FIG. 2 provides an end effector 10100 in accordance with an embodiment of the present disclosure in order to achieve the ease of manufacturing, reduced cost, and enhanced end effector properties, such as desired stiffness, mapping resolution, electrode contact with target anatomy, and conformity of the end effector disclosed herein to flat, curved, irregular and/or nonplanar tissue surfaces found in the target anatomy. The end effector 10100 can include a flexible circuit 10110 including a plurality of electrodes 10112, each electrode of the plurality of electrodes 10112 including a contact surface 10112c. As used herein, the term "flexible circuit" includes thin-film circuit, flexible printed circuit board, thin film deposition via lithography and etching processes on substrates such as polyimide, copper, LCP, nitinol substrate, TPU, silicone, thermoset resin, or other polymeric substrate as shown and described in the technical references incorporated herein by the Appendix attached in Priority Application No. 63/615,600. In some examples, the flexible circuits described herein can be made primarily of polyimide. In other examples, it can be made of any of biocompatible polyimides, glass-reinforced epoxy laminate materials, copper, or graphene, alone or in combination. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuit 10110 can be disposed on an insulative material 10120. The insulative material 10120 can be contiguous to the contact surfaces 10112c so that only the contact surfaces 10112c of at least a portion of the plurality of electrodes 10112 are exposed to the ambient environment. As used herein, the term "contact surface" includes the portion of an electrode having a generally flat surface and the edge or edges which immediately surround said flat surface. Electrodes 10112 may have a slightly rounded, radiused, or chamfered edge that comes into contact with tissue, along with the generally flat surface, when the end effector 10100 is placed against tissue. As used herein, "ambient environment" refers to the external environment such as the organ in which the end effector 10100 is deployed or in the operating theater prior to being deployed in the biological organ.

It is noted that not all of the electrodes on the end effector 10100 described herein need be exposed through the insulative material 10120 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 10163a on one side and 10162b on the other side in fluid communication with an irrigation line not shown disposed in a catheter shaft 1090. Instead of an irrigation line separate from the catheter shaft 1090, a lumen can be formed via extrusion of the catheter shaft 90 to provide for a lumen channel. It is noted that port 10163a or 10162b can be configured to have a sufficient flow diverter characteristic for irrigation fluid to cover the mapping electrodes during irrigation flow so as to prevent or reduce thrombus formations.

The flexible circuit 10110 can further include a framework 10130 contiguous to the insulative material or in the insulative material. In examples in which the flexible circuit 10110 includes framework 10130, the framework 10130 can be disposed directly on the flexible circuit 10110 with none, or very little, of the insulative material 10120 coming between the two.

Stated otherwise, an aspect of the present disclosure provides an end effector 10100 having a planar framework 10130 bisecting two flat, heat formed portions 10120a, 10120b of a flexible insulating mass 10120, with at least one flexible circuit 10110 disposed on one side of the framework 10130 and with contact surfaces 10112c of electrodes 10112 extending up to or slightly past the outer face of the flexible insulating mass 10120. In additional or alternative examples, the contact surfaces 10112c of electrodes 10112 can be slightly recessed, where there could be an opening through the insulative material 10120 to ensure that the electrode 10112 is exposed.

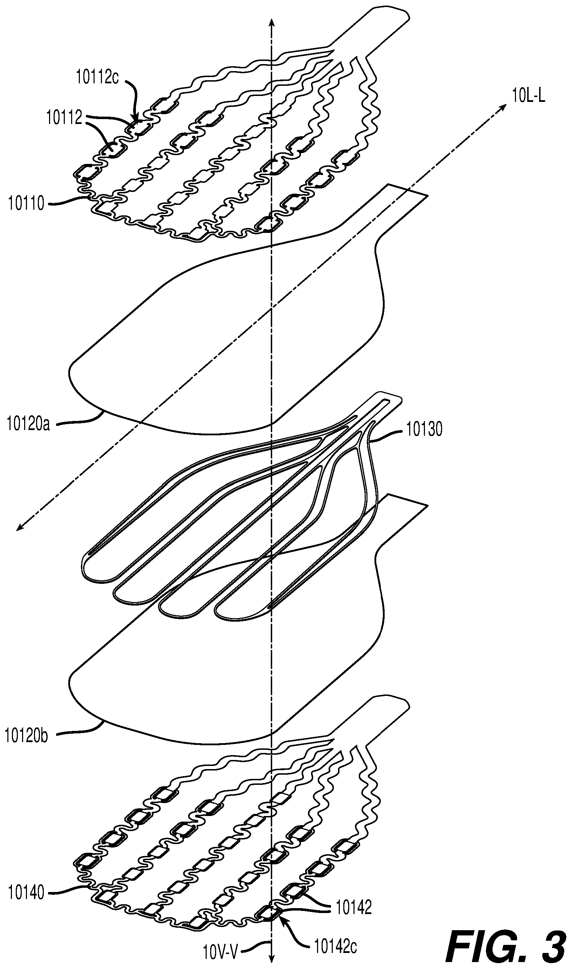

FIG. 3 shows an exploded view of the end effector 10100, with the components thereof exploded vertically along vertical axis 10V-V. The flexible circuit 10110 can be a first flexible circuit 10110 and the plurality of electrodes 10112 can be a first plurality of electrodes 10112, with each first electrode 10112 including a first contact surface 10112c. The end effector 10100 can further include a second flexible circuit 10140 having a second plurality of electrodes 10142. The second flexible circuit 10140 can be spaced apart from the first flexible circuit 10110 and each electrode of the second plurality of electrodes 10142 can have a second contact surface 1010142c.

In examples having a first 10110 and second 10140 flexible circuits, the insulative material 10120 can be disposed between the first flexible circuit 10110 and the second flexible circuit 10140, and the insulative material 10120 can be contiguous to the second contact surfaces 1010142c so that only the contact surface 1010142c of each second electrode 10142 is exposed to the ambient environment, similar to how first electrodes 10112 are disposed in and exposed through the insulative material.

Electrodes 10112, 10142 can sense or receive signals generated by the tissues or transmit energy AC or DC from an energy generator to the tissues. In some examples, there are about 92 electrodes. In some examples, there are about 48 electrodes. In some examples, there are about 64 electrodes. In some examples, there are about 72 electrodes. In some examples, there are about 98 electrodes. Details of the spacing of the electrodes for each pair vs spacing between discrete sets of pairs of electrodes can be found in US Provisional Patent Application S.N. 63/406,673 filed on September 14, 2022 and included in the Appendix of Priority Application No. 63/615,600.

In examples wherein the end effector 10100 includes a framework 10130 disposed between the first flexible circuit 10110 and the second flexible circuit 10140.

Framework 10130 can be a component of the end effector 10100 that is separate and distinct from the first flexible circuit 10110 and disposed proximate the first flexible circuit 10110. In this case, the insulative material 10120 can be further disposed between the framework 10130 and the second flexible circuit 10140. The framework 10130 can be formed from a planar or cylindrical stock of material using any suitable method. For example, the framework 10130 can be formed by cutting, laser cutting, stamping, etc.

Insulative material 10120 can include a first sheet of insulative material 10120a and a second sheet of insulative material 10120b fused together proximate the framework 10130 into a single, contiguous, generally planar insulative mass 10120. This insulative material 10120 also serves to enhance the atraumaticity of the end effector 10100 and to protect the subject from sharp edges. The insulative material 10120 can include polymer. The insulative material 10120 can be heat formed around at least a portion of the first flexible circuit 10110, the second flexible circuit 10140, and the framework 10130. The polymer can include thermoplastic polyurethane (TPU) or other heat formed or shaped material which lends itself to said heat forming.

Furthermore, while the insulative material 10120 is shown to be flat in these figures, insulative material 10120 can be shaped, scalloped, ribbed, ridged, concaved, convexed, or otherwise configured such that the overall profile of insulative material 10120 yields physical and/or mechanical properties, such as rigidity and flexion along multiple axes, required by the end effector 10100, mentioned above.

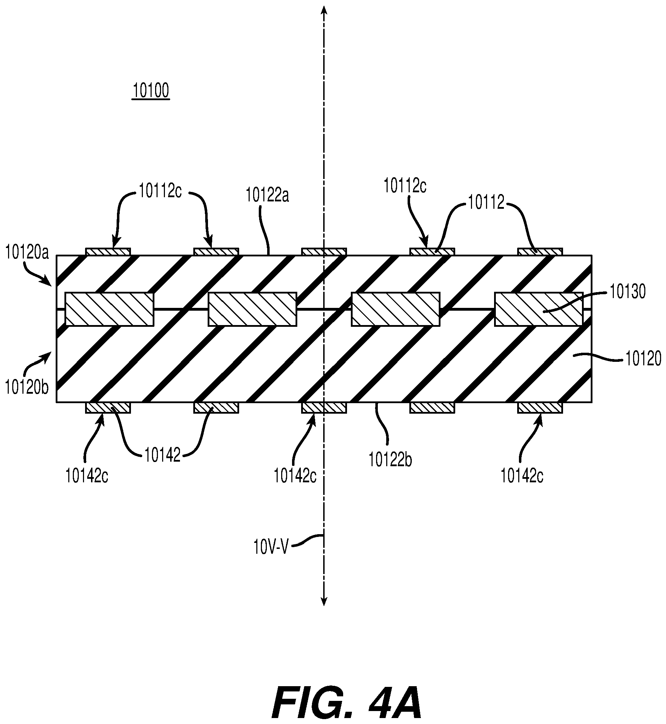

FIG. 4A shows a cross-section taken as indicated in FIG. 3. The first contact surfaces 10112c lie substantially parallel to a first outer surface 10122a of the insulative mass 10120. The first plurality of electrodes 10112 extending vertically and outwardly a first distance therefrom. Similarly, the second contact surfaces 10142c can lie substantially parallel to a second outer surface 10122b of the insulative mass 10120 and extend substantially vertically and outwardly a second distance therefrom.

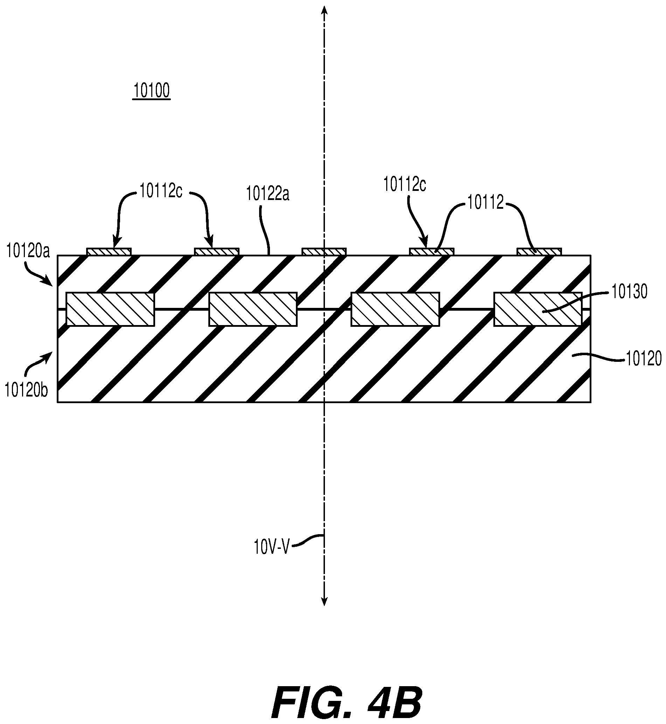

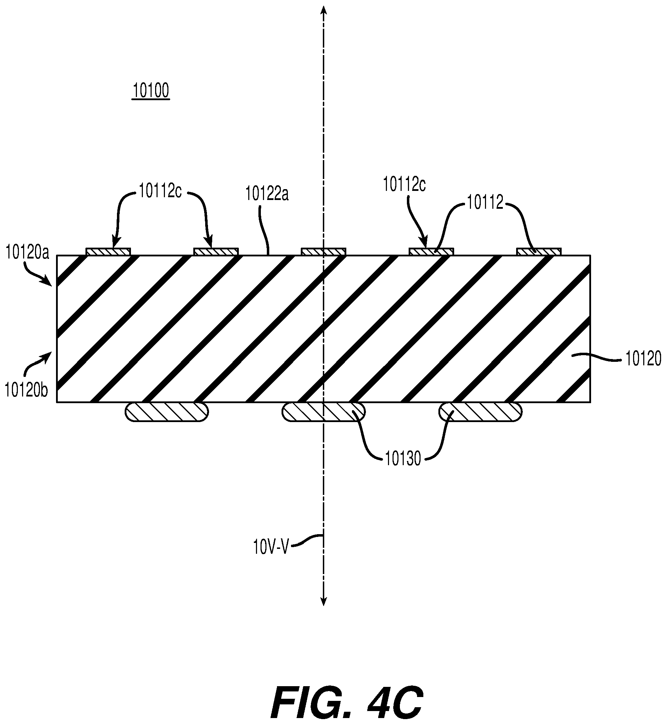

FIG. 4B shows a similar cross-section, but from an example end effector 10100 which does not have the second flexible circuit 10140. That is, the end effector in FIG. 4B has electrodes on only one side (single sided end effector) as compared to an end effector of FIG. 4A with electrodes on opposite sides (or double-sided end effector). FIG. 4C shows a variation of FIG. 4B in which the framework 10130 is not encapsulated in the insulative material but disposed outside the insulative material 10120. It should be noted that while framework 10130 is shown in cross-section as rectangular, the framework 10130 is not limited such cross-section and any suitable cross-sections can be utilized.

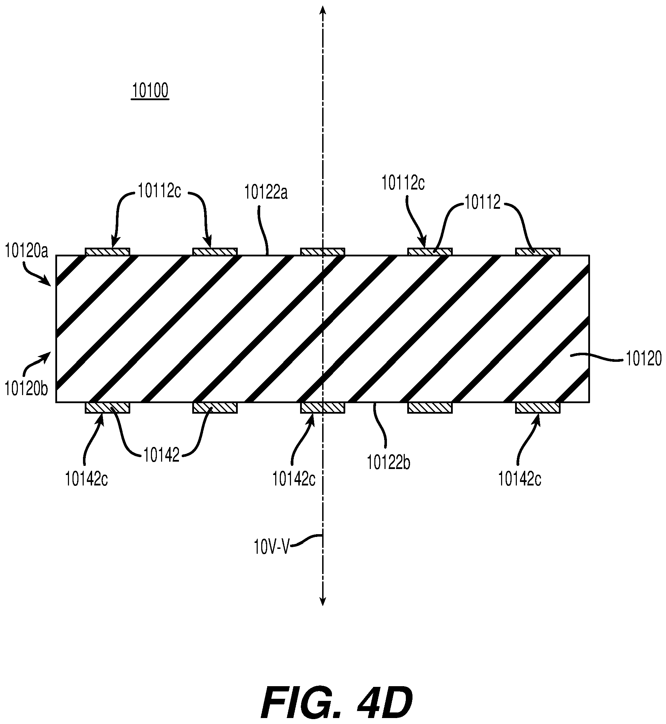

FIG. 4D shows a cross-section similar to that of FIG. 4A, but from an example end effector 10100 which does not have the framework 10130. It should be noted that in examples of FIG. 4D and the like, the insulative material 10120 may or may not have additional strength or formed thicker, as compared to the insulative material 10120 of the examples with framework 10130. The additional strength of the insulative material 10120 can be to compensate for the lack of framework 10130.

Importantly, the end effector 10100 having flush or outwardly protruding electrode contact surfaces 10112c, 10142c can be manufactured without the need to remove material in order expose electrode contact surfaces 10112c, 10142c via the method and/or fixture described in more detail below.

In other examples, the first contact surfaces 10112c can lie substantially coplanar to a first outer surface 10122a of the insulative mass 10120, and the second contact surfaces 10142c lying coplanar to a second outer surface 10122b of the insulative mass 10120.

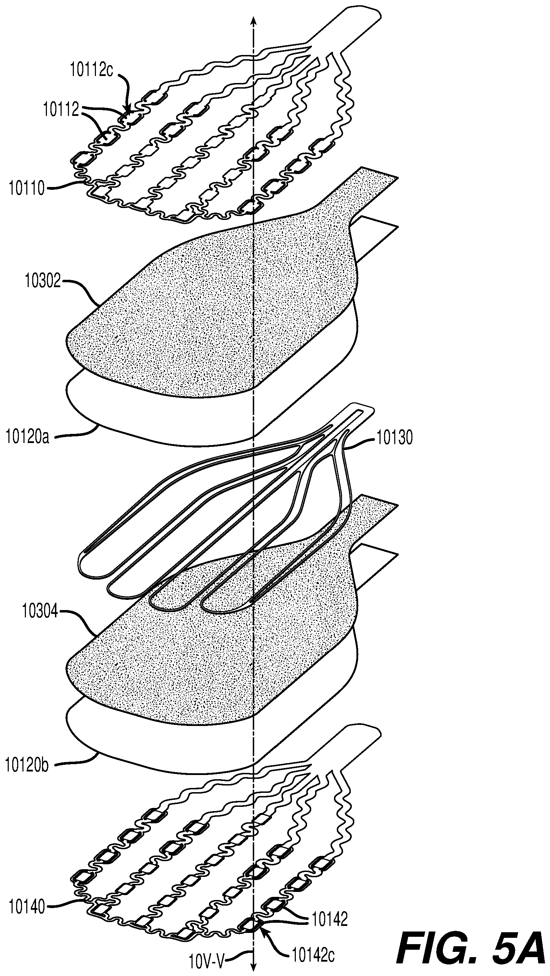

FIG. 5A an exploded perspective view of an end effector 10100 with support layers (e.g., first support layer 10302 and second support layer 10304), in accordance with the disclosed technology. The end effector 10100 shown in FIG. 5A is substantially similar to that which is shown and described with respect to FIG. 3, but with the addition of the support layers 10302, 10304. Suspending flexible circuits, such as flexible circuit 10110 and second flexible circuit 10140, in an insulative material 10120 such as a polymer matrix enables the flex circuits to maintain a certain amount of independence from each other. Introducing one or more support layers 10302, 10304 to the insulative material 10120 enables the end effector 10100 to take advantage of different characteristics of different types of materials while maintaining the independence of the flex circuits from each other, as well as from any structural metal components (e.g., the framework 10130). It should be noted that the support layers 10302, 10304 are depicted as being "above" their respective insulative material 10120a, 10120b in FIG. 5A, but it will be appreciated that the support layers 10302, 10304 can be positioned at any number of positions in or on the insulative material 10120a, 10120b.

The material for the support layers 10302, 10304 can include, for example, thermoplastic polyurethane (TPU), polyimide, polytetrafluoroethylene, ethylene tetrafluoroethylene, silicone, siloxane, and similar materials or polymers or combinations thereof. The support layers 10302, 10304 can enable the end effector 10100 to remain flexible, while also providing tensional support to prevent overstretching the flexible circuits. In some examples, the support layers 10302, 10304 can be a mesh polymer, thereby providing the aforementioned stretch resistance while also providing the flexibility needed for the end effector 10100.

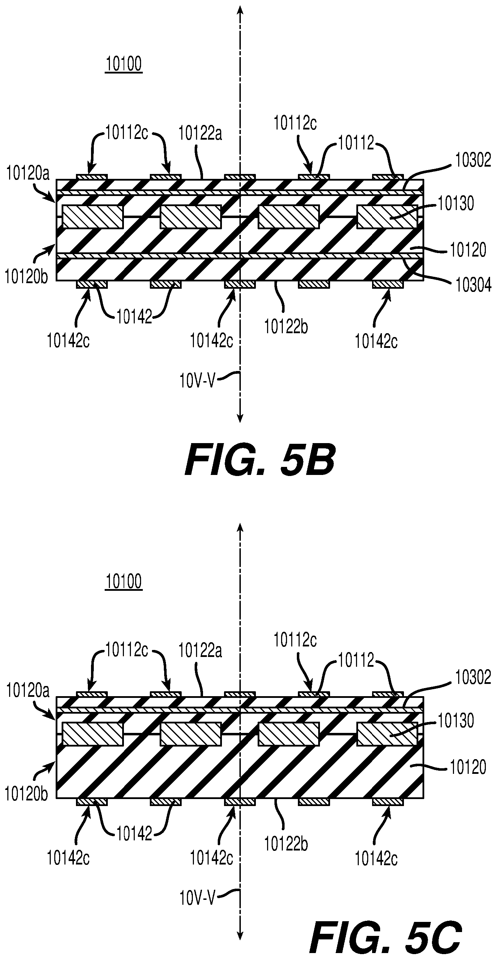

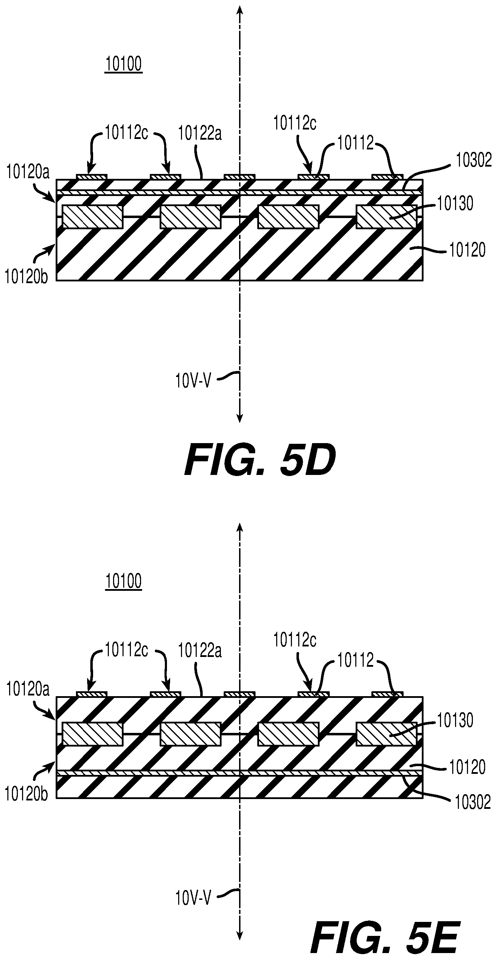

Referring now to FIG. 5B, the figure shows an example end effector 10100 including two separate support layers 10302, 10304. A first support layer 10302 is positioned (i) in the first sheet of insulative material 10120a and (ii) between the framework 10130 and the electrodes 10112 on that side of the end effector 10100. A second support layer 10304 is positioned (i) in the second sheet of insulative material 10120b and (ii) between the framework 10130 and the electrodes 10112 on that other side of the end effector 10100. This configuration can provide robust axial support for the flexible circuits. In some examples, however, a support layer may only be needed on one side of the framework 10130 to provide the necessary support for the flexible circuits. FIG. 5C, for example, shows an example end effector 10100 with electrodes 10112 on both surfaces of the end effector, yet the device includes a single support layer 10302 positioned between the framework 10130 and one surface of the end effector containing electrodes 10112. As shown in FIGs. 5D and 5E, and as described above with respect to FIG. 4B above, the end effector 10100 may include electrodes on only one surface. Considering this, in one example, a single support layer 10302 can be positioned between the framework 10130 and the first outer surface 10122a with the electrodes (see FIG. 5D); in other examples, a single support layer 10302 can be positioned between the framework 10130 and the opposite surface without the electrodes (see FIG. 5E).

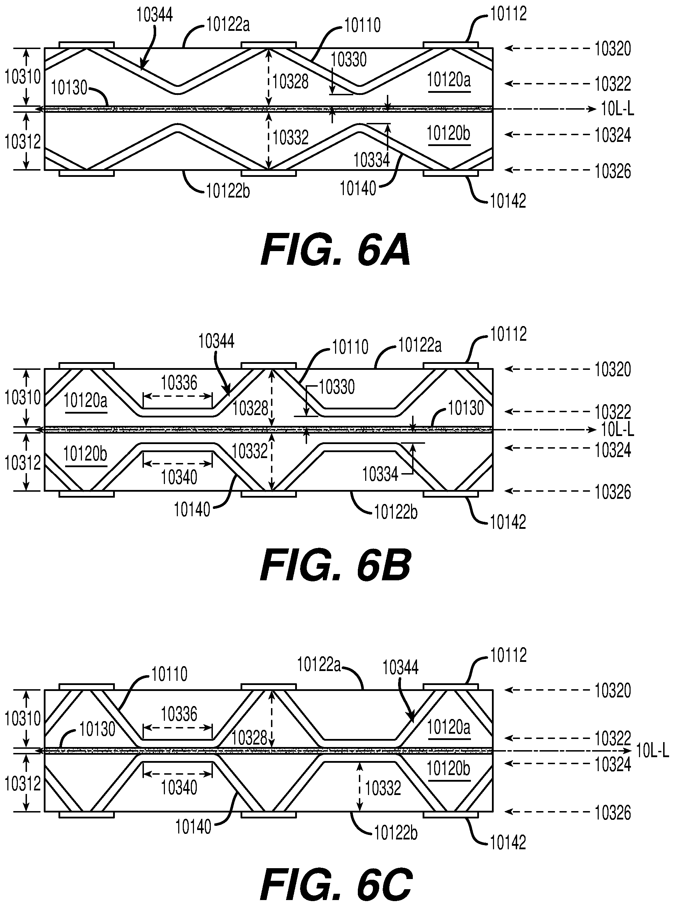

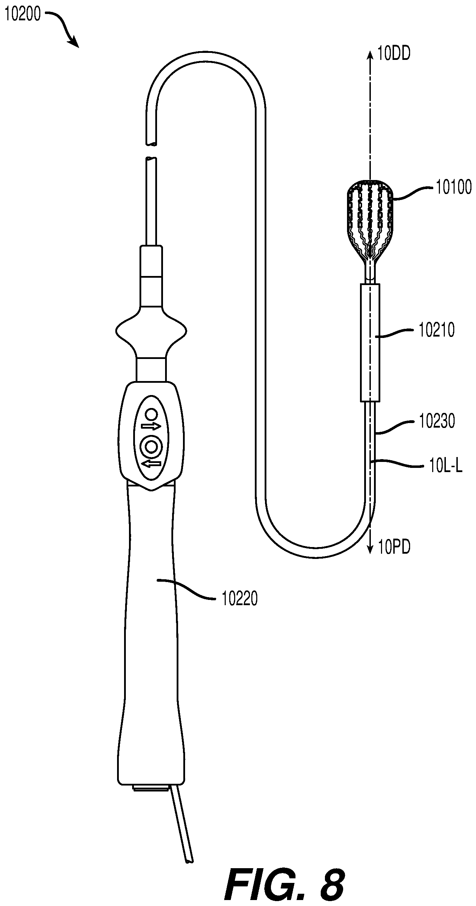

Referring now to FIGs. 6A-6F, the examples shown therein include flexible circuits that have planar shifts along their longitudinal axis 10L-L (see FIG. 3 for reference). For example, each of these examples include an embodiment wherein a flexible circuit (e.g., first flexible circuit 10110 and second flexible circuit 10140) is positioned longitudinally along and at least partially within the insulative material (e.g., insulative material 10120a, 10120b) and is further positioned such that a first portion of the flexible circuit is contiguous to a first plane and a second portion of the flexible circuit is contiguous to a second plane, the first plane being a first distance from the framework 10130 and the second plane being a second distance from the framework 10130, the second distance being less than the first distance. This shift in planes can be localized to areas known to undergo tight bend radii when being traversed through a vessel or outer sheath (e.g., tubular member 10230 in FIG. 8). Shifting the flexible circuit from the outside of the insulative material 10120a, 10120b (in locations where at least a portion of electrodes are exposed), to the inside of the insulative material 10120a, 10120b (in locations that undergo tight bends), can ensure that the layers flexible circuits remain intact. Shifting the flexible circuit plane closer to the neutral axis (e.g., the framework 10130 if the end effector 10100 includes a framework 10130 centrally placed like in FIGs. 6A-6F) can limit the amount of stress and strain from tension and compression on the circuits. The shift can also enable the overall end effector 10100 to undergo a tighter bend, while the flexible circuit(s) are slightly less tight.

FIG. 6A shows an example end effector 10100 with two flexible circuits 10110, 10140 that extend from a surface-exposed electrode 10120b, into the insulative material 10120a, 10120b, and then back to another surface-exposed electrode 10120b. Referring first to the top flexible circuit in FIG. 6A, which is first flexible circuit 10110, the flexible circuit 10110 extends from the surface electrodes in a first plane 10320, then into the insulative material 10120a to a second plane 10322, and then back to the surface electrodes in the first plane 10320. In this example, the flexible circuit 10110 is in the shape of a triangle wave, wherein (i) the trough of the flexible circuit 10110 is the second plane 10322, (ii) the peaks are at the surface electrodes 10112, and (iii) the peaks and troughs are connected with angled sections 10344.

The second flexible circuit 10140 is substantially the same on the other side of the end effector 10100. For instance, second flexible circuit 10140 extends from the surface electrodes in a fourth plane 10326, then into the insulative material 10120b to a third plane 10324, and then back to the surface electrodes in the fourth plane 10326.

Further, in the example shown in FIG. 6A, the planes within the end effector 10100 closer to the framework 10130 are separated a distance from the framework 10130. In other words, the first outer surface 10122a is at a first height 10310 from the framework 10130, and in this example a first distance 10328 between the framework 10130 and the first plane 10320 is therefore equal to the first height 10310; the second outer surface 10122b is at a second height 10312 from the framework 10130, and in this example a second distance 10332 between the framework 10130 and the fourth plane 10326 is therefore equal to the second height 10312. The embedded planes are separated from the framework 10130, however, such that there is a third distance 10330 between the framework 10130 and the second plane 10322, and there is a fourth distance 10332 between the framework 10130 and the third plane 10324. The third distance 10330 and the fourth distance 10334 are greater than zero to maintain certain gap between the framework 10130 and the flexible circuits 10110, 10140.

FIG. 6B shows a similar construct as shown in FIG. 6A, but instead of the flexible circuits 10110, 10140 having a pure triangle wave pattern, the flexible circuits 10110, 10140 extend into their respective insulative material 10120a, 10120b at an angled section 10344, and then extend for a length parallel to the framework 10130 before angling back toward the surface electrodes 10112. In this instance, the first flexible circuit 10110 includes a first medial planar section 10336 that extends for a length parallel to the framework 10130. The second flexible circuit 10140 includes a second medial planar section 10340 that extends for a length parallel to the framework 10130. The first medial planar section 10336 is embedded in the first insulative material 10120a within the second plane 10322, and the second medial planar section 10340 is embedded in the second insulative material 10120b within the third plane 10324. In the example shown in FIG. 6B, the embedded planes 10322, 10324 are separated from the framework 10130 such that there is a certain gap between the framework 10130 and the flexible circuits 10110, 10140. For example, the third distance 10330 between the framework 10130 and the second plane 10322 is greater than zero, and the fourth distance 10334 between the framework 10130 and the third plane 10324 is greater than zero.

FIG. 6C shows a similar construct as shown in FIG. 6B, but instead of a gap existing (e.g., at third distance 10330 and fourth distance 10334) between the framework 10130 and the embedded planes 10322, 10324, the embedded planes 10322, 10324 are contiguous with the framework 10130. For example, the first medial planar section 10336 extends along a length of the framework 10130 in direct contact with or proximate to the framework 10130, and the second medial planar section 10340 extends along a length of the framework 10130 in direct contact with or proximate to the framework 10130.

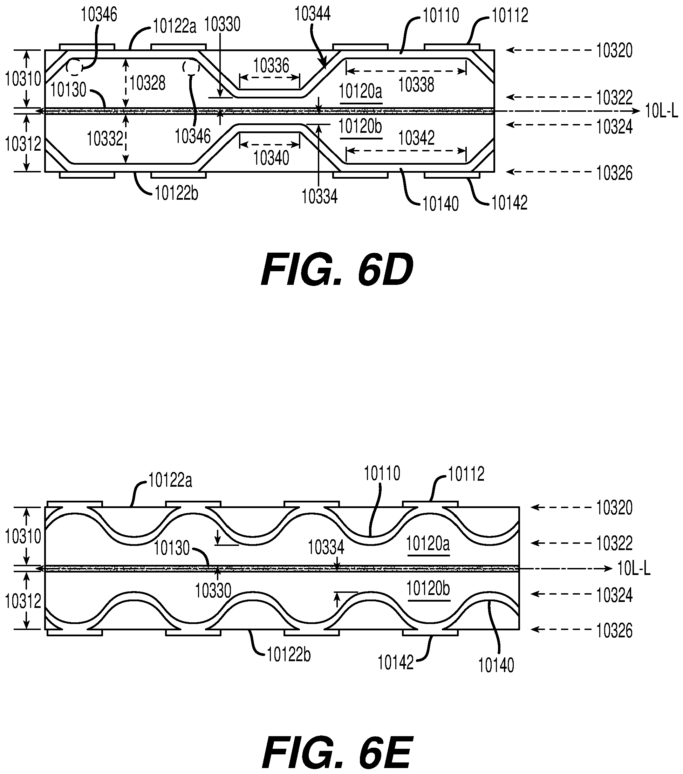

FIG. 6D shows a similar construct as shown in FIGs. 6B and 6C, but the respective circuits have longitudinal sections along the outermost planes. For example, and referring first to the first sheet of insulative material 10120a, the first flexible circuit 10110 includes a first superficial planar section 10338 that extends for a length parallel to the framework 10130 and within the first plane 10320. The first superficial planar section 10338 can be embedded with or partially within the insulative material 10120a as shown, or the first superficial planar section 10338 can extend along the first outer surface 10122a. The first superficial planar section 10338 enables more than one electrode on that first outer surface 10122a to be connected thereto before the first flexible circuit 10110 extends inwardly toward the second plane 10322. Referring now to the second sheet of insulative material 10120b, the second flexible circuit 10140 includes a second superficial planar section 10342 that extends for a length parallel to the framework 10130 and within the fourth plane 10326. The second superficial planar section 10342 can be embedded with or partially within the insulative material 10120b as shown, or the second superficial planar section 10342 can extend along the second outer surface 10122b. The second superficial planar section 10342 enables more than one electrode on that second outer surface 10122b to be connected thereto before the second flexible circuit 10140 extends inwardly toward the fourth plane 10324.

Referring to the bends between the planar sections (e.g., sections 10338 and 10342) and the angled sections 10344, a radius of curvature 10346 between the can be lengthened to prevent crimping of the flexible circuits. For example, an angle of the angled section can change as a first portion (e.g., the superficial planar section) bends or moves with respect to a second portion (e.g., the medial planar section). With a larger radius of curvature 10346, the flexible circuits 10110, 10140 can have more slack to move without crimping. In some examples, however, and as shown in FIG. 6E, the one or more flexible circuits 10110, 10140 may have a corrugate profile in or at least partially within the insulative material(s) 10120a, 10120b. Similar to the examples described above, the corrugate profile of the flexible circuits can have peaks and troughs. Referring to the first flexible circuit 10110 (which is on the top of the end effector 10100 shown in FIG. 6E), the "peaks" of the corrugate profile extend to the exposed or at least partially exposed electrodes 10112. Thus, the first plane 10320 of the "peaks" is either along the first outer surface 10122a or is proximate the first outer surface 10122a. The "troughs" of the first flexible circuit 10110 are at the second plane 10322, which can be touching the framework 10130 or, as shown, a third distance 10330 can be greater than zero to provide a gap between the first flexible circuit 10110 and the framework 10130. Referring to the second flexible circuit 10140 (which is on the bottom of the end effector 10100 shown in FIG. 6E), the "troughs" of the corrugate profile extend to the exposed or at least partially exposed electrodes 10142. Thus, the fourth plane 10326 of the "troughs" is either along the second outer surface 10122b or is proximate the second outer surface 10122b. The "peaks" of the second flexible circuit 10140 are at the third plane 10324, which can be touching the framework 10130 or, as shown, a fourth distance 10334 can be greater than zero to provide a gap between the second flexible circuit 10140 and the framework 10130.

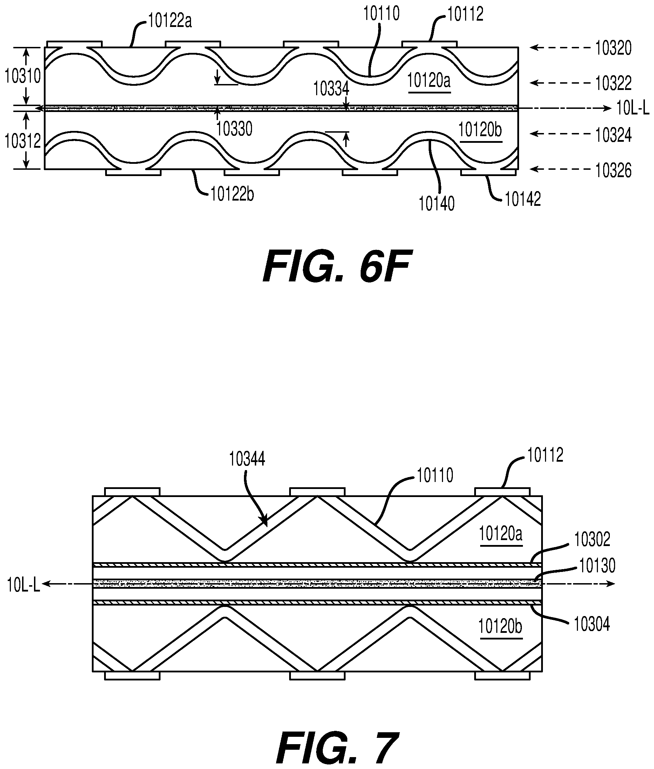

FIGs. 6E and 6F show substantially similar end effectors 10100, but the two designs differ in the placement of the electrodes 10120b. For instance, in FIG. 6E, the first plurality of electrodes 10112 are aligned directly opposite the second plurality of electrodes 10142. In FIG. 6F, a first position of the first plurality of electrodes 10112 is offset from a second position the second plurality of electrodes 10142 longitudinally along a length of the end effector 10110. This offsetting of the electrodes 10120b can provide increased and/or uniform surface area contact with targeted tissue.

As will be appreciated, any feature of the example end effectors 10100 described herein can be combined with any of the other features as needed to improve contact with targeted tissue, while also providing maneuverability and strength. FIG. 7 provides an example of such combination of features, as it shows an end effector 10100 with a first flexible circuit 10110 that extends from the surface electrodes 10112 in a first plane, then into the insulative material 10120a to a second plane, and then back to the surface electrodes 10112 in the first plane (e.g., as shown in FIG. 6A). In this example, the insulative material 10120a includes a first support layer 10302 embedded therein, and this first support layer 10302 can be substantially similar to the first support layer described above with reference to FIG. 5A. The end effector 10100 in FIG. 7 also includes a second flexible circuit 10140 that extends from the surface electrodes 10142 in a fourth plane, then into the insulative material 10120b to a third plane, and then back to the surface electrodes 10142 in the fourth plane (e.g., as shown in FIG. 6A). In this example, the insulative material 10120b includes a second support layer 10304 embedded therein, and the second support layer 10304 can be substantially similar to the second support layer described above with reference to FIG. 5A.

The present disclosure provides a catheter assembly 10200 as shown in FIG. 8 which can include a tubular member 10230 extending along a longitudinal axis 10L-L and configured to deliver end effector 10100 to an out of a sheath 10210. A physician 1024 can manipulate the catheter 200 with handle 10220. Appropriate examples for catheter assembly 10200 and its subcomponents such as handle 10220, sheath 10210, tubular member 10230, and others not mentioned herein are described in US Patent publication No. 2021/0369339, which is incorporated herein by reference and attached in the Appendix of Priority Application No. 63/615,600.

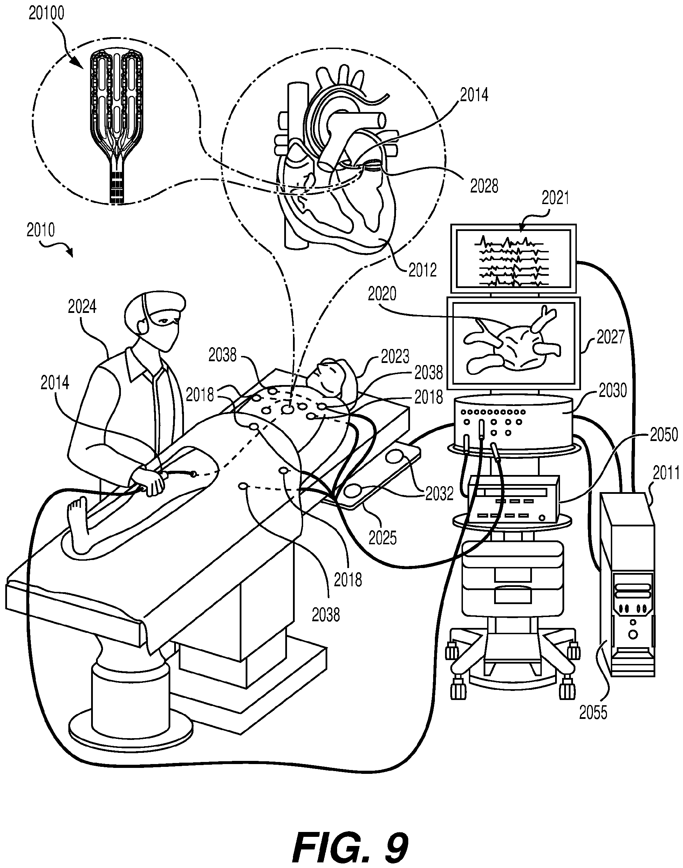

### End Effector With Offset Flexible Circuits (FIGs. 9-12B)

Reference is made to FIG. 9 showing an example catheter-based electrophysiology mapping and ablation system 2010. System 2010 includes multiple catheters, which are percutaneously inserted by physician 2024 through the patient's 2023 vascular system into a chamber or vascular structure of a heart 2012. Typically, a delivery sheath catheter is inserted into the left or right atrium near a desired location in heart 2012. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 2014 that is configured for sensing IEGM is illustrated herein. Physician 2024 brings a catheter shaft 2090 with distal tip of catheter 2014 (i.e., end effector 20100) into contact with the heart wall for sensing a target site in heart 2012. For ablation, physician 2024 would similarly bring a distal end of an ablation catheter to a target site for ablating.

Catheter 2014 is an exemplary catheter that includes one and preferably multiple electrodes 2026 optionally distributed over end effector 20100 coupled to a catheter shaft 2090 and configured to sense the IEGM signals as described in more detail below. Catheter 2014 may additionally include a position sensor embedded in or near end effector 20100 for tracking position and orientation of end effector 20100. Optionally and preferably, position sensor is a magnetic based position sensor including multiple magnetic coils for sensing three-dimensional (3D) position and orientation.

Magnetic based position sensor may be operated together with a location pad 2025 including a plurality of magnetic coils 2032 configured to generate magnetic fields in a predefined working volume. Real time position of end effector 20100 of catheter 2014 may be tracked based on magnetic fields generated with location pad 2025 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 2010 includes one or more electrode patches 2038 positioned for skin contact on patient 2023 to establish location reference for location pad 2025 as well as impedance-based tracking of electrodes 2026. For impedance-based tracking, electrical current is directed toward electrodes 2026 and sensed at electrode skin patches 2038 so that the location of each electrode can be triangulated via the electrode patches 2038. Details of the impedance-based location tracking technology are described in US Patent Nos. 7,536,218; 7,756,576; 7,848,787; 7,869,865; and 8,456,182, each of which are incorporated herein by reference.

A recorder 2011 displays electrograms 2021 captured with body surface ECG electrodes 2018 and intracardiac electrograms (IEGM) captured with electrodes 2026 of catheter 2014. Recorder 2011 may include pacing capability for pacing the heart rhythm and/or may be electrically connected to a standalone pacer.

System 2010 may include an ablation energy generator 2050 that is adapted to conduct ablative energy to one or more of electrodes 2026 at a distal tip of a catheter configured for ablating. Energy produced by ablation energy generator 2050 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 2030 is an interface configured to establish electrical communication between catheters, electrophysiological equipment, power supply and a workstation 2055 for controlling operation of system 2010. Electrophysiological equipment of system 2010 may include for example, multiple catheters, location pad 2025, body surface ECG electrodes 2018, electrode patches 2038, ablation energy generator 2050, and recorder 2011. Optionally and preferably, PIU 2030 additionally includes processing capability for implementing real-time computations of location of the catheters and for performing ECG calculations.

Workstation 2055 includes memory, processor unit with memory or storage with appropriate operating software loaded therein, and user interface capability. Workstation 2055 may provide multiple functions, optionally including (1) modeling the endocardial anatomy in three-dimensions (3D) and rendering the model or anatomical map 2020 for display on a display device 2027, (2) displaying on display device 2027 activation sequences (or other data) compiled from recorded electrograms 2021 in representative visual indicia or imagery superimposed on the rendered anatomical map 2020, (3) displaying real-time location and orientation of multiple catheters within the heart chamber, and (4) displaying on display device 2027 sites of interest such as places where ablation energy has been applied. One commercial product embodying elements of the system 2010 is available as the CARTO^{™} 3 System, available from Biosense Webster, Inc., 31 Technology Drive, Suite 200, Irvine, CA 92618.

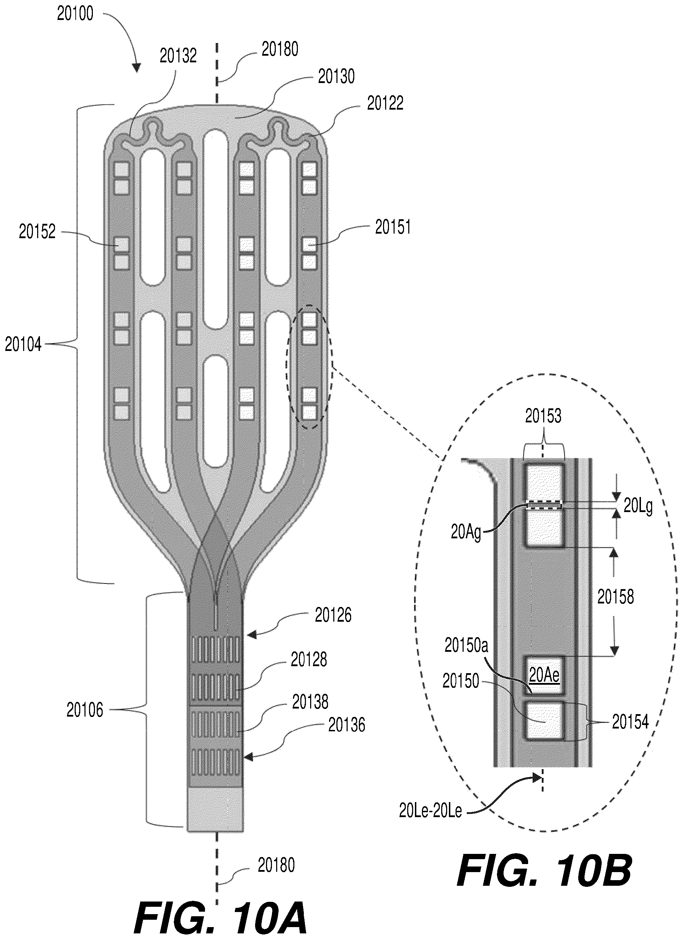

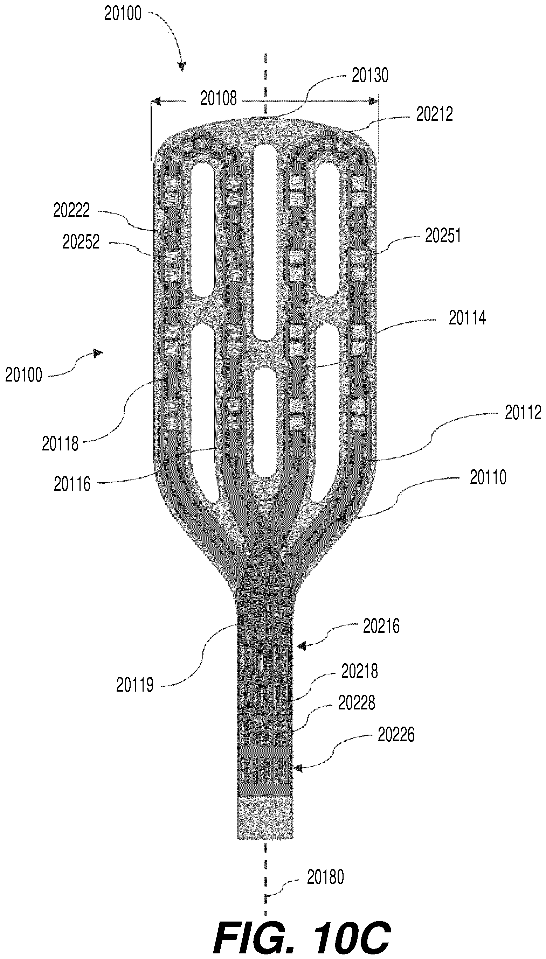

FIGs. 10A and 10C are schematic pictorial illustrations depicting a multi-layered end effector 20100 that is generally flat/planar in form. In order to achieve the desired stiffness, the mapping resolution, electrode contact with target anatomy, and conformity of the end effector 20100 disclosed herein to flat, curved, irregular and/or nonplanar tissue surfaces found in the target anatomy, the end effector 20100 has at least a planar spine framework 20110, flexible circuits (e.g., flexible circuits 20122, 20132, 20212, 20222), and an insulative material forming a substrate 20130. In some examples, the substrate 20130 can include a suitable substrate such as for example, a polymer material to ensure that the flexible circuits are coupled to the substrate 20130 as well as to ensure that no sharp edges of the flexible circuits are exposed.

The end effector 20100 can extend along the longitudinal axis 20180 from a proximal portion 20106 to a distal portion 20104. In some examples, the end effector 20100 comprises one or more flexible circuits disposed on a substrate 20130 of the end effector 20100. The flexible circuits can extend along the longitudinal axis 20180 from the proximal portion 20106 to the distal portion 20104 of the end effector 20100. In some examples, the flexible circuits layer can be made primarily of polyimide. In other examples, it can be made of any of biocompatible polyimides, glass-reinforced epoxy laminate materials, copper, or graphene, alone or in combination. The flexible circuit layer(s) can include conductive traces. As used herein, the term "flexible circuit" includes thin-film circuit, flexible printed circuit board, thin film deposition via lithography and etching processes on substrate such as polyimide or even a nitinol substrate. Each flexible circuit can comprise a plurality of electrodes provided on a distal portion 20104 of the end effector 20100. Electrical contacts corresponding to each electrode can be provided in a proximal portion of the circuit, which may be disposed in a proximal portion of the end effector. Traces provided within each circuit may provide the electrical contacts in electrical communication with the electrodes of the circuit, such that the electrical contacts can transmit electrical signals to and from the electrodes.

In some examples, the substrate 20130 comprises an insulative material. The frame 20110 of the end effector 20100 can be disposed in the substrate 20130 and can be approximately planar along a longitudinal axis 20180 such that the longitudinal axis 20180 is parallel to or coincident with a plane of the frame 20110. The longitudinal axis 20180 can be parallel or coaxial with a longitudinal center of the frame 20110, such that the longitudinal axis divides width 108 of the end effector equally. In some examples, the frame 20110 is formed from a flexible, resilient material. By way of example, the frame 20110 can be formed from a shape-memory alloy such as nickel-titanium, also known as Nitinol, cobalt chromium, stainless steel, and/or other alloys that exhibit pseudoelastic properties.

The substrate 20130 can be formed from an insulative material and can at least partially encapsulate and separate the frame 20110 and the flexible circuits. The substrate 20130 can also serve to enhance the atraumaticity of the end effector 20100 and to protect the subject from sharp edges.

FIG. 10A depicts a first side of an exemplary end effector 20100 comprising a first flexible circuit 20122 and a second flexible circuit 20132. In some examples, the first flexible circuit 20122 and the second flexible circuit 20132 are provided on a first side of the substrate 20130. In some examples, the first flexible circuit 20122 and the second flexible circuit 20132 are provided on the same side of the substrate 20130. In some examples, in the distal portion 20104 of the end effector 20100, the first flexible circuit 20122 is provided opposite of the second flexible circuit 20132, relative to longitudinal axis 20180. In some examples, the first flexible circuit 20122 comprises one or more electrodes 20151 provided on the distal portion 20104 of the end effector 20100 and one or more electrical contacts 20128 provided on a proximal portion 20126 of the first flexible circuit 20122 and corresponding to the one or more electrodes 20151. The electrodes 20151 and the electrical contacts 20128 of the first flexible circuit 20122 may be referred to as the first electrodes 20151 and the first electrical contacts 20128, respectively. In some examples, the second flexible circuit 20132 comprises one or more electrodes 20152 provided on the distal portion 20104 of the end effector 20100 and one or more electrical contacts 20138 provided on a proximal portion 20136 of the second flexible circuit 20132 and corresponding to the one or more electrodes 20152. The electrodes 20152 and the electrical contacts 20138 of the second flexible circuit 20132 may be referred to as the second electrodes 20152 and the second electrical contacts 20138, respectively.

In some examples, to accommodate for the small area of the end effector 20100, the first flexible circuit 20122 and the second flexible circuit are coplanar in the distal portion 20104 of the end effector 20100 and stacked on top of one another in the proximal portion 20106 of the end effector 20100. In such an example, a proximal portion 20126 of the first flexible substrate 20122 can be stacked onto the proximal portion 20136 of the second flexible substrate 20132 at the proximal portion 20106 of the end effector 20100. The arrangement can provide the electrical contacts 20128 of first flexible circuit 20122 and the electrical contacts 20138 of the second flexible circuit 20132 to both be disposed on the proximal portion 20106 of the end effector 20100.

FIG. 10C depicts a second side of an exemplary end effector 20100 comprising a third flexible circuit 20212 and a fourth flexible circuit 20222. In some examples, the third flexible circuit 20212 and the fourth flexible circuit 20222 are provided on a second side of the substrate 20130. In some examples, the third flexible circuit 20212 and the fourth flexible circuit 20222 are provided on the same side of the substrate 20130. In some examples, in the distal portion 20104 of the end effector 20100, the third flexible circuit 20212 is provided opposite of the fourth flexible circuit 20222, relative to longitudinal axis 20180. In some examples, the third flexible circuit 20212 comprises one or more electrodes 20251 provided on the distal portion 20104 of the end effector 20100 and one or more electrical contacts 20218 provided on a proximal portion 20216 of the third flexible circuit 20212 and corresponding to the one or more electrodes 20251. The electrodes 20251 and the electrical contacts 20218 of the third flexible circuit 20212 may be referred to as the third electrodes 20251 and the third electrical contacts 20218, respectively. In some examples, the fourth flexible circuit 20222 comprises one or more electrodes 20252 provided on the distal portion 20104 of the end effector 20100 and one or more electrical contacts 20228 provided on a proximal portion 20226 of the fourth flexible circuit 20222 and corresponding to the one or more electrodes 20252. The electrodes 20252 and the electrical contacts 20228 of the fourth flexible circuit 20222 may be referred to as the fourth electrodes 20252 and the fourth electrical contacts 20288, respectively.

FIG. 10B is a schematic pictorial illustration showing a detail view of an exemplary electrode arrangement provided on the end effector 20100. In some examples, the electrodes 20150 of the end effector are arranged in pairs. The electrodes 20150 pair of each pair can be spaced apart by a first predetermined longitudinal distance 20Lg. Each pair of electrodes can be spaced apart from adjacent pairs of electrodes by a second predetermined longitudinal distance 20158. The second predetermined longitudinal distance 20158 can be greater than the first predetermined longitudinal distance 20Lg. In some examples, the first predetermined longitudinal distance 20Lg between electrodes 20150 of a pair of electrodes is about 100 microns. In some examples, the second predetermined longitudinal distance 20158 between adjacent pairs of electrodes 20150 is about 1300 microns. In some examples, each electrode 150 comprises a width 20153 of about 500 microns and a length 20154 of about 500 microns.

In some versions, the space gap distance 20Lg separating each electrode 20150 (also referred to herein as a microelectrode) of a pair ranges from about 50 microns to about 300 microns. In some versions, the space gap distance 20Lg separating each microelectrode (20150) of a pair ranges from about 100 to about 200 microns. In some versions, the space gap distance Lg separating each microelectrode 20150 of a pair is about 50 microns. Moreover, in some versions, each microelectrode 20150 itself may have a width 20153 ranging from about 50 microns to about 100 microns. Each pair of microelectrodes 20150 is spaced apart from an adjacent pair of microelectrodes 20150 by a distance of about 5.0 mm, with each microelectrode 20150 having a width 20153 of about 50 microns and a length 20154 of about 2.56 mm. In some

In the present example, the width 20153 of microelectrode 20150 is equal to the width 20153 of the other microelectrode 20150 of the pair of microelectrodes, and the length 20154 of a microelectrode 20150 is equal to the length 20154 of the other microelectrode 20150 of the pair of microelectrodes 20150. Microelectrodes 20150, 20150 thus both have the same surface area (length 20154 * width 20153) in the present example. In a preferred embodiment, the two microelectrodes 20150, 20150 are arranged such that the length 20154 extends parallel to the longitudinal axis 20180 of the structure to which microelectrodes 20150, 20150 are mounted such that the width 20153 extends generally parallel to the electrodes' longitudinal axis 20Le-20Le or generally perpendicular to the longitudinal axis 20180 of the structure to which microelectrodes 20150, 20150 are mounted. Microelectrodes 20150, 20150 are positioned such that microelectrodes 20150, 20150 are spaced apart from each other along the length of the structure to which microelectrodes 20150, 20150, such that the gap 20Lg extends between the two nearest surfaces 20150a, 20150a of the microelectrodes. For convenience of nomenclature with respect to this embodiment, the gap 20Lg is also taken to be substantially parallel to the longitudinal axis 20180 of the structure to which microelectrodes 20150, 20150 are mounted. While microelectrodes 20150, 20150 are rectangular in the present example, microelectrodes 20150, 20150 may instead have any other suitable shape.

It is noted that any arrangement of electrodes may require the pair of electrodes for all embodiments described herein to conform to the following empirical rules: (1) the length 20154 of the electrode must always be at least the same as the spacing gap 20Lg between the pair of electrodes, and (2) the ratio of the area of the spacing gap 20Ag to the surface area 20Ae of one electrode must be less than or equal to 1. In shorthand, this can be restated as: (1) 20154 ≥ 20Lg and (2) 20Ag/20Ae ≤ 1. In some versions, the gap distance 20Lg can be determined by a product of the one electrode area 20Ae, up to one millimeter squared, and a conversion factor of about 1.25 mm⁻¹ or less.

An exemplary frame 20110 of the end effector 20100 is depicted in FIG. 10C. In some examples, the frame 20110 of the end effector 20100 embedded in the substrate 20130. As shown, the frame 20110 can include a first spine 20112, a second spine 20114, a third spine 20116, and a fourth spine 20118 provided in the distal portion 20104 of the end effector 20100. The spines 20112, 20114, 20116, 20118 can extend from a base 20119, provided in the proximal section 20106 of the end effector 20100, along the longitudinal axis 20180. The spines 20112, 20114, 20116, 20118 can be provided to resist bending when a force, e.g., a torsional force, is applied thereto. Gaps can be provided between each of the spines to increase flexibility of the frame. These forces can occur at various times during use, such as when being inserted into an introducer. This design aids in preventing electrical interconnections on the flexible circuits from breaking.

In some examples, the distal portions of the first flexible circuit 20122 and the third flexible circuit 20212 can each include a loop disposed over the first spine and the second spine. In some examples, the distal portions of the second flexible circuit 20132 and the fourth flexible circuit 20222 each comprise a second loop disposed over the third spine and the fourth spine.

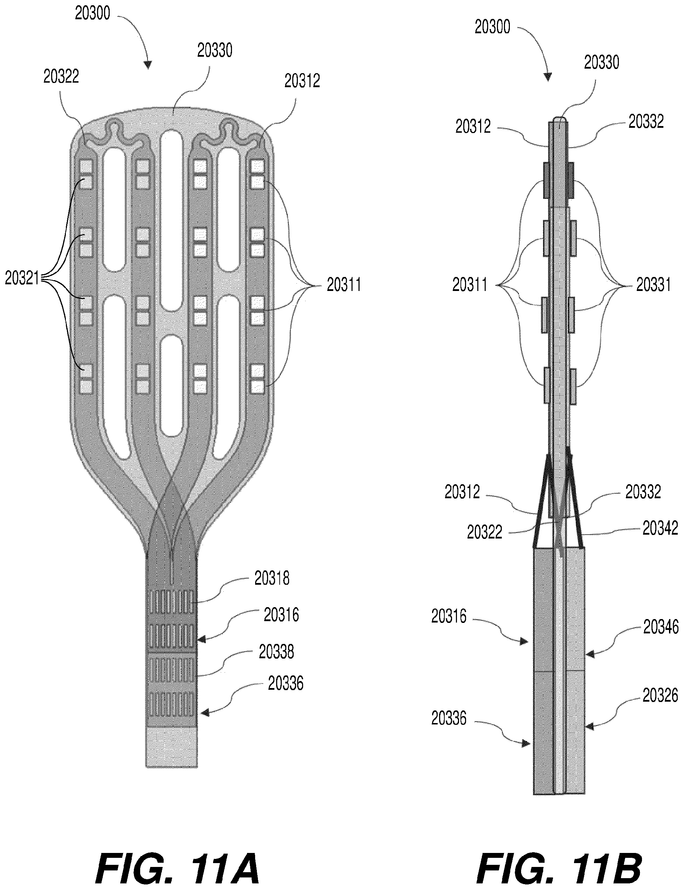

FIGs. 11A and 11B are schematic pictorial illustrations depicting a multi-layered end effector 20300 that is generally flat/planar in form, according to an example of the disclosed technology, with FIG. 11A being a front side view of end effector 20300 and FIG. 11B being a right-side view thereof. In some examples, the end effector 20300 comprises a first flexible circuit 20312, a second flexible circuit 20322, a third flexible circuit 20332, and a fourth flexible circuit 20342. In some embodiments, the first flexible circuit 20312 and the second flexible circuit 20322, and their corresponding electrodes 20311, 20321, are coplanar and provided on a first side of substrate 20330, and the third flexible circuit 20332 and the fourth flexible circuit 20342 are coplanar and provided on a second side of substrate 20330. Each circuit can comprise a proximal portion having electrical contacts corresponding to the electrodes of the circuit. For example, the first circuit 20312 comprises contacts 20318 corresponding to electrodes 20311, and the third circuit comprises contacts 20338 corresponding to electrodes 20331. Electrical contacts corresponding to the electrodes for each circuit can be provided in a proximal portion of the circuit. In some examples, the end effector 20300 is configured such that the proximal portion 20316 of the first circuit 20312 is provided on the same side of substrate 20330 as the proximal portion 20336 of the third circuit 20332 and the proximal portion 20326 of the second circuit 20322 is provided on the same side of substrate 20330 as the proximal portion 20346 of the fourth circuit 20342. As disclosed above, the proximal portion of the circuits may overlap, for example, the proximal portion 20316 of the first flexible circuit 20312 may be disposed on top of the proximal portion 20336 of the third circuit 20332, and the proximal portion 20346 of the fourth circuit 20342 may be disposed on top of the proximal portion 20326 of the second circuit 20342.

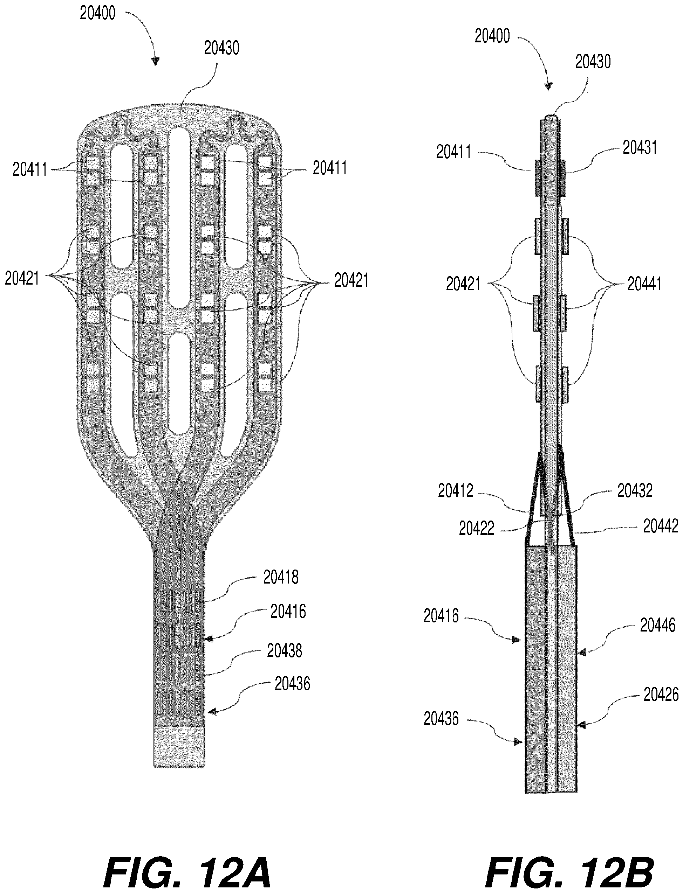

FIGs. 12A and 12B are schematic pictorial illustrations depicting a multi-layered end effector 20400 that is generally flat/planar in form, according to an example of the disclosed technology, with FIG. 12A being a front side view of end effector 20400 and FIG. 12B being a right-side view thereof. In some examples, the end effector 20400 comprises a first flexible circuit 20412 having electrodes 20411 disposed on a distal end of the end effector 20400. The end effector may further comprise a second flexible circuit 20422 having electrodes 20421 disposed proximal to the electrodes 20411 of the first circuit. In some examples, the first circuit 20412 and second circuit 20422 are coplanar in a distal portion of end effector 20400. In some examples, a proximal portion 20416 of the first circuit 20412 is provided on a side of the substrate 20430 opposite of the proximal portion 20426 of the second circuit 20422.

In some examples, the end effector 20400 further comprises a third flexible circuit 20432, and a fourth flexible circuit 20442. In some examples, the third flexible circuit 20432 comprises electrodes 20431 disposed on a distal end of the end effector 20400, and the fourth flexible circuit 20442 comprises electrodes 20441 disposed proximal to the electrodes 20431 of the third circuit. In some embodiments, the first flexible circuit 20412 and the second flexible circuit 20322 are coplanar and provided on a first side of substrate 20430, and the third flexible circuit 20432 and the fourth flexible circuit 20442 are coplanar and provided on a second side of substrate 20430. Each circuit can comprise a proximal portion having electrical contacts corresponding to the electrodes of the circuit. For example, the first circuit 20412 comprises proximal portion 20416 with contacts 20418 corresponding to electrodes 20411, and the third circuit comprises proximal portion 20436 contacts 20438 corresponding to electrodes 20431. Electrical contacts corresponding to the electrodes for each circuit can be provided in a proximal portion of the circuit. In some examples, the end effector 20300 is configured such that the proximal portion 20416 of the first circuit 20412 is provided on the same side of substrate 20430 as the proximal portion 20436 of the third circuit 20432 and the proximal portion 20426 of the second circuit 20422 is provided on the same side of substrate 20430 as the proximal portion 20446 of the fourth circuit 20442. As disclosed above, the proximal portion of the circuits may overlap, for example, the proximal portion 20416 of the first flexible circuit 20412 may be disposed on top of the proximal portion 20436 of the third circuit 20432 or vice versa. The proximal portion 20446 of the fourth circuit 20442 may be disposed on top of the proximal portion 20426 of the second circuit 20442 or vice versa. In some examples, the first and third circuits may be collectively referred to as a first circuit, and the second and fourth circuits may be collectively referred to as the second circuit.

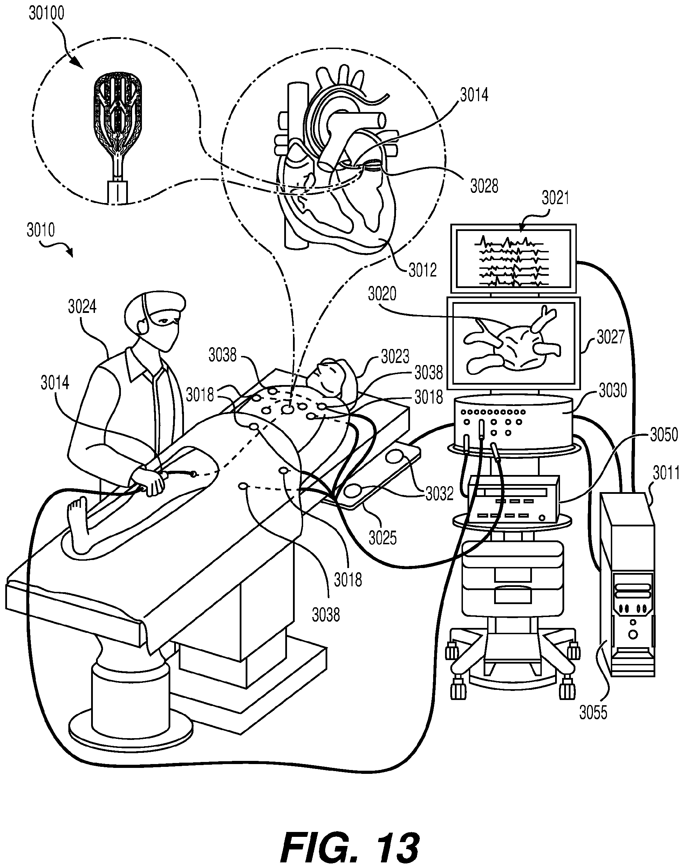

### Encapsulated Planar Catheter with Location Sensing Coils (FIGs. 13-20)

Reference is made to FIG. 13 showing an example catheter-based electrophysiology mapping and ablation system 3010. System 3010 includes multiple catheters, which are percutaneously inserted by physician 3024 through the patient's 3023 vascular system into a chamber or vascular structure of a heart 3012. Typically, a delivery sheath catheter is inserted into the left or right atrium near a desired location in heart 3012. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 3014 that is configured for sensing IEGM is illustrated herein. Physician 3024 brings a catheter shaft 3090 with distal tip of catheter 3014 (i.e., multilayered end effector 30100) into contact with the heart wall for sensing a target site in heart 3012. For ablation, physician 3024 would similarly bring a distal end of an ablation catheter to a target site for ablating.

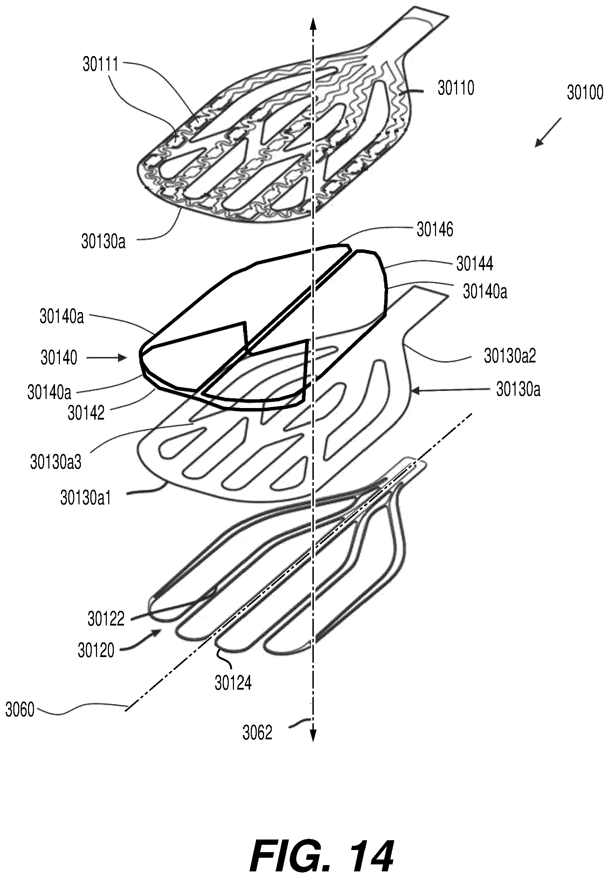

Catheter 3014 is an exemplary catheter that includes one and preferably multiple electrodes 3026 optionally distributed over end effector 30100 coupled to a catheter shaft 3090 and configured to sense the IEGM signals as described in more detail below. Catheter 3014 may additionally include a position sensor (as shown in FIG. 14B) embedded in or near end effector 30100 for tracking position and orientation of end effector 30100. Optionally and preferably, position sensor is a magnetic based position sensor including multiple magnetic coils for sensing three-dimensional (3D) position and orientation.

Magnetic based position sensor may be operated together with a location pad 3025 including a plurality of magnetic coils 3032 configured to generate magnetic fields in a predefined working volume. Real time position of end effector 30100 of catheter 3014 may be tracked based on magnetic fields generated with location pad 3025 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 3010 includes one or more electrode patches 3038 positioned for skin contact on patient 3023 to establish location reference for location pad 3025 as well as impedance-based tracking of electrodes 3026. For impedance-based tracking, electrical current is directed toward electrodes 3026 and sensed at electrode skin patches 3038 so that the location of each electrode can be triangulated via the electrode patches 3038. Details of the impedance-based location tracking technology are described in US Patent Nos. 7,536,218; 7,756,576; 7,848,787; 7,869,865; and 8,456,182, each of which are incorporated herein by reference.

A recorder 3011 displays electrograms 3021 captured with body surface ECG electrodes 3018 and intracardiac electrograms (IEGM) captured with electrodes 3026 of catheter 3014. Recorder 3011 may include pacing capability for pacing the heart rhythm and/or may be electrically connected to a standalone pacer.

System 3010 may include an ablation energy generator 3050 that is adapted to conduct ablative energy to one or more of electrodes 3026 at a distal tip of a catheter configured for ablating. Energy produced by ablation energy generator 3050 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 3030 is an interface configured to establish electrical communication between catheters, electrophysiological equipment, power supply and a workstation 3055 for controlling operation of system 3010. Electrophysiological equipment of system 3010 may include for example, multiple catheters, location pad 3025, body surface ECG electrodes 3018, electrode patches 3038, ablation energy generator 3050, and recorder 3011. Optionally and preferably, PIU 3030 additionally includes processing capability for implementing real-time computations of location of the catheters and for performing ECG calculations.

Workstation 3055 includes memory, processor unit with memory or storage with appropriate operating software loaded therein, and user interface capability. Workstation 3055 may provide multiple functions, optionally including (1) modeling the endocardial anatomy in three-dimensions (3D) and rendering the model or anatomical map 3020 for display on a display device 3027, (2) displaying on display device 3027 activation sequences (or other data) compiled from recorded electrograms 3021 in representative visual indicia or imagery superimposed on the rendered anatomical map 3020, (3) displaying real-time location and orientation of multiple catheters within the heart chamber, and (4) displaying on display device 3027 sites of interest such as places where ablation energy has been applied. One commercial product embodying elements of the system 3010 is available as the CARTO^{™} 3 System, available from Biosense Webster, Inc., 31 Technology Drive, Suite 200, Irvine, CA 92618.

FIG. 14 is a schematic pictorial illustration showing an exploded view of a portion of multi-layered end effector 30100 that is generally flat/planar in form. In order to achieve the desired stiffness, the mapping resolution, electrode contact with target anatomy, and conformity of the end effector 30100 disclosed herein to flat, curved, irregular and/or nonplanar tissue surfaces found in the target anatomy, the end effector 30100 has at least a flexible circuit layer 30110, a framework 30120, an insulative material 30130 that includes one or more flexible non-conductive layers 30130a, 30130b, and one or more location sensing loops 30140 that are stacked along a vertical axis 3062 the is substantially orthogonal to a longitudinal axis 3060 of the end effector. In FIG. 14, the flexible circuit 30110 is provided with a plurality of electrodes 30111 (e.g., generally planar electrodes) with its contact faces facing up in the figure. It should be noted that another flexible circuit 30110 can be provided below the framework 30120 with electrodes configured to face down in the figure.

End effector 30100 is illustrated as exploded in the orthogonal direction along the vertical axis 3062. While only one side (e.g., an upper side relative to the orientation shown in FIG. 13) of the end effector 30100 (relative to the framework 30120) is depicted, and as mentioned above, it will be appreciated that the opposite side (e.g., a lower side) of the end effector can also include other structure, such as, but not limited to, insulative material, one or more additional flexible circuits, and one or more additional location sensing loops.

The flexible circuit 30110 can extend along the longitudinal axis 3060 from a proximal portion (i.e., upper right section of FIG. 14) to a distal portion (i.e., lower left section of FIG. 14) of the end effector 30100. In some examples, the flexible circuit layer 30110 can be made primarily of polyimide. In other examples, it can be made of any of biocompatible polyimides, glass-reinforced epoxy laminate materials, copper, or graphene, alone or in combination. The flexible circuit layer(s) can include conductive traces. As used herein, the term "flexible circuit" includes thin-film circuit, flexible printed circuit board, thin film deposition via lithography and etching processes on substrate such as polyimide or even nitinol substrate.

The framework 30120 is disposed in the insulative material 30130 and is substantially planar along a longitudinal axis 3060 such that the longitudinal axis 3060 is parallel to or coincident with the framework 30120. Framework 30120 can include a first side 30122 (that forms a first planar surface) and an opposite second side 30124 (that forms a second planar surface) relative to the longitudinal axis 3060. The framework 30120 is also generally parallel with a plane defined by the flexible circuit 30110 such that the planar electrodes 30111 are aligned along at least one of the first planar surface or the second planar surface such that each electrode is spaced apart from the first planar surface or the second planar surface. In the example shown in FIG. 14, a plurality of location sensing loops 30140 are provided that can be sandwiched between the flexible circuit 30110 and the spine framework 30120 and generally parallel thereto. In some examples, the framework 30120 is formed from a flexible, resilient material. By way of example, the framework can be formed from a shape-memory alloy such as nickel-titanium, also known as Nitinol, cobalt chromium, stainless steel, and/or other alloys that exhibit pseudo-elastic and/or super-elastic properties.

A first non-conductive flexible layer 30130a can at least partially encapsulate and separate/space the framework 30120 and one side of the sensing loops 30140. Moreover, a second non-conductive flexible layer 30130b can at least partially encapsulate and separate the flexible circuit 30110 and another side of the sensing loops 30140. This insulative material 30130 also serves to enhance the atraumaticity of the end effector 30100 and to protect the subject from sharp edges.

Each sensing loop 30140 has one or more coils 30140a which each have a surface area (i.e., the area enclosed by the respective coil 30140a of the loop 30140). Moreover, each sensing loop 30140 has a cumulative surface area that is the product of its number of coils 30140a and the area enclosed by a single coil 30140a (in other words, the summation of all the surfaces areas of the coils in the loop 30140). The amount of energy each sensing loop 30140 receives, when subjected to a magnetic field, is proportionate to the size of the coil 30140a (e.g., proportionate to the cumulative surface area of the coil 30140a). The cumulative surface area of each loop 30140 defines a cumulative location sensing area for that loop 30140. In order for the magnetic field to work in order to most effectively track a location of the end effector 30100, the cumulative surface area in the presently disclosed technology is approximately one-hundred to three-hundred millimeters squared. In some examples, the cumulative surface area is at least approximately two-hundred millimeters squared.

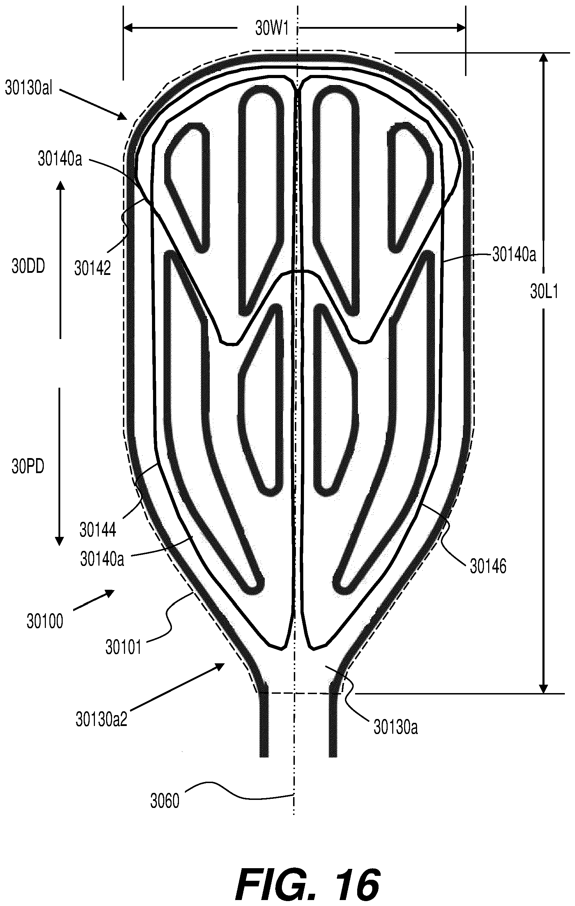

Making reference to FIGs. 14-16, the sensing loops 30140 of this example include a center loop 30142 and a pair of side loops 30144, 30146. In some examples (e.g., as seen in FIG. 14), the sensing loops 30140 can be coplanar. As seen best in FIGs. 14 and 16, the center loop 30142 partially overlaps with both the first loop 30144 and the second loop 30146 when viewed in a direction along the vertical axis 3062. However, it will be appreciated that, in other examples, the loops 30142, 30144, 30146 do not overlap, provided that the cumulative surface area of each loop 30142, 30144, 30146 falls within the range discussed above.

As previously discussed, each loop 30142, 30144, 30146 is configured to generate a current when subjected to a magnetic field. Each loop 30142, 30144, 30146 can comprise a conductive material formed into a loop of one or more coils 30140a. The loops 30142, 30144, 30146 can comprise electrical leads for conduction of current induced on each loop 30142, 30144, 30146 to the PIU 3030. As will be appreciated, by attaching a plurality of loops 30142, 30144, 30146 to the end effector 30100, it is possible to detect a position thereof. In this way, a physician 3024 can more accurately determine the position of the end effector 30100 before using it for purposes mentioned above.

Further to the above, the center loop 30142 is disposed over the longitudinal axis 3060 when viewed in a direction along the vertical axis 3062 (see FIG. 16, in particular). In this example, the center loop 30142 is disposed in a plane that is substantially parallel to and runs along the longitudinal axis 3060 such that it is has a symmetrical design relative thereto. In alternative examples, the center loop 30142 is not necessarily symmetrical relative to the longitudinal axis 3060. The center loop 30142 is also disposed over an area near a distal portion 30130a1 of the insulative material 30130 (as exemplified by its extent relative to first non-conductive flexible layer 30130a, as shown in FIGs. 14 and 16). The center loop 30142 conforms to a configuration of the legs/sections 30130a3 of the first non-conductive flexible layer 30130a.

In this example, the pair of side loops 30144, 30146 (referred to herein as a first side loop 30144 and a second side loop 30146) are disposed generally symmetrically about the longitudinal axis 3060. In other examples, the side loops 30144, 30146 are not necessarily symmetrical. Each are disposed within a plane. In the illustrated example of FIG. 14, the side loop planes are co-planar with one another and are parallel to or co-planar with the plane of the center loop 30142 (as well as a plane of the flexible circuit 30110 and a plane of the framework 30120). Each side loop 30144, 30146 extends from the proximal portion 30130a1 to a distal portion 30130a2 of the insulative material 30130, as exemplified by their extent relative to first non-conductive flexible layer 30130a in FIG. 16. The pair of side loops 30144, 30146 conform to a configuration of the legs/sections 30130a3 of the first non-conductive flexible layer 30130a.

Further to the above, each coil 30140a of the center loop 30142 has a center loop surface area 30143, while each coil 30140a of the first side loop 30144 has a first side loop surface area 30145 and each coil 30140a of the second side loop 30146 has a second side loop surface area 30147. For each loop 30142, 30144, 30146, the product of the loop surface area 30143, 145, 147 by its number of coils yields the aforementioned cumulative surface area of the loop 30142, 30144, 30146.

In some examples, the surface area 30143 of one coil 30140a of the center loop 30142 is at least approximately 69 millimeters squared. In some examples, the surface area 30145 of one coil 30140a of the first side loop 30144 and the surface area 30147 of one coil 30140a of the second side loop 30146 are each at least 59 millimeters squared. Due to the respective loops being wound such that they have multiple coils 30140a, the cumulative surface area of each loop 30142, 30144, 30146 can be made to fall in the range discussed above.

Further to the above, and referring to FIG. 16, the end effector 30100 has an overall length 30L1 (e.g., approximately 20 - 25mm) and an overall width 30W1 (e.g., approximately 9 - 12mm) and an overall area 30101 (i.e., an area of the planar surface generally defined by the end effector 30100 along the vertical axis 3062). In some examples, the overall area 30101 is approximately 100 millimeters squared to 350 millimeters squared.

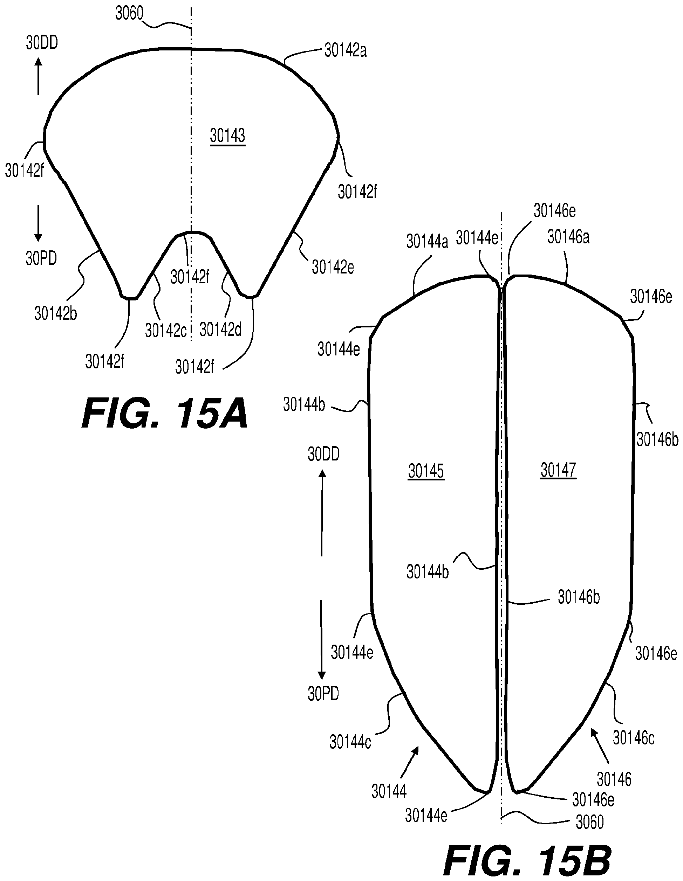

FIG. 15A is a schematic pictorial illustration showing the center loop 30142 of the end effector 30100 of FIG. 14. With specific reference to FIG. 15A, the center loop 30142 can include first through fifth legs 30142a-30142e. The first leg 30142a extends arcuately and defines a distal end of the center loop 30142. The second leg 30142b extends substantially linearly from the first leg 30142a in a proximal direction 30PD of the end effector 30100 and is angled (i.e., non-parallel) relative to the longitudinal axis 3060. The third leg 30142c extends substantially linearly from the second leg 30142b in a distal direction 30DD of the end effector 30100 and is angled (i.e., non-parallel) relative to the longitudinal axis 3060. The fourth leg 30142d extends substantially linearly from the third leg 30142c in the proximal direction of the end effector 30100 and is angled (i.e., non-parallel) relative to the longitudinal axis 3060. The fifth leg 30142e extends substantially linearly from the fourth leg 30142d in the distal direction 30DD of the end effector 30100, connects with the first leg 30142a to form the loop, and is angled (i.e., non-parallel) relative to the longitudinal axis 3060. Moreover, a plurality of center loop joint sections 30142f connect the respective aforementioned legs 30142a-30142e. Of course, it will be appreciated that the depicted center loop 30142 is exemplary may take a different shape/form in other implementations, without departing from the spirit and scope of the present disclosure.

FIG. 15B is a schematic pictorial illustration showing the two side loops 30144, 30146 of the end effector 30100 of FIG. 14. Compared with the center loop 30142, in this example, the two side loops 30144, 30146 are laterally offset from the longitudinal axis 3060 when viewed in a direction along the vertical axis 3062.

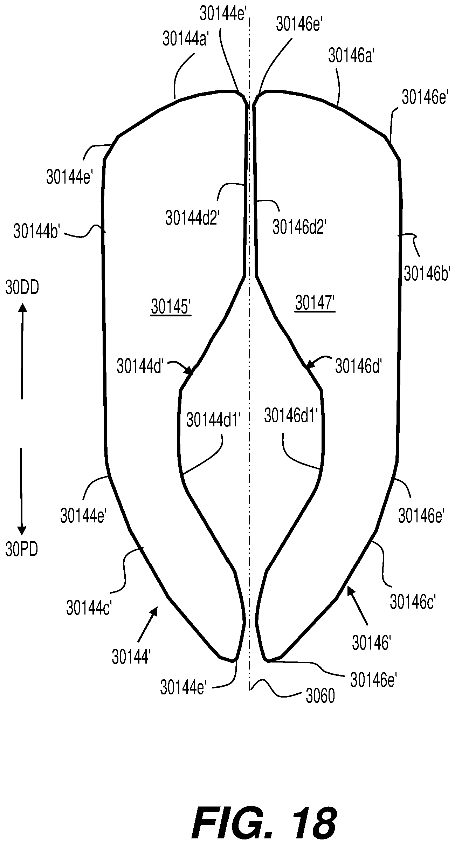

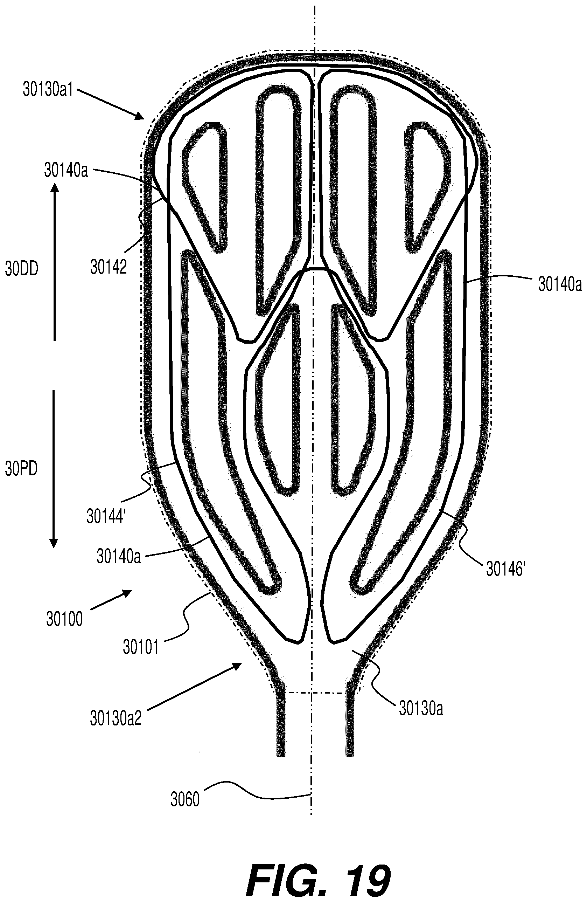

With specific reference to FIG. 15B, the first side loop 30144 can include sixth through ninth legs 30144a-30144d (the numbering being continued from the center loop 30142 numbering to clearly differentiate in the description). The sixth leg 30144a defines a distal end of the first side loop 30144 and extends arcuately. The seventh leg 30144b extends linearly from the sixth leg 30144a in the proximal direction PD of the end effector 30100 and is substantially parallel with the longitudinal axis 3060. The eighth leg 30144c extends arcuately from the seventh leg 30144b in the proximal direction 30PD of the end effector 30100. The ninth leg 30144d extends linearly from the eighth leg 30144c in the distal direction 30DD of the end effector 30100, is substantially parallel with the longitudinal axis 3060, and connects with the sixth leg 30144a to form the loop. Moreover, a plurality of first side loop joint sections 30144e connect the respective aforementioned legs 30144a-30144d. As best seen in FIGs. 14 and 16, all the legs 30144a-30144d of the first side loop 30144 are designed such that they run along legs/sections 30130a3 (see FIG. 14) of the insulative material 30130. Of course, it will be appreciated that the depicted first side loop 30144 is exemplary may take a different shape/form in other implementations (such as discussed below with respect to FIGs. 18 and 19), without departing from the spirit and scope of the present disclosure.

With continued reference to FIG. 15B, the second side loop 30146 can include tenth through thirteenth legs 30146a-30146d (the numbering being continued from the first side loop 30144 numbering to clearly differentiate in the description). As mentioned above, the second side loop 30146 is symmetrical with the first side loop 30144 relative to the longitudinal axis 3060. In other words, the second side loop 30146 is a mirror image of the first side loop 30144 relative to the longitudinal axis, in this example. However, note that non-symmetric and symmetry over different planes are contemplated examples. The tenth leg 30146a defines a distal end of the second side loop 30146 and extends arcuately. The eleventh leg 30146b extends linearly from the tenth leg 30146a in the proximal direction 30PD of the end effector 30100. The twelfth leg 30146c extends arcuately from the eleventh leg 30146b in the proximal direction 30PD of the end effector 30100. The thirteenth leg 30146d extends linearly from the twelfth leg 30146c in the distal direction 30DD of the end effector 30100, is substantially parallel with the longitudinal axis 3060, and connects with the tenth leg 30146a to form the loop. Moreover, a plurality of second side loop joint sections 30146e connect the respective aforementioned legs 30146a-30146d. As best seen in FIG. 16, all the legs 30146a-30146d of the second side loop 30146 are designed such that they run along legs/sections 30130a3 of the insulative material 30130. Of course, it will be appreciated that the depicted second side loop 30146 is exemplary may take a different shape/form in other implementations (such as discussed below with respect to FIGs. 18 and 19), without departing from the spirit and scope of the present disclosure.

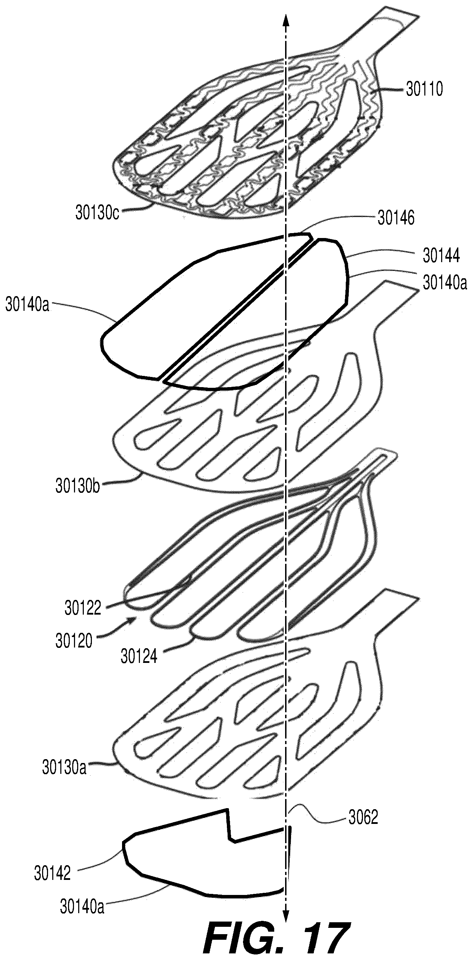

FIG. 17 is a schematic pictorial illustration showing an exploded view of a portion of a modified configuration of the end effector 30100 of FIG. 14. The example of FIG. 17 can be embodied and function identically to that of the foregoing description of FIGs. 14-16, with the exception of the layering of the center loop 30142 and the side loops 30144, 30146. Repetitive description of this example is, therefore, omitted from this disclosure. As shown in FIG. 17, rather than providing the positioning sensing loops 30140 on the same side of the framework 30120 along the vertical axis 3062 in the same layer, the loops 30140 can be positioned in different layers and/or on opposing sides/planes relative to the plane of the framework 30120.

As illustrated in FIG. 17, a first non-conductive flexible layer 30130a can at least partially encapsulate and separate a lower side of the framework 30120 and the center loop 30142. A second non-conductive flexible layer 30130b can at least partially encapsulate and separate the one side of the side loops 30142, 30144 and an upper side of the framework. Further, a third non-conductive flexible layer 30130c can at least partially encapsulate and separate the other side of the side loops 30142, 30144 and the flexible circuit 30110. Of course, other layered configurations can be employed without departing from the spirit and the scope of the present disclosure.

Turning now to FIGs. 18 and 19, a variant configuration of the side loops 30144', 30146' is shown. It is identical to the examples described above with respect to FIGs. 14-17, with the exception of the routing of the ninth leg 30144d' and the thirteenth leg 30146d'. In this example, the ninth leg 30144d' includes a proximal section 30144d1' that is non-parallel with the longitudinal axis 3060 and a distal section 30144d2' that is substantially parallel with the longitudinal axis 3060 (FIG. 18B), with the proximal section 30144d1' bowing away from the longitudinal axis 3060. Otherwise, it will be appreciated that the other legs/joints 30144a'-30144c', 30144e' and 30146a'-30146c', 30146e' may be respectively configured the same as their counterparts 30144a-30144c, 30144e and 30146a-30146c, 30146e in the previously described example (and as shown in FIGs. 15B and 16).

The presently described examples employ the use of three loops 30140. Of course, it will be appreciated that any number of loops (e.g., 1, 2, 3, 4, 5, etc.) may be employed without departing from the spirit and scope of the present disclosure, provided that the cumulative surface area of each loop 30140 (that each includes one or more coils 30140a) falls within the range discussed above.

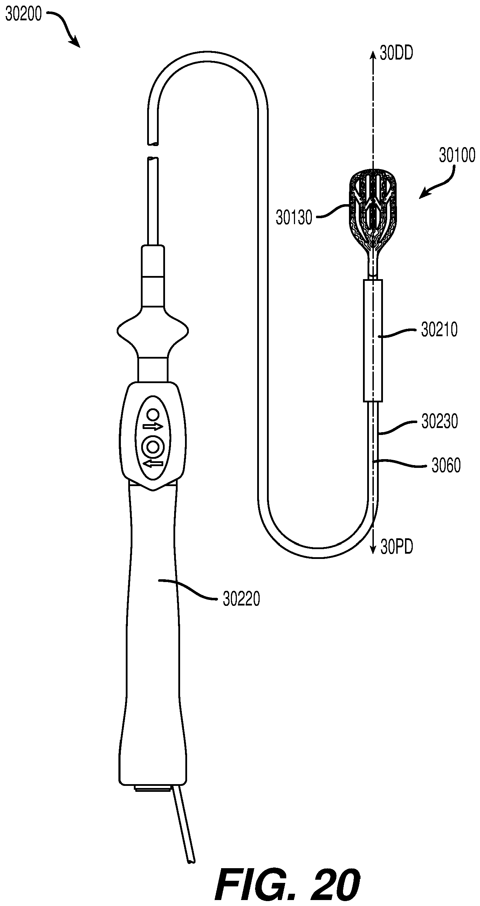

The present disclosure provides a catheter assembly 30200 as shown in FIG. 20 which can include a tubular member 30230 extending along a longitudinal axis 3060 and configured to deliver end effector 30100 to and out of a sheath 30210. A physician 3024 can manipulate the catheter 30200 with handle 30220. Appropriate examples for catheter assembly 30200 and its subcomponents such as handle 30220, sheath 30210, tubular member 30230, and others not mentioned herein are described in US Patent publication No. 2021/0369339, which is incorporated herein by reference.

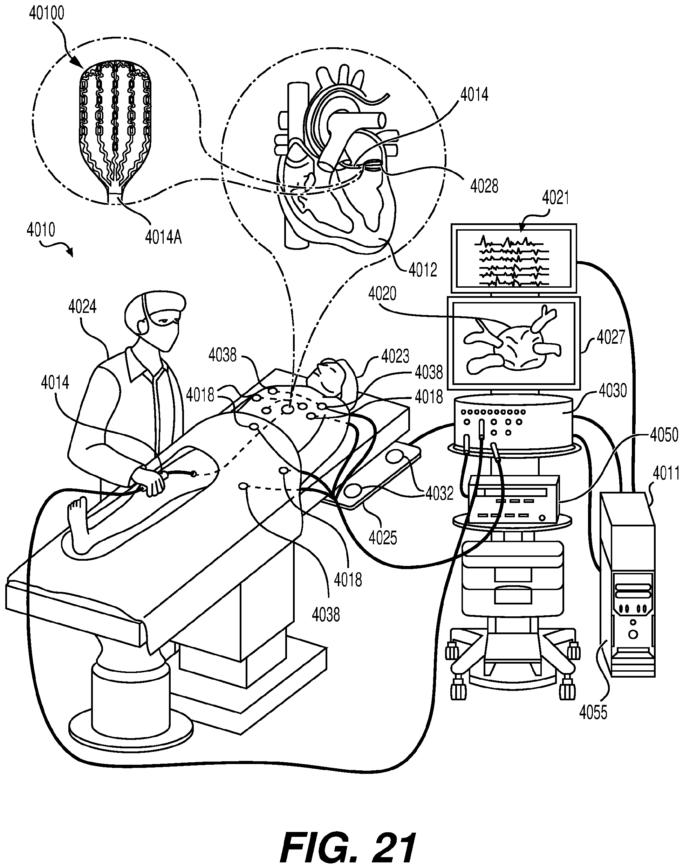

### Encapsulated Catheter with Framework (FIGs. 21-42)

Reference is made to FIG. 21 showing an example catheter-based electrophysiology mapping and ablation system 4010. System 4010 includes multiple catheters, which are percutaneously inserted by physician 4024 through the patient's 4023 vascular system into a chamber or vascular structure of a heart 4012. Typically, a delivery sheath catheter is inserted into the left or right atrium near a desired location in heart 4012. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 4014 that is configured for sensing IEGM is illustrated herein. Physician 4024 brings a catheter shaft 4090 with distal tip of catheter 4014 (i.e., multilayered end effector 40100) into contact with the heart wall for sensing a target site in heart 4012. For ablation, physician 4024 would similarly bring a distal end of an ablation catheter to a target site for ablating.

Catheter 4014 is an exemplary catheter that includes one and preferably multiple electrodes 4026 optionally distributed over end effector 40100 coupled to a catheter shaft 4090 and configured to sense the IEGM signals as described in more detail below. Catheter 4014 may additionally include a position sensor embedded in or near end effector 40100 for tracking position and orientation of end effector 40100. Optionally and preferably, position sensor is a magnetic based position sensor including multiple magnetic coils for sensing three-dimensional (3D) position and orientation.

Magnetic based position sensor may be operated together with a location pad 4025 including a plurality of magnetic coils 4032 configured to generate magnetic fields in a predefined working volume. Real time position of end effector 40100 of catheter 4014 may be tracked based on magnetic fields generated with location pad 4025 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 4010 includes one or more electrode patches 4038 positioned for skin contact on patient 4023 to establish location reference for location pad 4025 as well as impedance-based tracking of electrodes 4026. For impedance-based tracking, electrical current is directed toward electrodes 4026 and sensed at electrode skin patches 4038 so that the location of each electrode can be triangulated via the electrode patches 403 8. Details of the impedance-based location tracking technology are described in US Patent Nos. 7,536,218; 7,756,576; 7,848,787; 7,869,865; and 8,456,182, each of which are incorporated herein by reference.

A recorder 4011 displays electrograms 4021 captured with body surface ECG electrodes 4018 and intracardiac electrograms (IEGM) captured with electrodes 4026 of catheter 4014. Recorder 4011 may include pacing capability for pacing the heart rhythm and/or may be electrically connected to a standalone pacer.

System 4010 may include an ablation energy generator 4050 that is adapted to conduct ablative energy to one or more of electrodes 4026 at a distal tip of a catheter configured for ablating. Energy produced by ablation energy generator 4050 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 4030 is an interface configured to establish electrical communication between catheters, electrophysiological equipment, power supply and a workstation 4055 for controlling operation of system 4010. Electrophysiological equipment of system 4010 may include for example, multiple catheters, location pad 4025, body surface ECG electrodes 4018, electrode patches 4038, ablation energy generator 4050, and recorder 4011. Optionally and preferably, PIU 4030 additionally includes processing capability for implementing real-time computations of location of the catheters and for performing ECG calculations.

Workstation 4055 includes memory, processor unit with memory or storage with appropriate operating software loaded therein, and user interface capability. Workstation 4055 may provide multiple functions, optionally including (1) modeling the endocardial anatomy in three-dimensions (3D) and rendering the model or anatomical map 4020 for display on a display device 4027, (2) displaying on display device 4027 activation sequences (or other data) compiled from recorded electrograms 4021 in representative visual indicia or imagery superimposed on the rendered anatomical map 4020, (3) displaying real-time location and orientation of multiple catheters within the heart chamber, and (4) displaying on display device 4027 sites of interest such as places where ablation energy has been applied. One commercial product embodying elements of the system 4010 is available as the CARTO^{™} 3 System, available from Biosense Webster, Inc., 31 Technology Drive, Suite 200, Irvine, CA 92618.

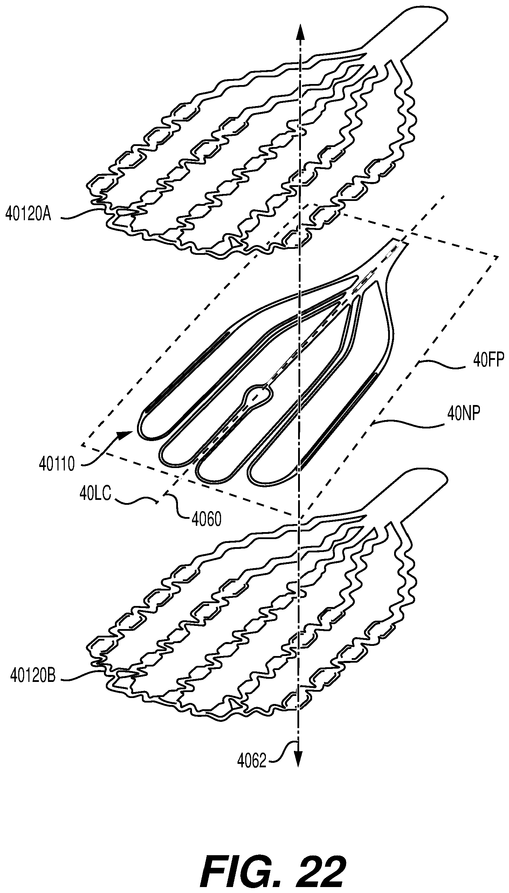

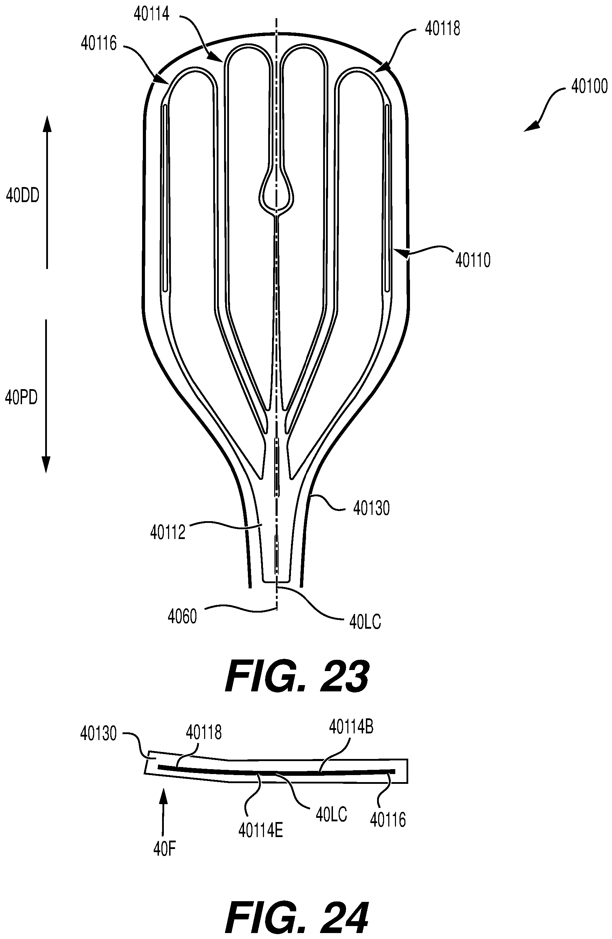

FIG. 22 is a schematic pictorial illustration showing an exploded view of a portion of multi-layered end effector 40100 that is generally flat in form. In order to achieve the desired stiffness, the mapping resolution, electrode contact with target anatomy, and conformity of the end effector 40100 disclosed herein to flat, curved, irregular and/or non-tissue surfaces found in the target anatomy, the end effector 40100 has a flexible circuit top layer 40120A, a framework 40110, flexible bottom circuit layer 40120B with an insulative material 40130 (see FIG. 23) partially encapsulating the circuit layers and framework while keeping these components separated from each other in orthogonal direction relative to the longitudinal axis 4060.

It will be appreciated that the end effector can also include other structure, such as, but not limited to one or more location sensing loops (not shown).

The flexible circuit 40120A or 40120B can extend along the longitudinal axis 4060 from a proximal portion (i.e., upper right section of circuit 40120 in FIG. 22) to a distal portion (i.e., lower left section of circuit 40120 in FIG. 22) of the end effector 40100. In some examples, the flexible circuit layer 40120 can be made primarily of polyimide. In other examples, it can be made of any of biocompatible polyimides, glass-reinforced epoxy laminate materials, copper, or graphene, alone or in combination. The flexible circuit layer(s) 40120 can include conductive traces. As used herein, the term "flexible circuit" includes thin-film circuit, flexible printed circuit board, thin film deposition via lithography and etching processes on substrate such as polyimide or even nitinol substrate. For ease of understanding, either or both flexible circuits 40120A and 40120B will be referred to as flexible circuit 40120 in the following description.

The framework 40110 is disposed in the insulative material 40130 (see FIG. 23) and is approximately along a longitudinal axis 4060 such that the longitudinal axis 4060 is parallel to or coincident with a framework plane 40FP of the framework 40110. The longitudinal axis 4060 is parallel or coaxial with a longitudinal center LC of the framework 40110, the longitudinal center 40LC being an imaginary straight line that divides the framework 40110 equally in a width direction thereof. The framework plane 40FP is also generally parallel with a plane defined by the flexible circuit 40120. In some examples, the framework 40110 is formed from a super elastic material. By way of example, the framework 40110 can be formed from a shape-memory alloy such as nickel-titanium, also known as Nitinol, cobalt chromium, stainless steel, and/or other alloys that exhibit pseudo-elastic and/or super-elastic properties.

A first non-conductive flexible layer 40130A of the insulative material 40130 can at least partially encapsulate and separate the framework 40110 and the flexible circuit 40120. Moreover, a second non-conductive flexible layer 40130B can at least partially encapsulate and separate the flexible circuit 40120 and another side of the sensing loops 40140. This insulative material 40130 also serves to enhance the atraumaticity of the end effector 40100 and to protect the subject from sharp edges.

FIG. 23 is a schematic pictorial illustration showing the framework 40110 of the end effector 40100 embedded in insulative material 40130. As shown, the framework 40110 includes a base 40112, a central first spine loop 40114, and two side spines 40116, 40118 (i.e., a left spine 40116 and a right spine 40118) on opposing lateral sides of the first spine loop 40114. The base 40112 connects with an elongated shaft 4014A (FIG. 21) of the medical device 4014 and extends along the longitudinal axis 4060. The spines 40114, 40116, 40118 extend from the base 40112 along the longitudinal axis 4060.

As seen in FIG. 24, the central first spine loop 40114 is configured to increase the stiffness along the center of the framework 40110 and to resist bending when a force 40F, e.g., a torsional force, is applied thereto. These forces 40F can occur at various times during use, such as when being inserted into an introducer. This design aids in preventing electrical interconnections on the flexible circuit 40120 that run along the longitudinal center 40LC from breaking.

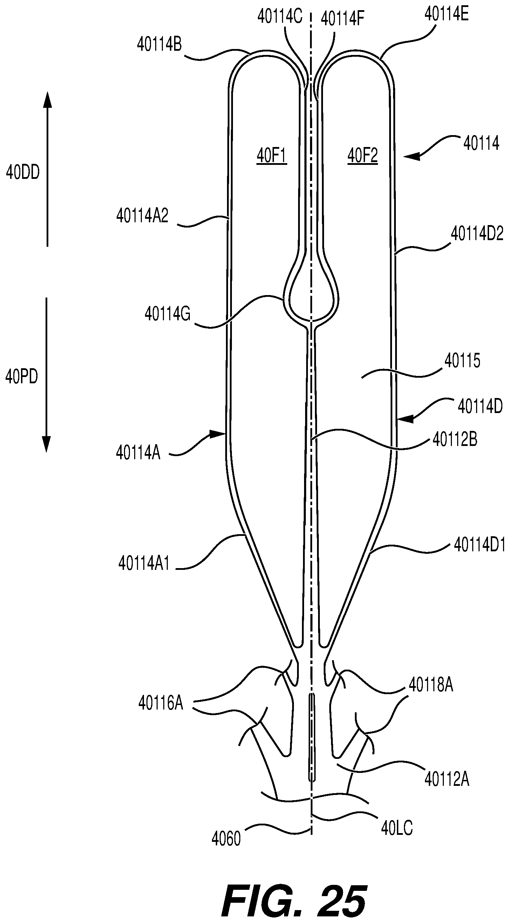

Further to the above, and with reference to FIG. 25, the first spine loop 40114, which includes features that resist bending, is shown in greater detail. The first spine loop 40114 includes first through seventh segments 40114A-40114G. The curvilinear first segment 40114A is connected to the base 40112 and extends from the base 40112 in a distal direction 40DD along the longitudinal axis 4060. The curvilinear first segment 40114A includes (1) a first section 40114A1 directly connected to the base 40112 that is angled relative to the longitudinal axis 4060 and (2) a second section 40114A2 connected to the first section 40114A1 that is generally parallel to the longitudinal axis 4060. The second segment 40114B is arcuate and is connected to and extends from the curvilinear first segment 40114A. The third segment 40114C is connected to and extends from the second segment 40114B in a proximal direction 40PD along the longitudinal axis 4060. The fourth segment 40114D2 curvilinear fourth segment 40114D is connected to and extends from the base 40112 in the distal direction 40DD along the longitudinal axis 4060. Like the curvilinear first segment 40114A, the fourth segment 40114D2 curvilinear fourth segment 40114D includes (1) a first section 40114D1 directly connected to the base 40112 that is angled relative to the longitudinal axis 4060 and (2) a second section 40114D2 connected to the first section 40114D1 that is generally parallel to the longitudinal axis 4060. The fifth segment 40114E is arcuate and is connected to and extends from the fourth segment 40114D2 curvilinear fourth segment 40114D. The sixth segment 40114F is connected to and extends from the fifth segment 40114E in the proximal direction 40PD along the longitudinal axis 4060. The seventh segment 40114G is arcuate (resembling the form of a teardrop), is bisected by the longitudinal axis 4060, and connects the third segment 40114C and the sixth segment 40114F to aid in the aforementioned bend resistance.

Cumulatively, the segments 40114A-40114G of the first spine loop 40114 defines an opening 40115 that includes two finger shaped openings 40F1, 40F2 (therefore, it is noted that opening 40115 is also referred to herein as a finger shaped opening since it comprises two openings of this form). Moreover, the curvilinear first segment 40114A, the second segment 40114B, and the third segment 40114C are symmetrical with the fourth segment 40114D2 curvilinear fourth segment 40114D, the fifth segment 40114E, and the sixth segment 40114F relative to the longitudinal axis 4060 and longitudinal center 40LC.

The base 40112 includes (1) a first section 40112A that connects with the elongated shaft 4014A and (2) a second section 40112B that extends within the finger shaped opening 40115 to connect with the seventh segment 40114G. Thus, the second section 40112B effectively splits the finger shaped opening 40115 into two halves (i.e., the two finger shaped openings 40F1, 40F2.

As particularly seen in FIG. 25, the second spine loop 40116 is connected to the first section 40112A of the base 40112 via a pair of terminal sections 40116A. Similarly, the third spine loop 118 is connected to an opposite side of the first section 40112A of the base 40112 via a pair of terminal sections 40118A.

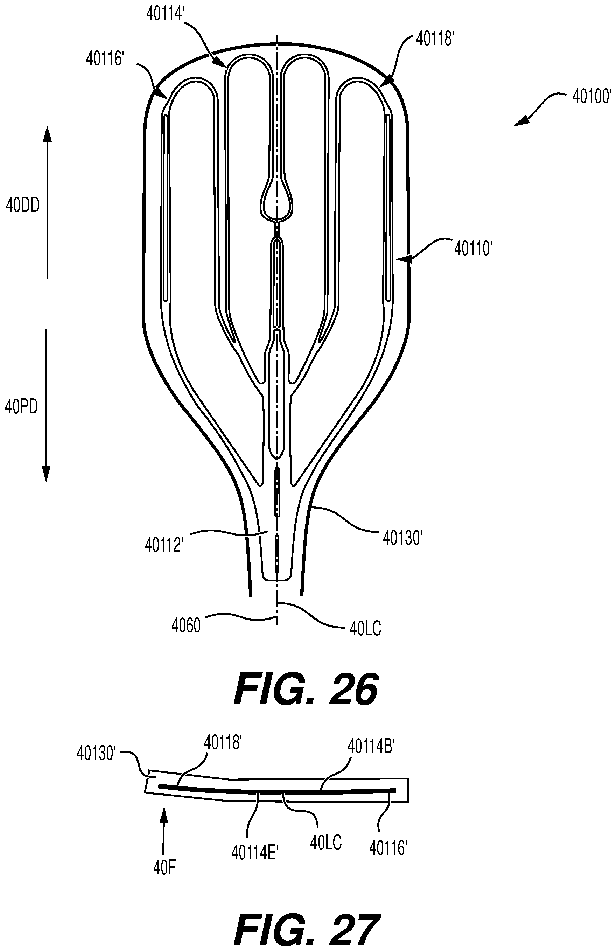

FIG. 26 is a schematic pictorial illustration showing a slightly modified framework 40110' (relative to the previously discussed framework 40110) of an end effector 40100' embedded in insulative material 40130. As shown, and like the previous example, the framework 40110' includes a base 40112', a central first spine loop 40114', and two side spines 40116', 40118' (i.e., a left spine 40116' and a right spine 40118') on opposing lateral sides of the first spine loop 40114'. The base 40112' connects with an elongated shaft 4014A (FIG. 21) of the medical device 4014 and extends along the longitudinal axis 4060. The spines 40114', 40116', 40118' extend from the base 40112' along the longitudinal axis 4060.

As seen in FIG. 27, like the previous example, the central first spine loop 40114' is configured to increase the stiffness along the center of the framework 40110' and to resist bending when a force 40F, e.g., a torsional force, is applied thereto. These forces 40F can occur at various times during use, such as when being inserted into an introducer. As discussed above, this design aids in preventing electrical interconnections on the flexible circuit 40120 that run along the longitudinal center 40LC from breaking.

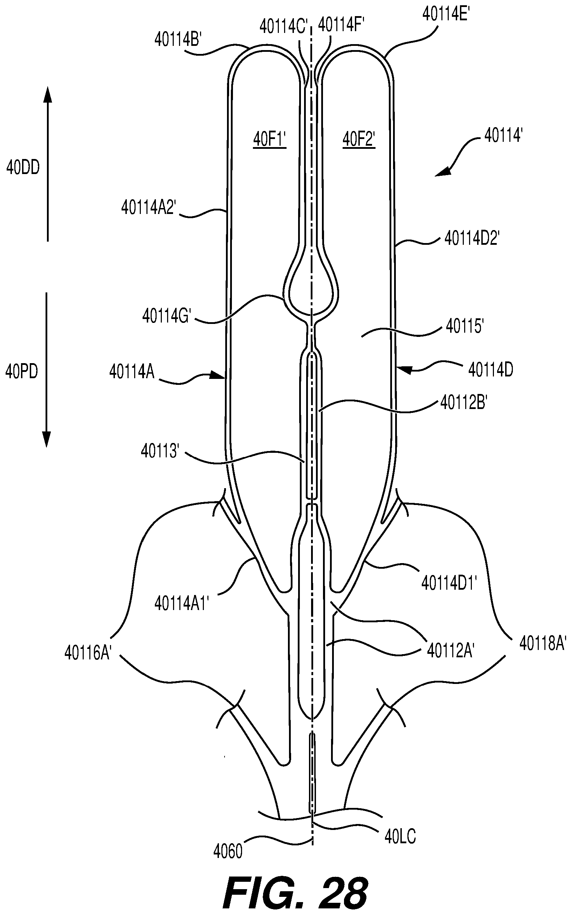

Further to the above, and with reference to FIG. 28, the first spine loop 40114', which includes features that resist bending, is shown in greater detail. The first spine loop 40114 includes first through seventh segments 114A'-114G' which are similarly configured as the example described in FIGs. 22-25. The curvilinear first segment 40114A' is connected to the base 40112 and extends from the base 40112 in a distal direction 40DD along the longitudinal axis 4060. The curvilinear first segment 40114A' includes (1) a first section 40114A1' directly connected to the base 40112' that is angled relative to the longitudinal axis 4060 and (2) a second section 40114A2' connected to the first section 40114A1' that is generally parallel to the longitudinal axis 4060. The second segment 40114B' is arcuate and is connected to and extends from the curvilinear first segment 40114A'. The third segment 40114C' is connected to and extends from the second segment 40114B' in a proximal direction 40PD along the longitudinal axis 4060. The fourth segment 40114D2 curvilinear fourth segment 40114D' is connected to and extends from the base 40112' in the distal direction 40DD along the longitudinal axis 4060. Like the curvilinear first segment 40114A', the fourth segment 40114D2 curvilinear fourth segment 40114D' includes (1) a first section 40114D1' directly connected to the base 40112' that is angled relative to the longitudinal axis 4060 and (2) a second section 40114D2' connected to the first section 40114D1' that is generally parallel to the longitudinal axis 4060. The fifth segment 40114E' is arcuate and is connected to and extends from the fourth segment 40114D2curvilinear fourth segment 40114D'. The sixth segment 40114F' is connected to and extends from the fifth segment 40114B' in the proximal direction 40PD along the longitudinal axis 4060. The seventh segment 40114G' is arcuate (resembling the form of a teardrop), is bisected by the longitudinal axis 4060, and connects the third segment 40114C' and the sixth segment 40114F' to aid in the aforementioned bend resistance.

Cumulatively, the segments 40114A'-40114G' of the first spine loop 40114' define a finger shaped opening 40115' that includes two finger shaped openings 40F1', 40F2'. Moreover, the curvilinear first segment 40114A', the second segment 40114B', and the third segment 40114C' are symmetrical with the fourth segment 40114D2 curvilinear fourth segment 40114D', the fifth segment 40114E', and the sixth segment 40114F' relative to the longitudinal axis 4060 and longitudinal center 40LC.

The base 40112' includes (1) a first section 40112A' that connects with the elongated shaft 4014A and (2) a second section 40112B' that extends within the finger shaped opening 40115 to connect with the seventh segment 40114G. Thus, the second section 40112B' effectively splits the opening 40115' into two halves (i.e., the two finger shaped openings 40F1', 40F2'). Moreover, the first section 40112A' and second section 40112B' each define one or more apertures 40113' therein.

As particularly seen in FIG. 28, the second spine loop 40116' has a pair of terminal sections 40116A'. One terminal section 40116A' is connected to the first section 40112A' of the base 40112', while the other terminal section 40116A' is connected to the curvilinear first segment 40114A' of the first spine loop 40114. Similarly, the third spine loop 40118 has a pair of terminal sections 40118A'. One terminal section 40118A' is connected to and opposite side of the first section 40112A' of the base 40112', while the other terminal section 40118A' is connected to the fourth segment 40114D2 curvilinear fourth segment 40114D' of the first spine loop 40114'.

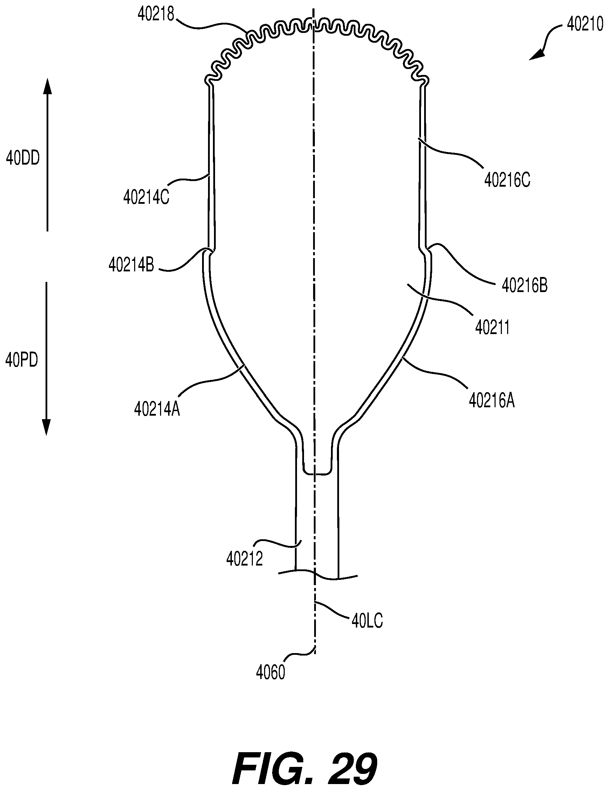

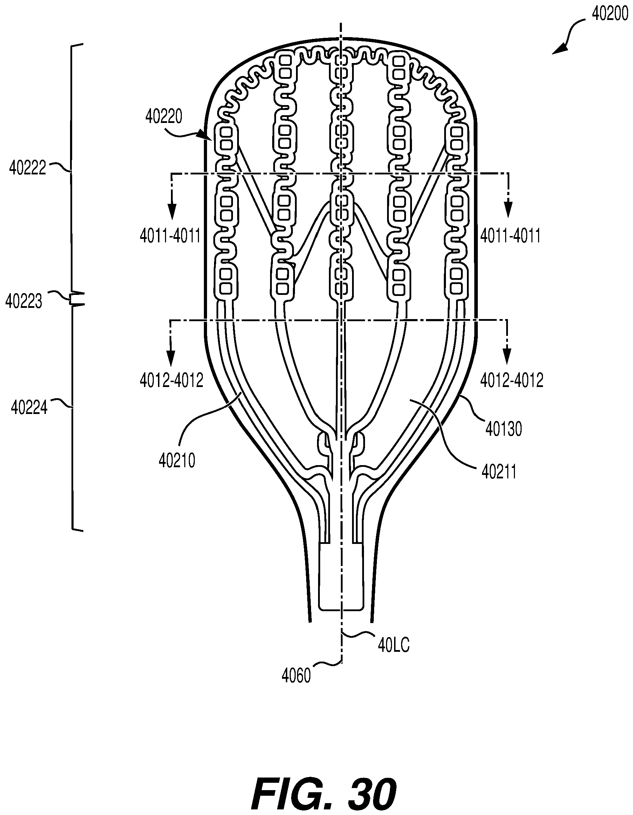

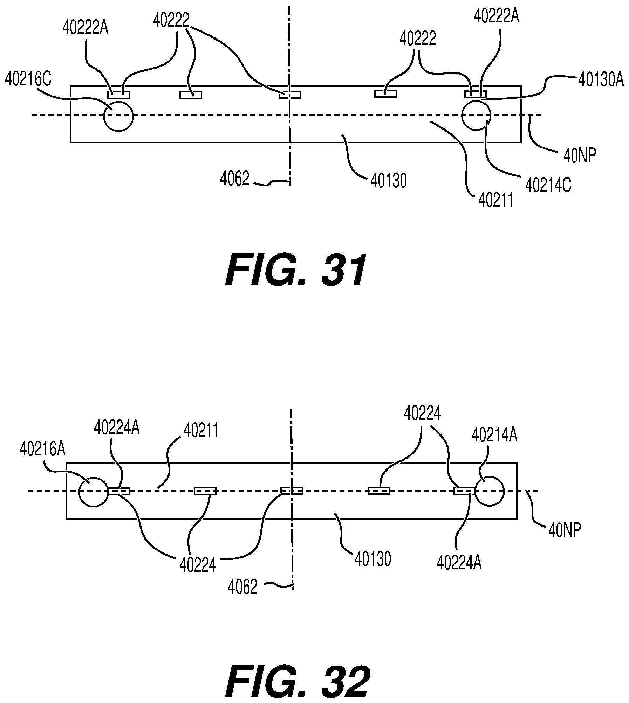

FIG. 29 is a schematic pictorial illustration showing another framework 40210 of another end effector 40200. FIG. 30 is a schematic pictorial illustration showing the framework 40210 embedded in insulative material 40130 and with a flexible circuit 40220 assembled therewith. FIGs. 31-32 are cross-sectional views taken from FIG. 30.

Turning now to FIGs. 29-32, as mentioned above, another example of a framework 40210 for another end effector 40200 is shown. As seen, in particular, in FIGs. 30-32, the end effector 40200 also includes an insulative material 40130 disposed on the framework 40210 and a flexible circuit 40220. During use, the flexible circuit 40220 incurs stresses/loads that can lead to its failure at one or more points. The example shown in these figures reduces stress applied thereto by routing a section of the flexible circuit to the neutral plane 40NP of the framework 40210, and is discussed in greater detail below.

Like in the previous example, the framework 40210 can be formed from a super elastic material. By way of example, the framework can be formed from a shape-memory alloy, such as Nitinol, cobalt chromium, stainless steel, and/or other alloys that exhibit pseudo-elastic and/or super-elastic properties.

The framework 40210 includes a base 40212, a first spine loop 40214, a second spine loop 40216, and a third spine loop 40218. The base 40212 connects with the elongated shaft 4014A of the medical device 4014 and extends along a longitudinal axis 4060. The framework 40210 also has a framework plane 40FP that extends along the neutral plane 40NP of the framework 40210.

The first spine loop 40214 extends from the base 40212 along the longitudinal axis 4060 and includes a first segment 40214A, a second segment 40214B, and a third segment 40214C. The first segment 40214A is connected to the base 40212 and extends therefrom in a distal direction 40DD along the longitudinal axis 4060. The second segment 40214B is connected to the first segment 40214A and extends therefrom inwardly towards the longitudinal axis 4060. The third segment 40214C is connected to the second segment 40214B and extends therefrom in the distal direction 40DD along the longitudinal axis 4060. In some examples, the third segment 40214C extends approximately parallel to the longitudinal axis 4060.

The second spine loop 40216 extends from an opposite side of the base 40212 (compared with the first spine loop 40214) along the longitudinal axis 4060 and includes a first segment 40216A, a second segment 40216B, and a third segment 40216C. The first segment 40216A is connected to the base 40212 and extends therefrom in the distal direction 40DD along the longitudinal axis 4060. The second segment 40216B is connected to the first segment 40216A and extends therefrom inwardly towards the longitudinal axis 4060. The third segment 40216C is connected to the second segment 40216B and extends therefrom in the distal direction 40DD along the longitudinal axis 4060. In some examples, the third segment 40216C extends approximately parallel to the longitudinal axis 4060.

The third spine loop 40218 connects the third segment 40214C of the first spine loop 40214 and the third segment 40216C of the second spine loop 40216 and is bisected by the longitudinal axis 4060 and a longitudinal center of the framework 40210 (with the longitudinal center 40LC being coaxial with the longitudinal axis 4060, like in the previous example). In some examples, the third spine loop 40218 has an undulating form, which assists in lateral flexibility and collapsibility of the framework 40210. In these examples, the undulations can be modified to tune the lateral resistance of the framework 40210. In other examples, the third spine loop 40218 can be omitted.

To readily permit the flexible circuit 40220 to have a section that runs along the neutral plane 40NP, the framework 40210 is provided with a hollow design. In other words, the framework 40210 has a finger shaped opening 40211 that is defined by its base 40212, first spine loop 40214, second spine loop 40216, and third spine loop 40218.

As seen best in and exemplified by FIG. 31, the flexible circuit 40220 has a first section 40222 vertically spaced from the framework 40210 along the vertical axis 4062 by the insulative material 40130 and disposed parallel to the framework plane FP. The first section 40222 also includes a peripheral section 40222A that is disposed directly above the first spine loop 40214, the second spine loop 40216, and the third spine loop 40218.

As seen best in and exemplified by FIG. 32, the flexible circuit 40220 further includes a second section 40224 that is offset from the first section 40222 along the vertical axis 4062 (see FIG. 32 in comparison with FIG. 31) and extends along the framework plane 40FP within the finger shaped opening 40211 defined by the framework 40210. The second section 40224 of the flexible circuit 40220 also includes a peripheral section 40224A that runs along an inner surface of the first spine loop 40214 and an inner surface of the second spine loop 40216 from a mid-section of the framework 40210 to the base 40212.

Further to the above, the flexible circuit 40220 includes a transition section 40223 that connects the first section 40222 and the second section 40224 of the flexible circuit. Referring to FIGs. 29 and 30, the transition section 40223 is disposed proximal the second segment 40214B of the first spine loop 40214 and the second segment 40216B of the second spine loop 40216 along the longitudinal axis 4060, with both second segments 40214B, 40216B functioning as connecting segments between the first and third segments of the first spine loop 40214 and second spine loop 40216, respectively.

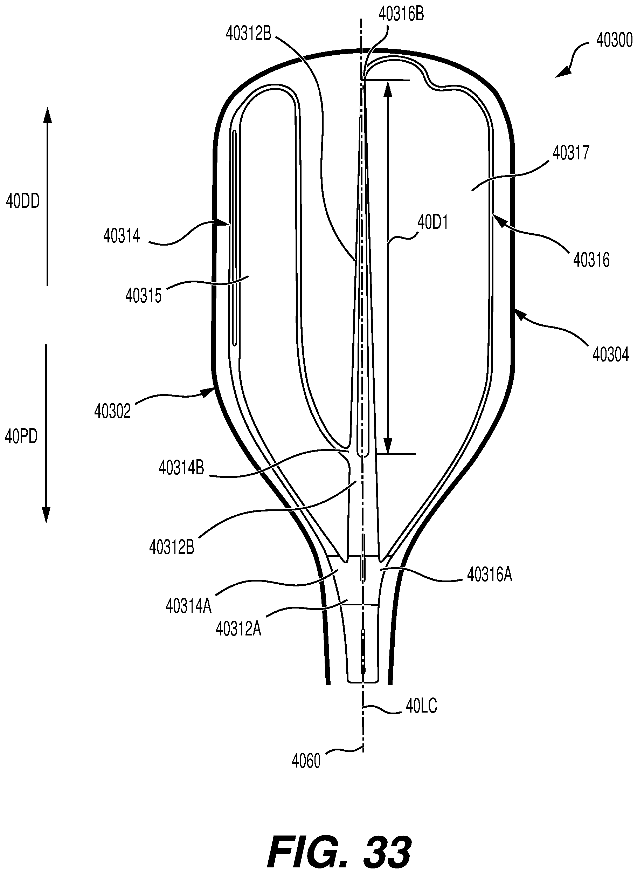

FIG. 33 is a schematic pictorial illustration showing yet another design of framework 40310 embedded in insulative material 40130 of an end effector 40300 for a medical device 4014. The framework 40310 extends along a longitudinal axis 4060 that is coaxial with a longitudinal center 40LC of the framework 40310. The framework 40310 includes a base 40312, a first spine loop 40314 extending from the base 40312 on one side of the longitudinal axis 4060, and a second spine loop 40316 extending from the base 40312 on an opposite side of the longitudinal axis 4060. In some examples, the base 40312 and spines 40314, 40316 are a monolithic member (i.e., single unitary piece).

The base 40312 includes a first section 40312A and a second section 40312B. The first section 40312A connects with the elongated shaft 4014A, and the second section 40312B extends away from the first section 40312A in a distal direction 40DD. In some examples, the base 40312 is bisected by and approximately symmetrical relative to the longitudinal axis 4060.

The first spine loop 40314 has a first distal end 40314A that is connected to the base 40312 at a first longitudinal location along the longitudinal axis 4060 and a second distal end 40314B connected to the base 40312 at a second longitudinal location along the longitudinal axis 4060 so as to define a first finger shaped opening 40315. It is noted that "longitudinal location" (in this and other examples) refers to the longitudinal component of the referred to element's location and excludes the lateral component of the location (i.e., the distance of the referred to element from the longitudinal axis). With respect to the orientation of the framework 40310 in FIG. 13, the longitudinal component is analogous with the y-coordinate in a Cartesian coordinate system.

Similarly, the second spine loop 40316 has a first distal end 40316A connected to the base 40312 at a third longitudinal location along the longitudinal axis 4060 a second distal end 40316B connected to the base 40312 at a fourth longitudinal location along the longitudinal axis 4060 so as to define a second finger shaped opening 40317.

The first longitudinal location, the second longitudinal location, the third longitudinal location, and the fourth longitudinal location of the respective distal ends 40314A, 40314B, 40316A, 40316B are disposed along the longitudinal axis 4060 such that the framework 40310 is asymmetric relative to the longitudinal axis 4060.

In particular, in the example shown in FIG. 33, the first distal end 40314A of the first spine loop 40314 and the first distal end 40316A of the second spine loop 40316 are connected to the first section 40312A of the base 40312 such that the first longitudinal location and the third longitudinal location are the same or approximately the same. Moreover, the second distal end 40314B of the first spine loop 40314 and the second distal end 40316B of the second spine loop 40316 are connected to the second section 40312B. More specifically, the second distal end 40314B of the first spine loop 40314 is connected to a proximal end of the second section 40312B, and the second distal end 40316B of the second spine loop 40316 is connected to a distal end of the second section 40312B such that the second longitudinal location and the fourth longitudinal location are spaced by a predetermined distance 40D1 along the longitudinal axis 4060. This configuration of the ends 40314A, 40314B, 40316A, 40316B, in part, results in the first finger shaped opening 40315 having a smaller area than that of the second finger shaped opening 4040317.

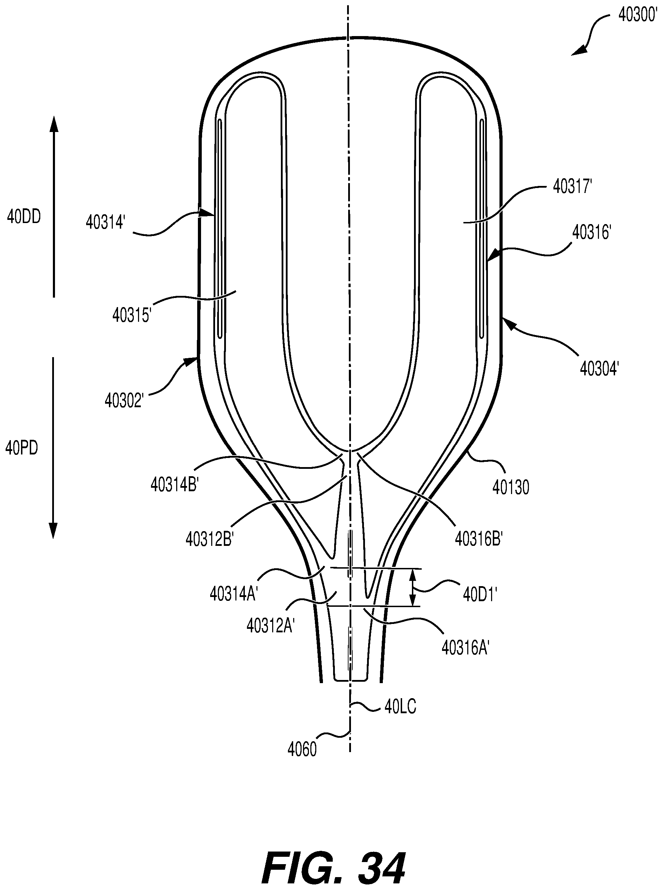

FIG. 34 is a schematic pictorial illustration showing another example of framework 40310', similar to the framework 40310 of FIG. 33, embedded in insulative material 40130, of an end effector 40300' for a medical device 4014. The framework 40310' extends a longitudinal axis 4060 that is coaxial with a longitudinal center LC of the framework 40310'. The framework 40310' includes a base 40312', a first spine loop 40314' extending from the base 40312' on one side of the longitudinal axis 4060, and a second spine loop 40316' extending from the base 40312' on an opposite side of the longitudinal axis 4060. In some examples, the base 40312' and spines 40314', 40316' are a monolithic member.

The base 40312' includes a first section 40312A' and a second section 40312B'. The first section 40312A' connects with the elongated shaft 4014A', and the second section 40312B' extends away from the first section 40312A' in a distal direction 40DD. In some examples, the base 40312' is bisected by and approximately symmetrical relative to the longitudinal axis 4060.

The first spine loop 40314' has a first distal end 40314A' that is connected to the base 40312' at a first longitudinal location along the longitudinal axis 4060 and a second distal end 40314B' connected to the base 40312' at a second longitudinal location along the longitudinal axis 4060 so as to define a first finger shaped opening 40315'.

Similarly, the second spine loop 40316' has a first distal end 40316A' connected to the base 40312' at a third longitudinal location along the longitudinal axis 4060' a second distal end 40316B' connected to the base 40312' at a fourth longitudinal location along the longitudinal axis 4060 so as to define a second finger shaped opening 4040317'.

Like the previous example, the first longitudinal location, the second longitudinal location, the third longitudinal location, and the fourth longitudinal location of the respective distal ends 40314A', 40314B', 40316A', 40316B' are disposed along the longitudinal axis 4060 such that the framework 40310' is asymmetric relative to the longitudinal axis 4060.

In particular, in the example shown in FIG. 34, the first distal end 40314A' of the first spine loop 40314' and the first distal end 40316A' of the second spine loop 40316' are connected to the first section 40312A' of the base 40312' such that the first longitudinal location and the third longitudinal location are spaced along the longitudinal axis 4060 by a predetermined distance 40D1' (with the third longitudinal location being more proximal a proximal end of the base 40312' than the first longitudinal location). Moreover, the second distal end 40314B' of the first spine loop 40314' and the second distal end 40316B' of the second spine loop 40316' are connected to the second section 40312B' such that the second longitudinal location and the fourth longitudinal location are the same or approximately the same along the longitudinal axis 4060. This configuration of the ends 40314A', 40314B', 40316A', 40316B', in part, results in the first finger shaped opening 40315' having a smaller area than that of the second finger shaped opening 40317'.

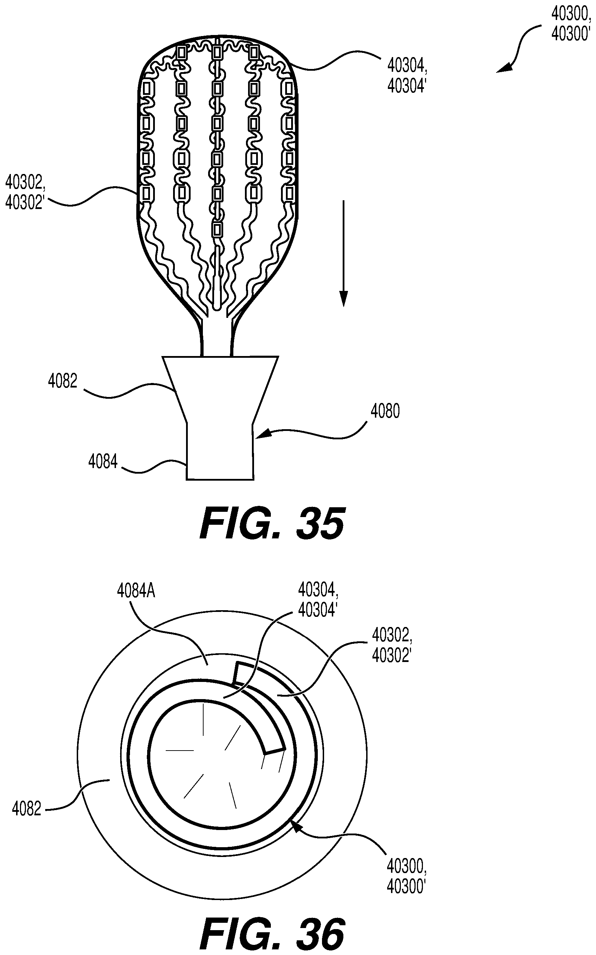

FIG. 35 is a schematic pictorial illustration showing the end effectors 40300, 40300' of FIGs. 33 and 34 being inserted into an introducer 4080 that has an entrance section 4082 and a sheath 4084. FIG. 36 is a schematic pictorial illustration showing the end effector 40300, 40300' inserted into a finger shaped opening 4084A of the introducer 4080. In order to collapse either end effector 40300, 40300' previously shown, an introducer 4080 is used. Due to the asymmetric design of the respective sides 40302, 40302' and 40304, 40304' of the end effectors 40300, 40300', one side of the end effector 40300, 40300' can be made (1) to begin insertion into the introducer 4080 first and/or (2) to require less force from the sidewalls of the introducer 4080 to collapse within the finger shaped opening 4084A. In this way, the collapse pattern of the end effector 40300, 40300' can be made more predictable.

It should be noted that while the framework 40110 is shown as being on a common plane, it is well within the scope of the present disclosure that the spine loops can be offset on different planes or the framework 40110 itself being curved about a central axis to define a cylindric framework.

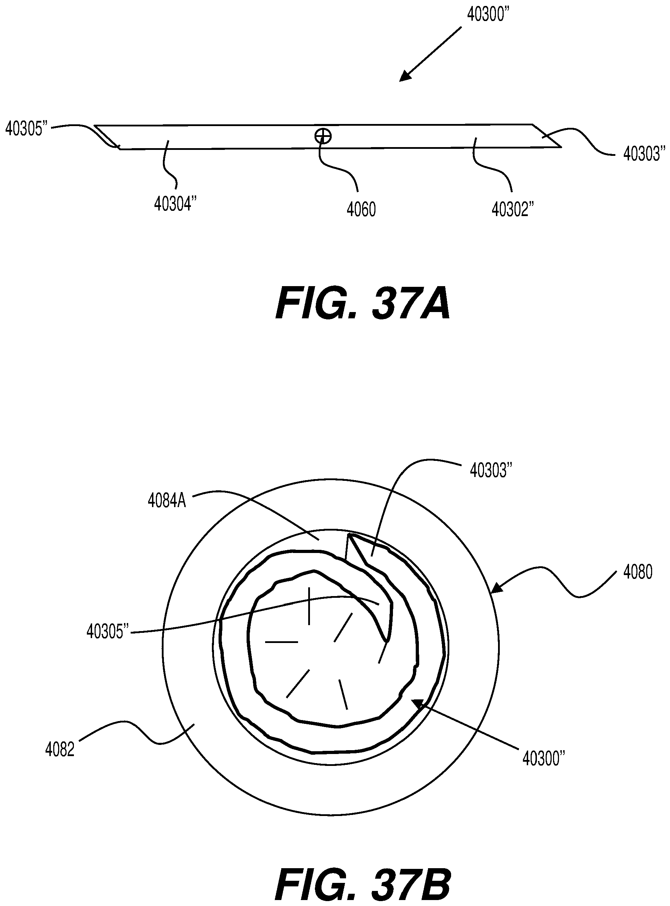

Turning now to FIGs. 37A-40B, these figures depict schematic pictorial illustrations of exemplary end effectors 40300", 40300A", 40300B", 40300C" that include treated edges for optimized retraction and/or folding thereof. Similarly to the previously described examples of, e.g., FIGs. 33-36, these examples of end effectors 40300", 40300A", 40300B", 40300C" enable a consistent collapse shape and reduce retraction force of the end effector. It is noted that the treated edges described herein can be applied to any of the end effectors described in this application without departing from the spirit and scope of the present disclosure.

Making reference to FIG. 37A, a top view of an end effector 40300" is depicted. Similar to the example of FIG. 33, the end effector 40300" includes a framework (not explicitly shown in these figures; see, e.g., FIG. 22 that depicts a framework 40110) embedded in insulative material 40302", 40304". The end effector 40300" extends along a longitudinal axis 4060 that is coaxial with a longitudinal center of the end effector 40300". The framework can be symmetrical or asymmetrical and is substantially planar along its longitudinal axis 4060. It is noted that, as used herein, and similar to the terms "about" and "approximately" discussed above, the term "substantially planar" includes frameworks that have a slight curvature to their form along the longitudinal axis 4060. In other words, frameworks that are not completely flat are fully within the spirit and scope of the present disclosure. Moreover, those skilled in the art will appreciate that the presently described end effector 40300" can include other features of previously described examples. For example, the end effector 40300" can include one or more flexible circuit layers 40120A, 40120B including substantially planar electrodes and/or sensing loop layers, with the flexible circuit being disposed in the insulative material 40302", 40304" and vertically spaced from the framework 40110 along the vertical axis.

As seen in FIG. 37A, the insulative material 40302", 40304" of the end effector 40300" is divided, relative to the longitudinal axis 4060, into a first side 40302" and a second side 40304", each side with a respective outer edge (e.g., first outer edge 40303" and second outer edge 40305") at its distalmost extent from the longitudinal axis 4060. At least one of the edges 40303", 40305" includes a treatment to aid in the collapsing/folding of the end effector 40300".

In the present example of FIGs. 37A-37B, both outer edges 40303", 40305" include a tapered cut such that the outer edges 40303", 40305" form a non-perpendicular angle relative to the substantially planar front and rear surface of the insulative material (see the upper and lower portions of end effector 40300" in FIG. 37A). In some examples, the tapered cuts are approximately parallel to one another.

FIG. 37B is a schematic pictorial illustration showing the end effector 40300" of FIG. 37A inserted into an opening 4084A of an introducer 4080 that has an entrance section 4082 and a sheath 4084 (see FIG. 35). In order to collapse the end effector 40300", an introducer 4080 is used. Due to the tapered cut design of one or more of the respective outer edges 40303", 40305" of the end effector 40300", the outer edges 40303", 40305" of the end effector 40300" can be made to more easily slide on and past one another (rather than abutting and causing the end effector 40300" to buckle irregularly). In this way, the collapse pattern of the end effector 40300" can be made more predictable.

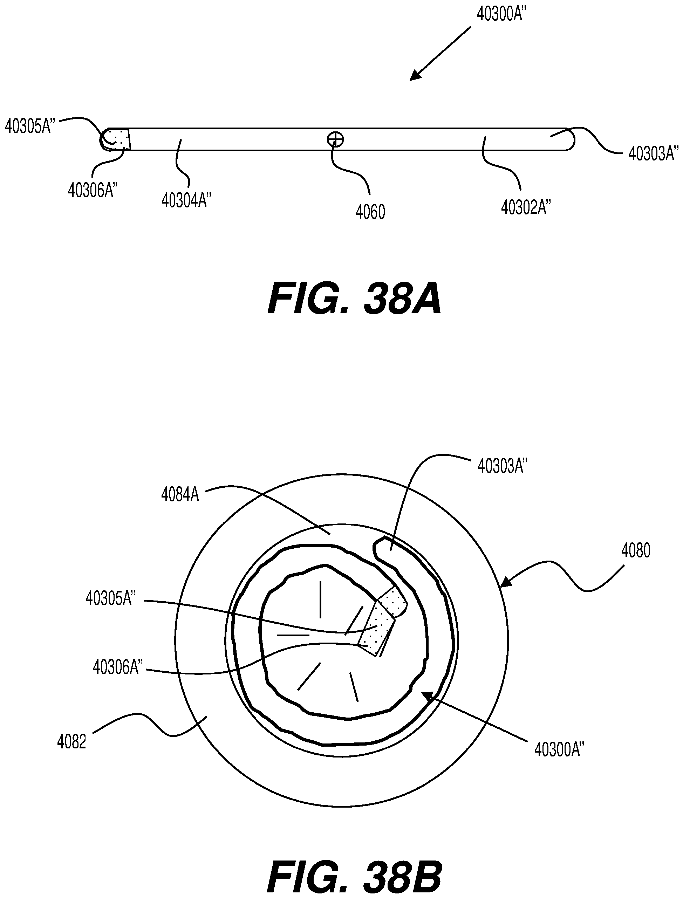

FIG. 38A is a top view of another end effector 40300A" that is identical to the end effector 40300" of FIG. 37A, but with a different and/or additional end treatment. Specifically, the insulative material 40302A", 40304A" of the end effector 40300A" is divided, relative to the longitudinal axis 4060, into a first side 40302A" and a second side 40304A", each side with a respective outer edge (e.g., first outer edge 40303A" and second outer edge 40305A") at its distalmost extent from the longitudinal axis 4060. At least one of the edges (e.g., second edge 40305A") includes a treatment 40306A" to aid in the collapsing/folding of the end effector 40300A".

In the present example of FIGs. 38A-38B, at least a portion of the second outer edge 40305A" include a lubricious coating 40306A", such as, but not limited to, polytetrafluorethylene (PTFE). In some examples, both outer edges 40303A", 40305A" can have portions (or its entirety) coated with the lubricious coating 40306A". It is noted that this exemplary end effector 40300A" also includes a rounded cut at both outer edges 40302A", 40304A"

FIG. 38B is a schematic pictorial illustration showing the end effector 40300A" of FIG. 38A inserted into an opening 4084A of an introducer 4080 that has an entrance section 4082 and a sheath 4084 (see FIG. 35). In order to collapse the end effector 40300A", an introducer 4080 is used. Due to the lubricious coating 40306A" and/or rounding of one or more of the respective outer edges 40303A", 40305A" of the end effector 40300A", the reduced friction permits the outer edges 40303A", 40305A" of the end effector 40300A" to more easily slide on and past one another. In this additional or alternative way, the collapse pattern of the end effector 40300A" can be made more predictable.

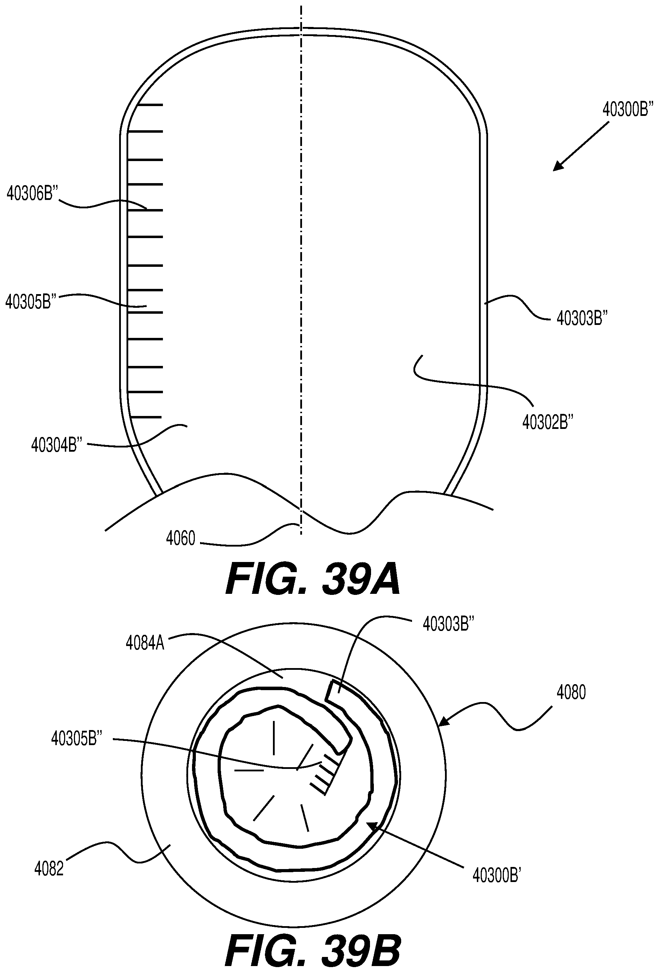

FIG. 39A is a top view of yet another end effector 40300B" that is identical to the end effectors 40300", 40300A" of FIGs. 37A-38B, but with a different and/or additional end treatment. Specifically, the insulative material 40302B", 40304B" of the end effector 40300B" is divided, relative to the longitudinal axis 4060, into a first side 40302B" and a second side 40304CB", each side with a respective outer edge (e.g., first outer edge 40303B" and second outer edge 40305B") at its distalmost extent from the longitudinal axis 4060. At least one of the edges (e.g., second edge 40305B") includes a treatment 40306B" to aid in the collapsing/folding of the end effector 40300B".

In the present example of FIGs. 39A-39B, at least a portion of the second outer edge 40305B" includes multiple incised cuts 40306B" formed into the insulative material, in a direction towards the longitudinal axis 4060 and substantially perpendicular thereto. In some examples, both outer edges 40303B", 40305B" can have portions (or their entirety) with the incised cuts 40306B". The incised cuts 40306B" function to soften at least one side of the insulative material to make it more capable of deformation.

FIG. 39B is a schematic pictorial illustration showing the end effector 40300B" of FIG. 39A inserted into an opening 4084A of an introducer 4080 that has an entrance section 4082 and a sheath 4084 (see FIG. 35). In order to collapse the end effector 40300B", an introducer 4080 is used. Due to the incised cuts 40306B" of at least a portion of the second outer edge 40305B" of the end effector 40300B", the increased pliability of the second outer edge 40305B" enables it to collapse inwardly relative to the first outer edge 40303B" when the two edges 40303B", 40305B" engage one another. In this additional or alternative way, the collapse pattern of the end effector 40300B" can be made more predictable.

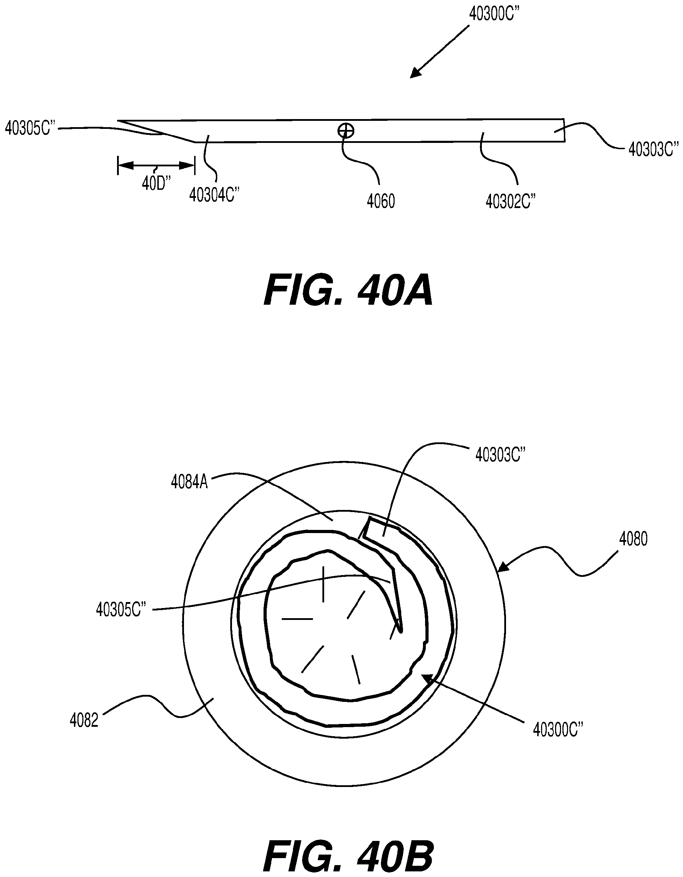

FIG. 40A is a top view of yet another end effector 40300C" that is identical to the end effectors 40300", 40300A", 40300C" of FIGs. 37A-39B, but with a different and/or additional end treatment. Specifically, the insulative material 40302C", 40304C" of the end effector 40300C" is divided, relative to the longitudinal axis 4060, into a first side 40302C" and a second side 40304C", each side with a respective outer edge (e.g., first outer edge 40303C" and second outer edge 40305C") at its distalmost extent from the longitudinal axis 4060. At least one of the edges (e.g., second edge 40305C") includes a treatment, similar to that of end effector 40300", to aid in the collapsing/folding of the end effector 40300C".

In the present example of FIGs. 40A-40B, a single outer edge 40305C" includes a tapered cut such that the second outer edge 40305C" forms a non-perpendicular angle relative to the substantially planar front and rear surface of the insulative material (see the upper and lower portions of end effector 40300C" in FIG. 40A). In versions where a single tapered cut is employed, the cut can extend a distance 40D" in a direction towards the longitudinal axis 4060. The distance 40D" is optimized to increase the pliability of the second outer edge 40305C", enabling it to collapse inwardly relative to the first outer edge 40303C" when the two edges 40303C", 40305C" engage one another.

FIG. 40B is a schematic pictorial illustration showing the end effector 40300C" of FIG. 40A inserted into an opening 4084A of an introducer 4080 that has an entrance section 4082 and a sheath 4084 (see FIG. 35). In order to collapse the end effector 40300", an introducer 4080 is used. Due to the tapered cut design of the second outer edge 40305C" of the end effector 40300C", the second outer edge 40305C" is able to collapse inwardly relative the first outer edge 40303C" when the two edges 40303C", 40305C" engage one another. In this way, the collapse pattern of the end effector 40300C" can be made more predictable.

As those skill in the art will appreciate, the examples described with respect to FIGs. 37A-40B can be combined with one another without departing from the spirit and scope of the present disclosure. By way of non-limiting example, a lubricious coating 40306A" can be employed over the tapered cut(s) of the end effector 40300" described in FIGs. 37A-37B.

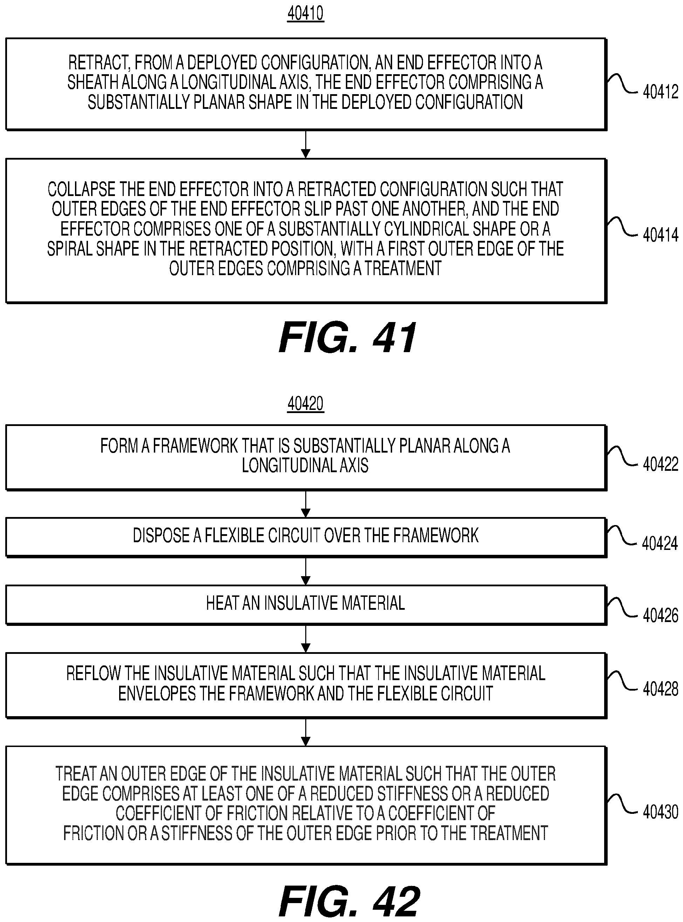

Further to the above-described examples of FIGs. 37A-40B, and with reference to FIG. 41, a method 40410 of using a medical can include the following. An end effector is retracted 40412, from a deployed configuration, into a sheath along a longitudinal axis. As discussed above, the end effector comprising a substantially planar shape in the deployed configuration. The end effector is collapsed 40414 into a retracted configuration such that outer edges of the end effector slip past one another. In the retracted configuration, end effector has one of a substantially cylindrical shape or a spiral shape in the retracted position, Moreover, a first outer edge of the outer edges has one or more of the aforementioned treatments, which aids in the collapse of the end effector.

Further to the above-described examples of FIGs. 37A-40B, and with reference to FIG. 42, a method 40420 of manufacturing an end effector for a medical device can include the following. A framework is formed 40422 that is substantially planar along a longitudinal axis. A flexible circuit is disposed 40424 over the framework and vertically spaced relative thereto. An insulative material is heated 40426 and reflowed 40428 such that the insulative material at least partially envelopes the framework and the flexible circuit. An outer edge of the insulative material is treated 40430 such that the outer edge has a reduced stiffness and/or a reduced coefficient of friction relative to an original coefficient of friction and/or an original stiffness of the outer edge (i.e., prior to the treatment).

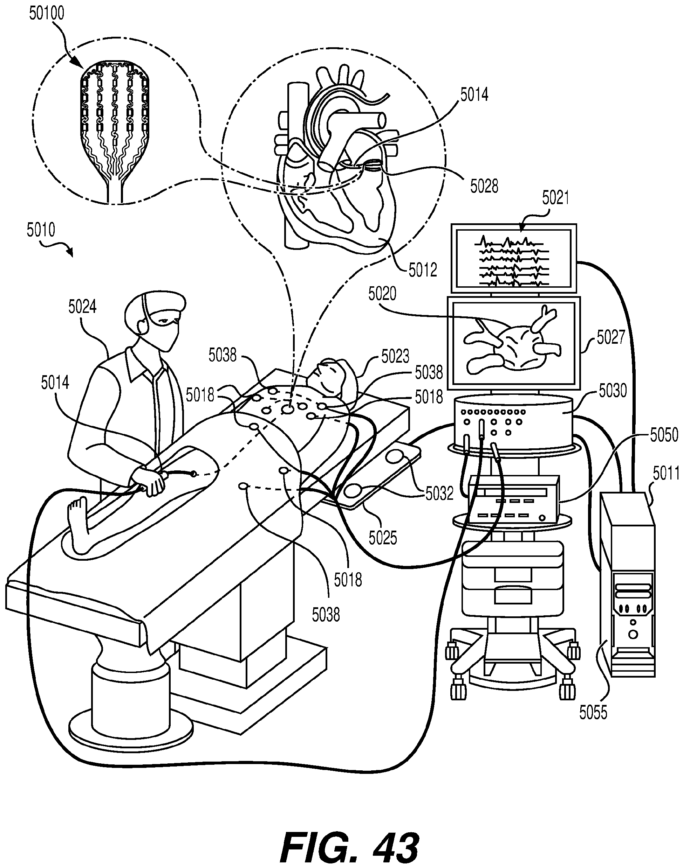

### Encapsulated Planar Catheters with Flexible Circuits Extending to a Neutral Axis (FIGs. 43-51)

Reference is made to FIG. 43 showing an example catheter-based electrophysiology mapping and ablation system 5010. System 5010 includes multiple catheters, which are percutaneously inserted by physician 5024 through the patient's 5023 vascular system into a chamber or vascular structure of a heart 5012. Typically, a delivery sheath catheter is inserted into the left or right atrium near a desired location in heart 5012. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 5014 that is configured for sensing IEGM is illustrated herein. Physician 5024 brings a catheter shaft with distal tip of catheter 5014 (i.e., multilayered end effector 50100) into contact with the heart wall for sensing a target site in heart 5012. For ablation, physician 5024 would similarly bring a distal end of an ablation catheter to a target site for ablating.

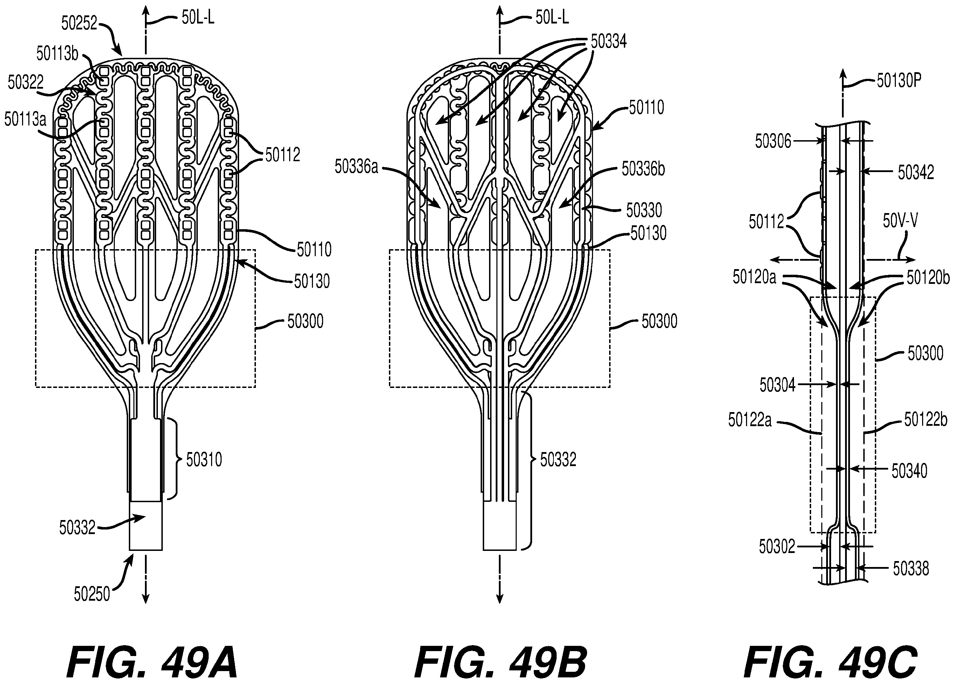

Catheter 5014 is an exemplary catheter that includes one and preferably multiple electrodes 50112 optionally distributed over end effector 50100 coupled to a catheter shaft and configured to sense the IEGM signals as described in more detail below. Catheter 5014 may additionally include a position sensor (see for example the electromagnetic coil 50330 in FIGs. 49A-49C) embedded in or near end effector 50100 for tracking position and orientation of end effector 50100. Optionally and preferably, position sensor is a magnetic based position sensor including multiple magnetic coils for sensing three-dimensional (3D) position and orientation (see distal loop 50334, first side loop 50336a, and second side loop 50336b in FIG. 49B).

Magnetic based position sensor may be operated together with a location pad 5025 including a plurality of magnetic coils 5032 configured to generate magnetic fields in a predefined working volume. Real time position of end effector 50100 of catheter 5014 may be tracked based on magnetic fields generated with location pad 5025 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 5010 includes one or more electrode patches 5038 positioned for skin contact on patient 5023 to establish location reference for location pad 5025 as well as impedance-based tracking of electrodes 50112. For impedance-based tracking, electrical current is directed toward electrodes 50112 and sensed at electrode skin patches 5038 so that the location of each electrode can be triangulated via the electrode patches 5038. Details of the impedance-based location tracking technology are described in US Patent Nos. 7,536,218; 7,756,576; 7,848,787; 7,869,865; and 8,456,182, each of which are incorporated herein by reference.

A recorder 5011 displays electrograms 5021 captured with body surface ECG electrodes 5018 and intracardiac electrograms (IEGM) captured with electrodes 50112 of catheter 5014. Recorder 5011 may include pacing capability for pacing the heart rhythm and/or may be electrically connected to a standalone pacer.

System 5010 may include an ablation energy generator 5050 that is adapted to conduct ablative energy to one or more of electrodes 50112 at a distal tip of a catheter configured for ablating. Energy produced by ablation energy generator 5050 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 5030 is an interface configured to establish electrical communication between catheters, electrophysiological equipment, power supply and a workstation 5055 for controlling operation of system 5010. Electrophysiological equipment of system 5010 may include for example, multiple catheters, location pad 5025, body surface ECG electrodes 5018, electrode patches 5038, ablation energy generator 5050, and recorder 5011. Optionally and preferably, PIU 5030 additionally includes processing capability for implementing real-time computations of location of the catheters and for performing ECG calculations.

Workstation 5055 includes memory, processor unit with memory or storage with appropriate operating software loaded therein, and user interface capability. Workstation 5055 may provide multiple functions, optionally including (1) modeling the endocardial anatomy in three-dimensions (3D) and rendering the model or anatomical map 5020 for display on a display device 5027, (2) displaying on display device 5027 activation sequences (or other data) compiled from recorded electrograms 5021 in representative visual indicia or imagery superimposed on the rendered anatomical map 5020, (3) displaying real-time location and orientation of multiple catheters within the heart chamber, and (4) displaying on display device 5027 sites of interest such as places where ablation energy has been applied. One commercial product embodying elements of the system 5010 is available as the CARTOTM 3 System, available from Biosense Webster, Inc., 31 Technology Drive, Suite 200, Irvine, CA 92618.

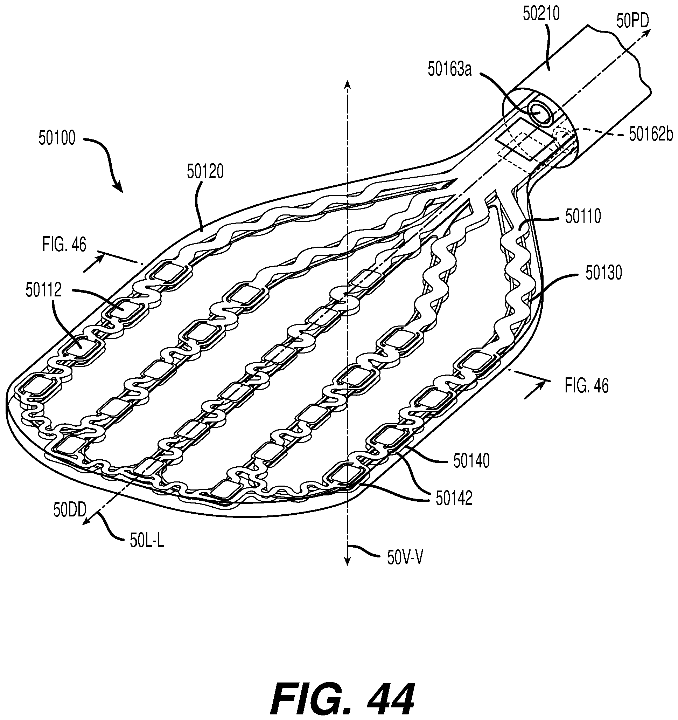

FIG. 44 provides an end effector 50100 in accordance with an embodiment of the present disclosure in order to achieve the ease of manufacturing, reduced cost, and enhanced end effector properties, such as desired stiffness, mapping resolution, electrode contact with target anatomy, and conformity of the end effector disclosed herein to flat, curved, irregular and/or nonplanar tissue surfaces found in the target anatomy. The end effector 50100 can include a flexible circuit 50110 including a plurality of electrodes 50112, each electrode of the plurality of electrodes 50112 including a contact surface 50112c. As used herein, the term "flexible circuit" includes thin-film circuit, flexible printed circuit board, thin film deposition via lithography and etching processes on substrates such as polyimide, copper, LCP, nitinol substrate, TPU, silicone, thermoset resin, or other polymeric substrate as shown and described in the attached technical references incorporated herein by the Appendix of Priority Application No. 63/615,574. In some examples, the flexible circuits described herein can be made primarily of polyimide. In other examples, it can be made of any of biocompatible polyimides, glass-reinforced epoxy laminate materials, copper, or graphene, alone or in combination. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuit 50110 can be disposed on an insulative material 50120. The insulative material 50120 can be contiguous to the contact surfaces 50112c so that only the contact surfaces 50112c of at least a portion of the plurality of electrodes 50112 are exposed to the ambient environment. As used herein, the term "contact surface" includes the portion of an electrode having a generally flat surface and the edge or edges which immediately surround said flat surface. Electrodes 50112 may have a slightly rounded, radiused, or chamfered edge that comes into contact with tissue, along with the generally flat surface, when the end effector 50100 is placed against tissue. As used herein, "ambient environment" refers to the external environment such as the organ in which the end effector 50100 is deployed or in the operating theater prior to being deployed in the biological organ.

It is noted that not all of the electrodes on the end effector 50100 described herein need be exposed through the insulative material 50120 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 50163a on one side and 50162b on the other side in fluid communication with an irrigation line not shown disposed in a catheter shaft. Instead of an irrigation line separate from the catheter shaft, a lumen can be formed via extrusion of the catheter shaft to provide for a lumen channel. It is noted that port 50163a or 50162b can be configured to have a sufficient flow diverter characteristic for irrigation fluid to cover the mapping electrodes during irrigation flow so as to prevent or reduce thrombus formations.

The flexible circuit 50110 can further include a framework 50130 contiguous to the insulative material or in the insulative material. In examples in which the flexible circuit 50110 includes framework 50130, the framework 50130 can be disposed directly on the flexible circuit 50110 with none, or very little, of the insulative material 50120 coming between the two.

Stated otherwise, an aspect of the present disclosure provides an end effector 50100 having a planar framework 50130 bisecting two flat, heat formed portions 50120a, 50120b of a flexible insulating mass 50120, with at least one flexible circuit 50110 disposed on one side of the framework 50130 and with contact surfaces 50112c of electrodes 50112 extending up to or slightly past the outer face of the flexible insulating mass 50120. In additional or alternative examples, the contact surfaces 50112c of electrodes 50112 can be slightly recessed, where there could be an opening through the insulative material 50120 to ensure that the electrode 50112 is exposed.

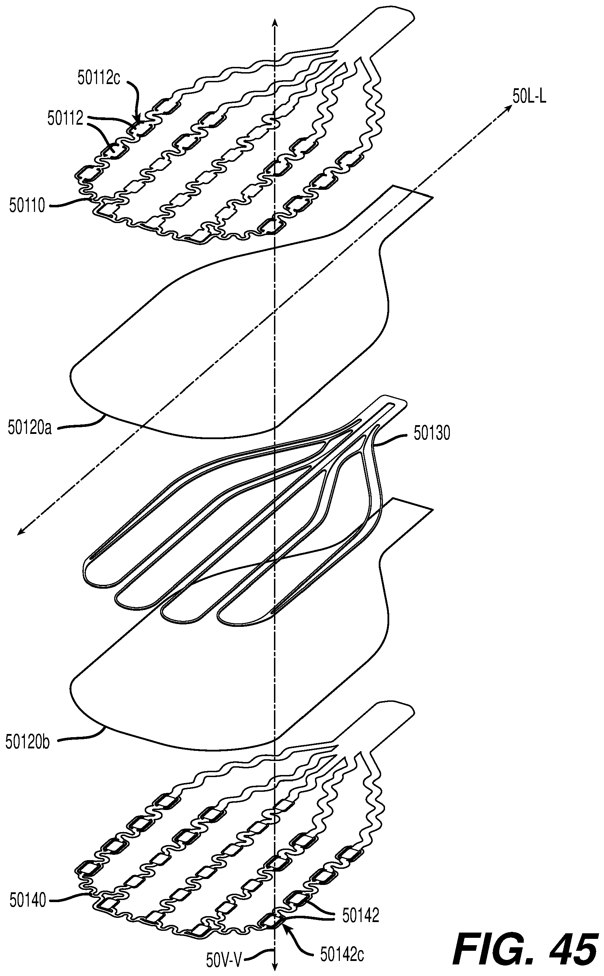

FIG. 45 shows an exploded view of the end effector 50100, with the components thereof exploded vertically along vertical axis 50V-V. The flexible circuit 50110 can be a first flexible circuit 50110 and the plurality of electrodes 50112 can be a first plurality of electrodes 50112, with each first electrode 50112 including a first contact surface 50112c. The end effector 50100 can further include a second flexible circuit 50140 having a second plurality of electrodes 50142. The second flexible circuit 50140 can be spaced apart from the first flexible circuit 50110 and each electrode of the second plurality of electrodes 50142 can have a second contact surface.

In examples having a first 50110 and second 50140 flexible circuits, the insulative material 50120 can be disposed between the first flexible circuit 50110 and the second flexible circuit 50140, and the insulative material 50120 can be contiguous to the second contact surfaces 50142c so that only the contact surface 50142c of each second electrode 50142 is exposed to the ambient environment, similar to how first electrodes 50112 are disposed in and exposed through the insulative material.

Electrodes 50112, 50142 can sense or receive signals generated by the tissues or transmit energy AC or DC from an energy generator to the tissues. In some examples, there are about 92 electrodes. In some examples, there are about 48 electrodes. In some examples, there are about 64 electrodes. In some examples, there are about 72 electrodes. In some examples, there are about 98 electrodes. Details of the spacing of the electrodes for each pair vs spacing between discrete sets of pairs of electrodes can be found in US Provisional Patent Application S.N. 63/406,673 filed on September 14, 2022, and included in the Appendix of Priority Application No. 63/615,574 for incorporation by reference.

In examples wherein the end effector 50100 includes a framework 50130 disposed between the first flexible circuit 50110 and the second flexible circuit 50140.

Framework 50130 can be a component of the end effector 50100 that is separate and distinct from the first flexible circuit 50110 and disposed proximate the first flexible circuit 50110. In this case, the insulative material 50120 can be further disposed between the framework 50130 and the second flexible circuit 50140. The framework 50130 can be formed from a planar or cylindrical stock of material using any suitable method. For example, the framework 50130 can be formed by cutting, laser cutting, stamping, etc.

Insulative material 50120 can include a first sheet of insulative material 50120a and a second sheet of insulative material 50120b fused together proximate the framework 50130 into a single, contiguous, generally planar insulative mass 50120. This insulative material 50120 also serves to enhance the atraumaticity of the end effector 50100 and to protect the subject from sharp edges. The insulative material 50120 can include polymer. The insulative material 50120 can be heat formed around at least a portion of the first flexible circuit 50110, the second flexible circuit 50140, and the framework 50130. The polymer can include thermoplastic polyurethane (TPU) or other heat formed or shaped material which lends itself to said heat forming.

Furthermore, while the insulative material 50120 is shown to be flat in these figures, insulative material 50120 can be shaped, scalloped, ribbed, ridged, concaved, convexed, or otherwise configured such that the overall profile of insulative material 50120 yields physical and/or mechanical properties, such as rigidity and flexion along multiple axes, required by the end effector 50100, mentioned above.

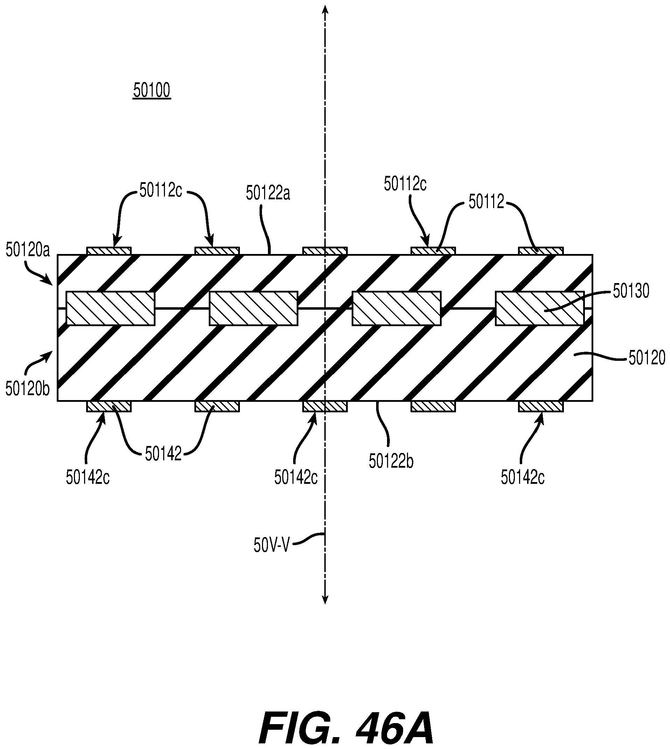

FIG. 46A shows a cross-section taken as indicated in FIG. 45. The first contact surfaces 50112c lie substantially parallel to a first outer surface 50122a of the insulative mass 50120. The first plurality of electrodes 50112 extending vertically and outwardly a first distance therefrom. Similarly, the second contact surfaces 50142c can lie substantially parallel to a second outer surface 50122b of the insulative mass 50120 and extend substantially vertically and outwardly a second distance therefrom.

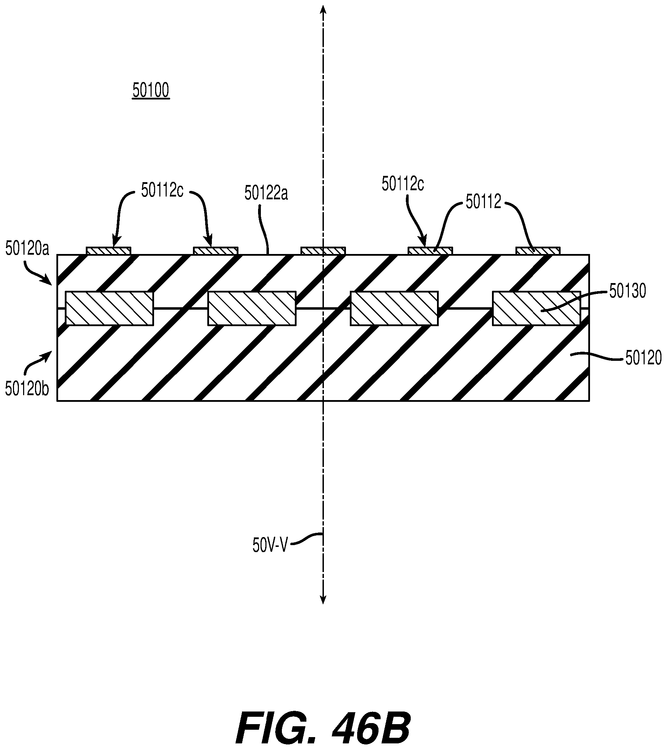

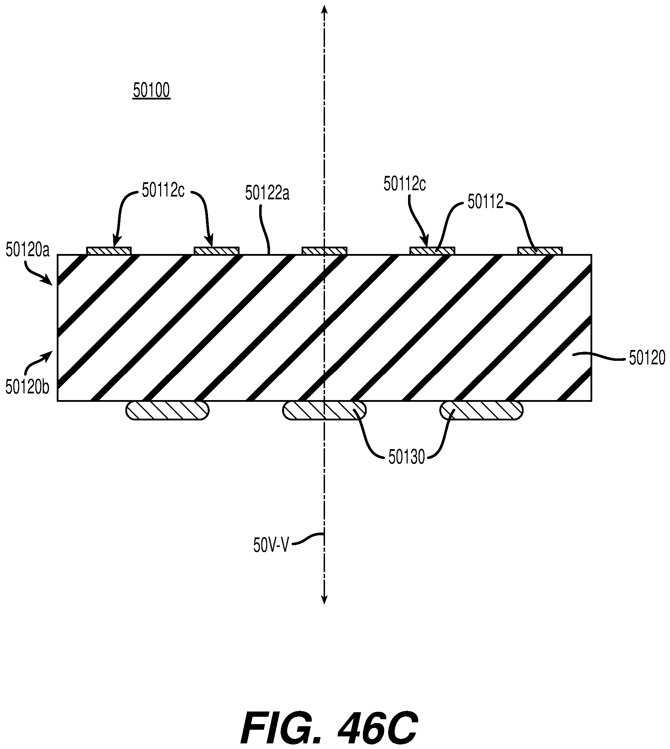

FIG. 46B shows a similar cross-section, but from an example end effector 50100 which does not have the second flexible circuit 50140. That is, the end effector in FIG. 46B has electrodes on only one side (single-sided end effector) as compared to an end effector of FIG. 46A with electrodes on opposite sides (or double-sided end effector). FIG. 46C shows a variation of FIG. 46B in which the framework 50130 is not encapsulated in the insulative material but disposed outside the insulative material 50120. It should be noted that while framework 50130 is shown in cross-section as rectangular, the framework 50130 is not limited such cross-section and any suitable cross-sections can be utilized.

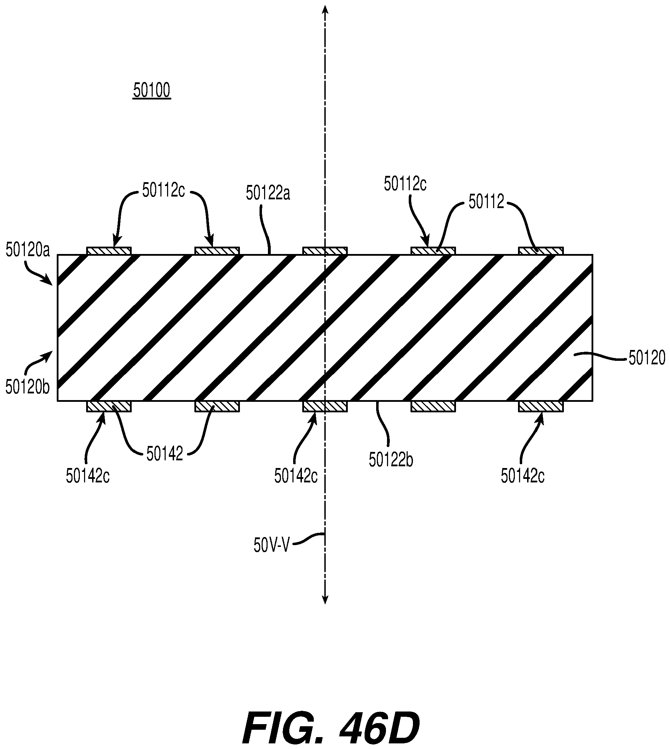

FIG. 46D shows a cross-section similar to that of FIG. 46A, but from an example end effector 50100 which does not have the framework 50130. It should be noted that in examples of FIG. 46D and the like, the insulative material 50120 may or may not have additional strength or formed thicker, as compared to the insulative material 50120 of the examples with framework 50130. The additional strength of the insulative material 50120 can be to compensate for the lack of framework 50130.

Importantly, the end effector 50100 having flush or outwardly protruding electrode contact surfaces 50112c, 50142c can be manufactured without the need to remove material in order expose electrode contact surfaces 50112c, 50142c via the method and/or fixture described in more detail below.

In other examples, the first contact surfaces 50112c can lie substantially coplanar to a first outer surface 50122a of the insulative mass 50120, and the second contact surfaces 50142c lying coplanar to a second outer surface 50122b of the insulative mass 50120.

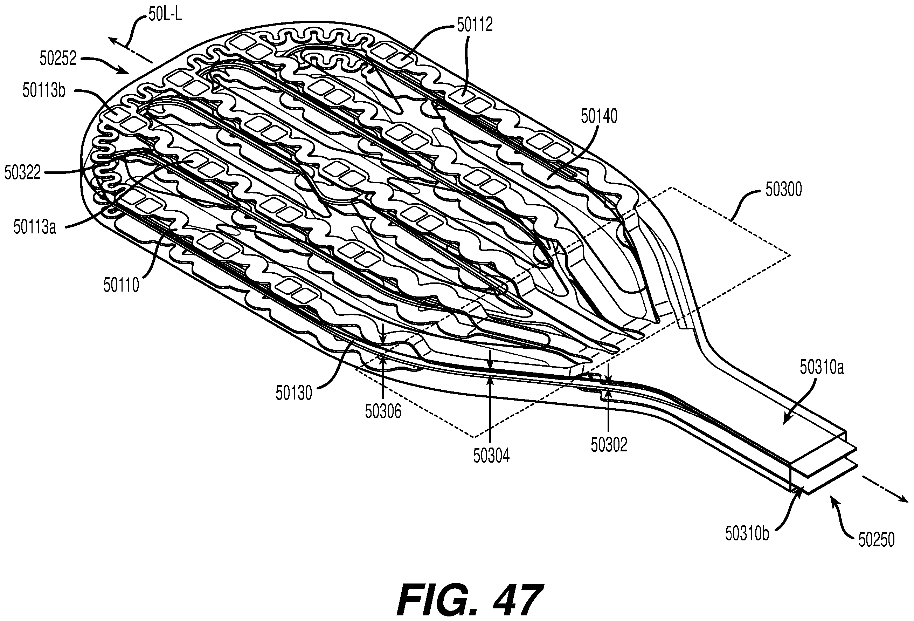

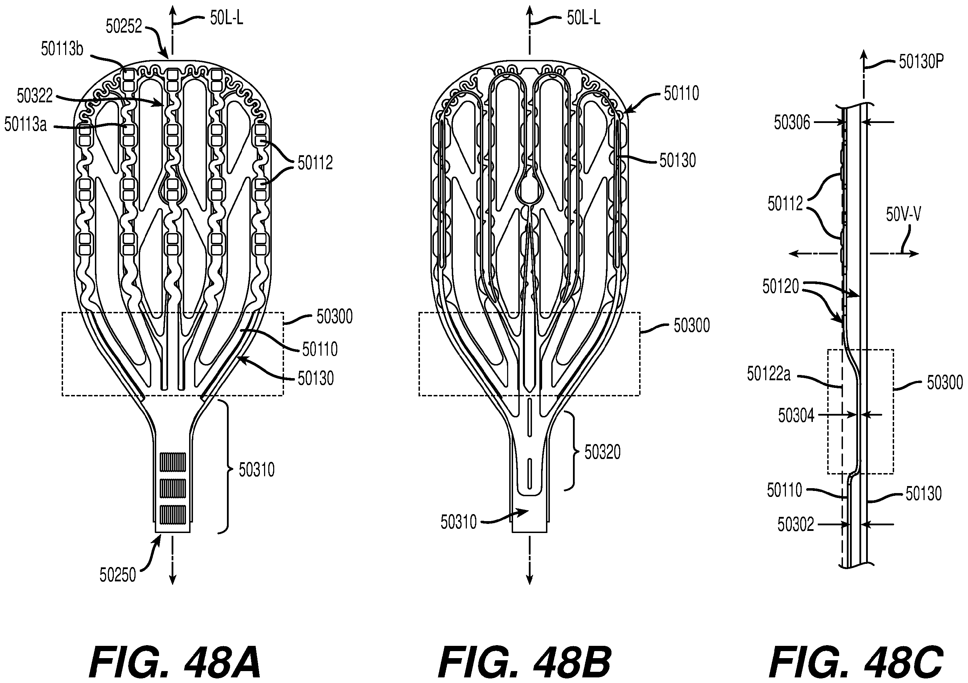

As described throughout this disclosure, end effectors 50100 according to the examples described are to be flexible in order to traverse the patient's vasculature. However, when the flexible circuits (e.g., first 50110 and second 50140 flexible circuits) are positioned farther away from a neutral axis (e.g., framework 50130), for example when there is a layer of insulative material 50120 as shown in FIGs. 45, 46A, and 46B, considerable tensile and compressive strain can be placed on both the insulative material 50120 and the flexible circuits. The strain can be the highest at locations of the end effector 50100 that bend the most. Accordingly, the present disclosure provides a solution to issues with strains applied to the circuits when they are bending. FIGs. 47-50B show such example solutions by providing a planar transition zone 50300 positioned proximally on the end effector 50100 at a location of the highest amount of bend for the end effector 50100 as it is traversing the vasculature. In this planar transition zone 50300, the flexible circuit positioned on that side of the end effector 50100 transitions from a first plane closer to the neutral plane (see 50130P in FIGs. 48C and 49C) to a second plane farther from the neutral plane.

Referring now to FIG. 47, the end effector 50100 shown therein is like that which is shown in FIG. 45 but with the addition of the planar transition zone 50300. The example shown in the figure includes a framework 50130 as described above, and also includes a first flexible circuit 50110 on one side of the framework 50130 and a second flexible circuit 50140 on a second side of the framework 50130. It is contemplated, as is described with reference to FIG. 48A, that the end effector 50100 only includes a flexible circuit on one side. A proximal end 50250 of the end effector 50100 includes tails of the respective components, the tails extending into a sheath 50210 (see FIG. 44) and supporting the end effector 50100. To illustrate, the flexible circuits can include one or more circuit tails 50310. The example shown in FIG. 47 includes a first flexible circuit 50110 with a first circuit tail 50310a, and includes a second flexible circuit 50140 with a second circuit tail 50310b. The framework 50130 also includes a framework tail 50320 (see also FIG. 48B).

The transitions between the tail sections (e.g., tails 50310, 50310a, 50310b, and/or 50322) and the more distal portions of the end effector 50100 can experience the most bending strain as the end effector 50100 is deployed or used, as such this can be a location of the planar transition zone 50300, i.e., the planar transition zone 50300 can be positioned distal to the tail sections (e.g., tails 50310, 50310a, 50310b, and/or 50322). Referring now to the first flexible circuit 50110, the circuit near the first circuit tail 50310a can be positioned in a plane that is at a first distance 50302 from the framework 50130. Moving distally, the first flexible circuit 50110 transitions to a second plane that is at a second distance 50304 from the framework 50130, the second distance 50304 being less than (i.e., a shorter distance) than the first distance 50302. Moving distally, the first flexible circuit 50110 then transitions to a third plane that is at a third distance 50306 from the framework 50130, the third distance 50306 being greater than (i.e., a longer distance) than the second distance 50304. As such, the first flexible circuit 50110 transitions in the planar transition zone 50300 from a plane closer to the neutral plane 50130P to a different plane farther from the neutral plane 50130P (see also FIGs. 48C and 49C). The flexible circuits are spaced apart from the framework along a vertical axis 50L-L that is orthogonal to the neutral plane 50130P (see also FIGs. 48C and 49C). The neutral plane in the example shown in FIG. 47 is the plane in which the framework 50130 extends. It is contemplated, however, that the end effector 50100 does not include a framework 50130 (see FIG. 46D), and in these examples the neutral plane 50130P can be located at some point within or on the insulative material 50120. It is also contemplated that end effectors 50100 with frameworks 50130 have a neutral axis that is not coplanar with the framework 50130. Referring again to the transition zone, in some examples the first distance 50302 and the third distance 50306 are the same, such that the section of the first flexible circuit 50110 with the electrodes 50112 near the distal end 50252 is coplanar with the tail 50310a (see also tail 50310 in FIGs. 48A-48C) at the proximal end 50250.

As shown in FIG. 47, the end effector 50100 can include flexible circuits (e.g., first flexible circuit 50110 and second flexible circuit 50140) that include serpentine connections 50322 in the plane that is distal to the planar transition zone 50300. For example, between two adjacent electrodes, e.g., proximal electrode 50113a and distal electrode 50113b, the first flexible circuit 50110 can have a serpentine connections 50322 that provides the non-linear pattern shown that enables a greater degree of bending for the first flexible circuit 50110. In other words, the serpentine connections 50322 enable the first flexible circuit 50110 to bend more easily in the same plane but in an axis transverse to the longitudinal axis 50L-L. The serpentine connections 50322 can begin distal to the planar transition zone 50300, as shown, or the serpentine connections 50322 can begin in the planar transition zone 50300.

FIGs. 48A-48C show an embodiment similar to the one shown in FIG. 47, but the end effector 50100 includes a first flexible circuit 50110 on one side of the framework 50130 and does not include a second circuit (see second flexible circuit in FIG. 47) on the other side. This example could be similar to the one shown in FIG. 46B, but with the addition of the planar transition zone 50300 as shown. The example shows how electrodes 50112 may be placed only on one side of the end effector 50100. FIG. 48A, which is a top plan view of the end effector 50100, shows the side of the end effector 50100 with a first flexible circuit 50110 having electrodes 50112; FIG. 48B, which is a bottom plan view of the end effector 50100, shows a side of the end effector 50100 without a flexible circuit. The views also show the singular circuit tail 50310 (see FIG. 48A) and the framework tail 50320 (see FIG. 48B) that also extends proximally with the circuit tail 50310.

FIG. 48C is a side view of the end effector 50100, and shows details of the distances between the first flexible circuit 50110 and the neutral plane 50130P at different positions along the longitudinal axis 50L-L. In FIG. 48C, the neutral plane 50130P is labeled as coplanar with the framework 50130, which is in accordance with some examples. Proximal to the planar transition zone 50300, the first flexible circuit 50110 is at the first distance 50302, then the first flexible circuit 50110 transitions closer to the neutral plane 50130P in the planar transition zone 50300, i.e., the second distance 50304. Distally, the first flexible circuit 50110 is at the second distance 50304 within the zone, then the first flexible circuit 50110 transitions farther from the neutral plane 50130P, i.e., the third distance 50306. The end effector 50100 can include first insulative layer 50120, as described above, and the first flexible circuit 50110 can transition in or on the first insulative layer 50120. In some examples, at least a portion of the first plurality of electrodes 50112 can extend from a first outer surface 50122a defined by the insulative material 50120, as shown in FIG. 48C. Further, the first outer surface 50122a can remain planar and parallel to the neutral plane 50130P, and the first flexible circuit 50110 can transition with respect to the vertical axis 50V-V in or on the first insulative layer 50120. As will be appreciated, the example shown in FIG. 47 could look substantially similar to the example shown in FIG. 48C, though the device would be mirrored along the plane of the framework 50130 such that the left side would include the first flexible circuit 50110 and a first insulative material 50120, and the right side would include the second flexible circuit 50140 and a second insulative material. As will be appreciated and is described above, the first and second insulative material in this example could be a continuous material-i.e., insulative material 50120 can include a first sheet of insulative material 50120a and a second sheet of insulative material 50120b fused together proximate the framework 50130 into a single, contiguous, generally planar insulative mass 50120.

FIGs. 49A-49C show an embodiment similar to the one shown in FIGs. 47 and 48A-48C, but the end effector 50100 includes a first flexible circuit 50110 on one side of the framework 50130 and a second flexible circuit on the opposite side that comprises an electromagnetic coil 50330. The first flexible circuit 50110 in FIGs. 49A-49C is substantially the same as the circuit discussed with reference to FIGs. 48A-48C. The electromagnetic coil 50330 on the opposite side (FIG. 49B) can be used as a position sensor, as described above with reference to FIG. 43, for locating the end effector 50100 in the patient. For example, the magnetic coils 5032 (see FIG. 43) can generate a magnetic fields, and the electromagnetic coil 50330 can be used to track those magnetic fields when the device is deployed. In some examples, the electromagnetic coil 50330 can be separated into separate loops, for example the distal loop 50334, first side loop 50336a, and second side loop 50336b shown in FIG. 49B. The distal loop 50334, first side loop 50336a, and second side loop 50336b can individually detect the magnetic field, and the current generated at each of the loops can be triangulated to map the position of the end effector 50100.

Referring now to FIG. 49C, the electromagnetic coil 50330 at one side of the end effector 50100 can also include the planar shifts discussed above. In the planar transition zone 50300, the electromagnetic coil 50330 positioned on this side of the end effector 50100 transitions from a first plane closer to the neutral plane (see 50130P in FIG. 49C) to a second plane farther from the neutral plane. The electromagnetic coil 50330 can include a coil tail 50332, similar to the circuit tail 50310 described above. The transition between the coil tail 50332 and the more distal portions of the electromagnetic coil 50330 can experience the most bending strain as the end effector 50100 is deployed or used. As such, the planar transition zone 50300 is positioned distal to the coil tail 50332. Referring now to the electromagnetic coil 50330, the coil near the coil tail 50332 can be positioned in a plane that is at a first distance 50338 from the framework 50130. Moving distally, the electromagnetic coil 50330 transitions to a second plane that is at a second distance 50340 from the framework 50130, the second distance 50340 being less than (i.e., a shorter distance) than the first distance 50338. Moving distally, the electromagnetic coil 50330 then transitions to a third plane that is at a third distance 50342 from the framework 50130, the third distance 50342 being greater than (i.e., a longer distance) than the second distance 50340. As such, the electromagnetic coil 50330 transitions in the planar transition zone 50300 from a plane closer to the neutral plane 50130P (see FIG. 49C) to a different plane farther from the neutral plane 50130P.

As described herein, the end effector 50100 here can include any combination of the circuits described herein on either side of the end effector 50100. To illustrate, the end effector 50100 can have an electrode circuit on one side only (e.g., first flexible circuit 50110 with electrodes 50112, as shown in FIGs. 48A-48C); the end effector 50100 can have electrode circuits on both sides (e.g., first flexible circuit 50110 with electrodes 50112 and second flexible circuit 50140 with electrodes 50142, as shown in FIG. 47); the end effector 50100 can have an electrode circuit on one side and a coil on the other (e.g., first flexible circuit 50110 with electrodes 50112 on one side and electromagnetic coil 50330 on the other, as shown in FIGs. 49A-49C); and the end effector 50100 can have an electromagnetic coil 50330 on one side only and have no electrode circuit on the other side.

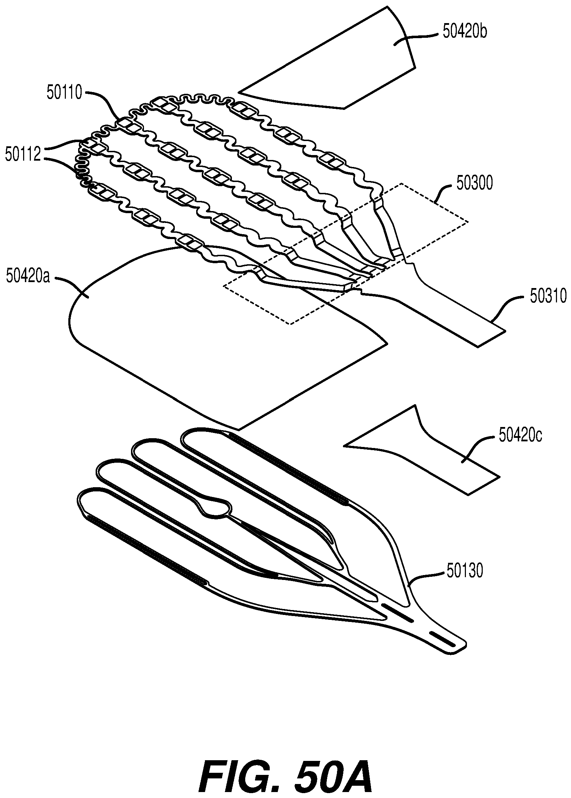

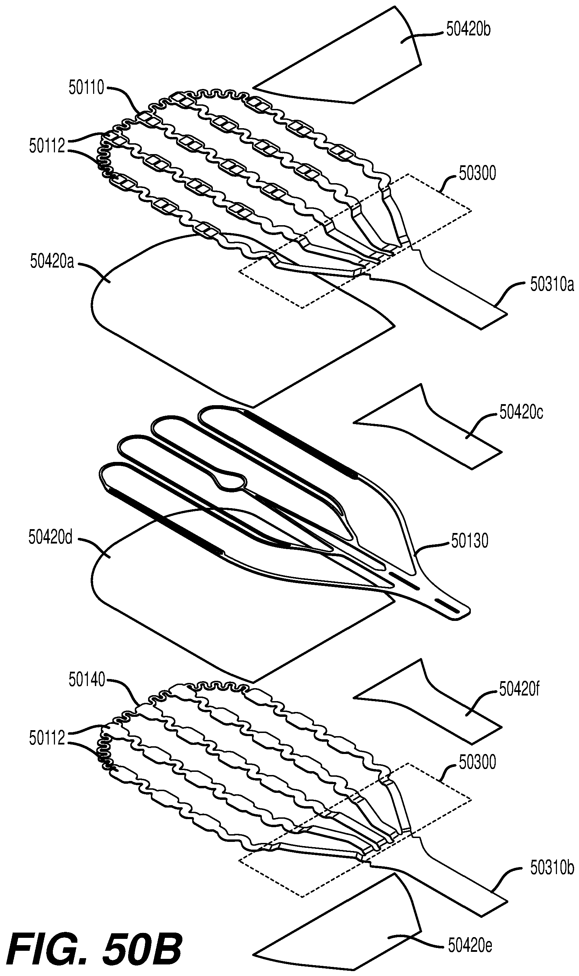

FIG. 50A is an illustration of a perspective, exploded view of an end effector 50100 with a planar transition zone 50300 proximally and alternating levels layers of insulative sheets (e.g., distal insulative material sheet 50420a, intermediate insulative material sheet 50420b, and/or proximal insulative material sheet 50420c). As described above, the flexible circuits and/or electromagnetic coils described herein can be embedded in and/or on an insulative material (see insulative material 50120, first sheet of insulative material 50120a, and second sheet of insulative material 50120b above). As such, the respective circuits can transition planarly as described herein by changing positions within a continuous sheet of insulative material. It is also contemplated that the planar transitions are created by separate sheets of material that have thicknesses equal to the aforementioned distances from the 50130P (see, e.g., FIGs. 48C and 49C). Referring now to the example in FIG. 50A, the end effector 50100 can include a distal insulative material sheet 50420a positioned between the framework 50130 and the first plurality of electrodes 50112 (it will be understood that the example shown in FIG. 50A includes electrodes distally, but the embodiment shown in both FIGs. 50A and 50B could equally apply to any of the examples described herein, including those examples with an electromagnetic coil 50330). The distal insulative material sheet 50420a can taper proximal near the planar transition zone 50300 to enable the circuit on that side of the end effector 50100 to transition nearer to the neutral plane 50130P. The end effector 50100 can include an intermediate insulative material sheet 50420b positioned within the planar transition zone 50300 such that at least a portion of the circuit/coil on that side of the end effector 50100 is positioned between the intermediate insulative material sheet 50420b and the framework 50130. For example, in this section, the circuit (e.g., first flexible circuit 50110) is closer to the neutral plane 50130P and more insulative material is positioned over the circuit. The end effector 50100 can also include a proximal insulative material sheet 50420c positioned between the framework 50130 and a tail (e.g., circuit tail 50310). The proximal insulative material sheet 50420c can taper distally to enable the planar transition of the first flexible circuit 50110. The distal insulative material sheet 50420a can have a thickness equal to the third distances 50306, 50342, the intermediate insulative material sheet 50420b can have a thickness equal to the second distances 50304, 50340, and the proximal insulative material sheet 50420c can have a thickness equal to the first distances 50302, 50338.

FIG. 50B is an illustration of a perspective, exploded view of an end effector 50100 with a planar transition zone 50300 proximally and alternating levels layers of insulative sheets and flexible circuits on both sides of the end effector 50100. The example shown in FIG. 50B is substantially similar to the example shown in FIG. 50A, expect the example shown in FIG. 50B includes a circuit (e.g., second flexible circuit 50140) on the opposite side of the end effector from the first circuit 50110. The top side of the image is the same as shown in FIG. 50A, though the circuit tail is labeled as first circuit tail 50310a. The opposite side includes a second distal insulative material sheet 50420d, a second intermediate insulative material sheet 50420e, and a second proximal insulative material sheet 50420f.

The second distal insulative material sheet 50420d can taper proximal near the planar transition zone 50300 to enable the circuit on that side of the end effector 50100 to transition nearer to the neutral plane 50130P. The end effector 50100 can include a second intermediate insulative material sheet positioned within the planar transition zone 50300 such that at least a portion of the circuit/coil on that side of the end effector 50100 is positioned between the second intermediate insulative material sheet 50420e and the framework 50130. For example, in this section, the circuit (e.g., second flexible circuit 50140) is closer to the neutral plane 50130P and more insulative material is positioned more superficially on the circuit. The end effector 50100 can also include a second proximal insulative material sheet 50420f positioned between the framework 50130 and a tail (e.g., second circuit tail 50320b). The second proximal insulative material sheet 50420f can taper distally to enable the planar transition of the second flexible circuit 50140. The second distal insulative material sheet 50420d can have a thickness equal to the third distances 50306, 50342, the second intermediate insulative material sheet 50420e can have a thickness equal to the second distances 50304, 50340, and the second proximal insulative material sheet 50420f can have a thickness equal to the first distances 50302, 50338.

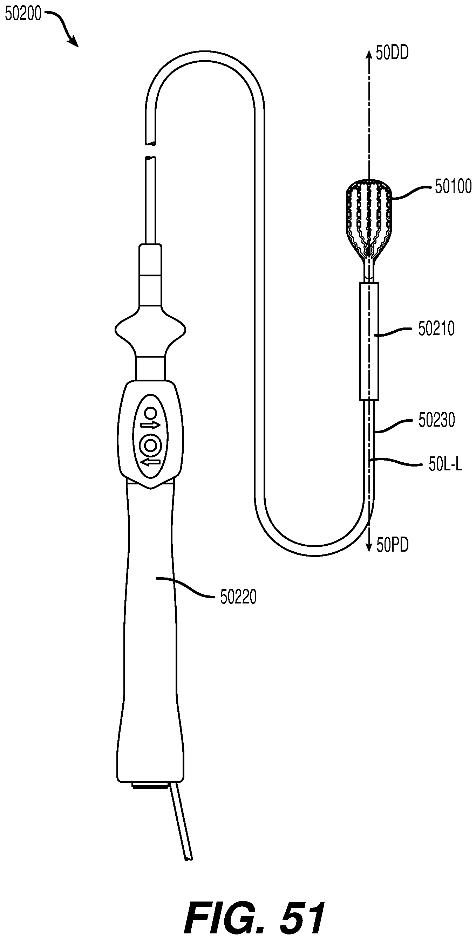

The present disclosure provides a catheter assembly 50200 as shown in FIG. 51 which can include a tubular member 50230 extending along a longitudinal axis 50L-L and configured to deliver end effector 50100 to an out of a sheath 50210. A physician 5024 can manipulate the catheter 200 with handle 50220. Appropriate examples for catheter assembly 50200 and its subcomponents such as handle 50220, sheath 50210, tubular member 50230, and others not mentioned herein are described in US Patent publication No. 2021/0369339, which is incorporated herein by reference and included in the Appendix of Priority Application No. 63/615,574.

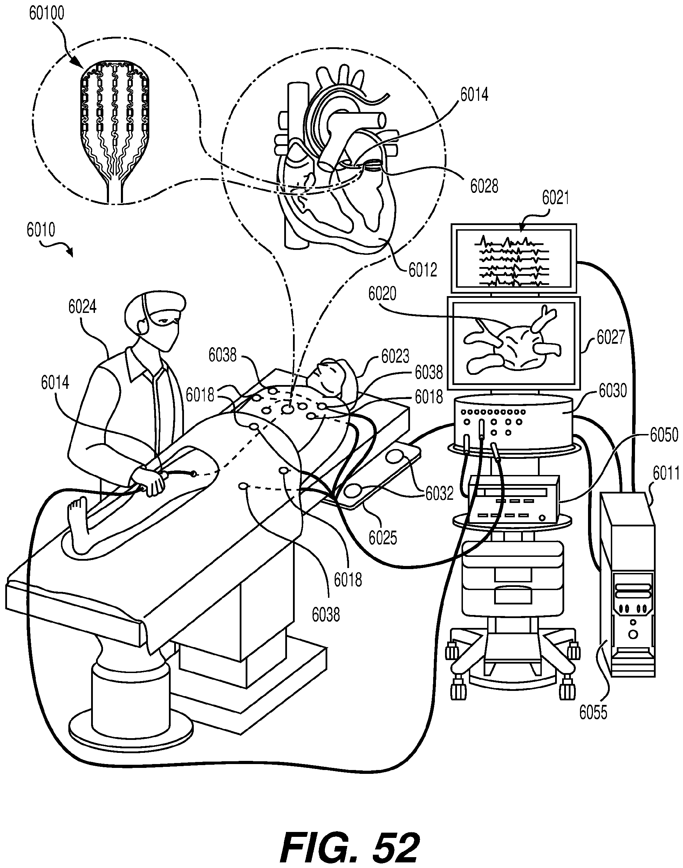

### Serpentine Flexible Circuits for Medical Probe (FIGs. 52-58)

Reference is made to FIG. 52 showing an example catheter-based electrophysiology mapping and ablation system 6010. System 6010 includes multiple catheters, which are percutaneously inserted by physician 6024 through the patient's 6023 vascular system into a chamber or vascular structure of a heart 6012. Typically, a delivery sheath catheter is inserted into the left or right atrium near a desired location in heart 6012. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 6014 that is configured for sensing IEGM is illustrated herein. Physician 6024 brings a catheter shaft with distal tip of catheter 6014 (i.e., multilayered end effector 60100) into contact with the heart wall for sensing a target site in heart 6012. For ablation, physician 6024 would similarly bring a distal end of an ablation catheter to a target site for ablating.

Catheter 6014 is an exemplary catheter that includes one and preferably multiple electrodes 60112 optionally distributed over end effector 60100 coupled to a catheter shaft and configured to sense the IEGM signals as described in more detail below. Catheter 6014 may additionally include a position sensor embedded in or near end effector 60100 for tracking position and orientation of end effector 60100. Optionally and preferably, position sensor is a magnetic based position sensor including multiple magnetic coils for sensing three-dimensional (3D) position and orientation.

Magnetic based position sensor may be operated together with a location pad 6025 including a plurality of magnetic coils 6032 configured to generate magnetic fields in a predefined working volume. Real time position of end effector 60100 of catheter 6014 may be tracked based on magnetic fields generated with location pad 6025 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 6010 includes one or more electrode patches 603 8 positioned for skin contact on patient 6023 to establish location reference for location pad 6025 as well as impedance-based tracking of electrodes 60112. For impedance-based tracking, electrical current is directed toward electrodes 60112 and sensed at electrode skin patches 6038 so that the location of each electrode can be triangulated via the electrode patches 6038. Details of the impedance-based location tracking technology are described in US Patent Nos. 7,536,218; 7,756,576; 7,848,787; 7,869,865; and 8,456,182, each of which are incorporated herein by reference.

A recorder 6011 displays electrograms 6021 captured with body surface ECG electrodes 6018 and intracardiac electrograms (IEGM) captured with electrodes 60112 of catheter 6014. Recorder 6011 may include pacing capability for pacing the heart rhythm and/or may be electrically connected to a standalone pacer.

System 6010 may include an ablation energy generator 6050 that is adapted to conduct ablative energy to one or more of electrodes 60112 at a distal tip of a catheter configured for ablating. Energy produced by ablation energy generator 6050 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 6030 is an interface configured to establish electrical communication between catheters, electrophysiological equipment, power supply and a workstation 6055 for controlling operation of system 6010. Electrophysiological equipment of system 6010 may include for example, multiple catheters, location pad 6025, body surface ECG electrodes 6018, electrode patches 6038, ablation energy generator 6050, and recorder 6011. Optionally and preferably, PIU 6030 additionally includes processing capability for implementing real-time computations of location of the catheters and for performing ECG calculations.

Workstation 6055 includes memory, processor unit with memory or storage with appropriate operating software loaded therein, and user interface capability. Workstation 6055 may provide multiple functions, optionally including (1) modeling the endocardial anatomy in three-dimensions (3D) and rendering the model or anatomical map 6020 for display on a display device 6027, (2) displaying on display device 6027 activation sequences (or other data) compiled from recorded electrograms 6021 in representative visual indicia or imagery superimposed on the rendered anatomical map 6020, (3) displaying real-time location and orientation of multiple catheters within the heart chamber, and (4) displaying on display device 27 sites of interest such as places where ablation energy has been applied. One commercial product embodying elements of the system 6010 is available as the CARTOTM 3 System, available from Biosense Webster, Inc., 31 Technology Drive, Suite 200, Irvine, CA 92618.

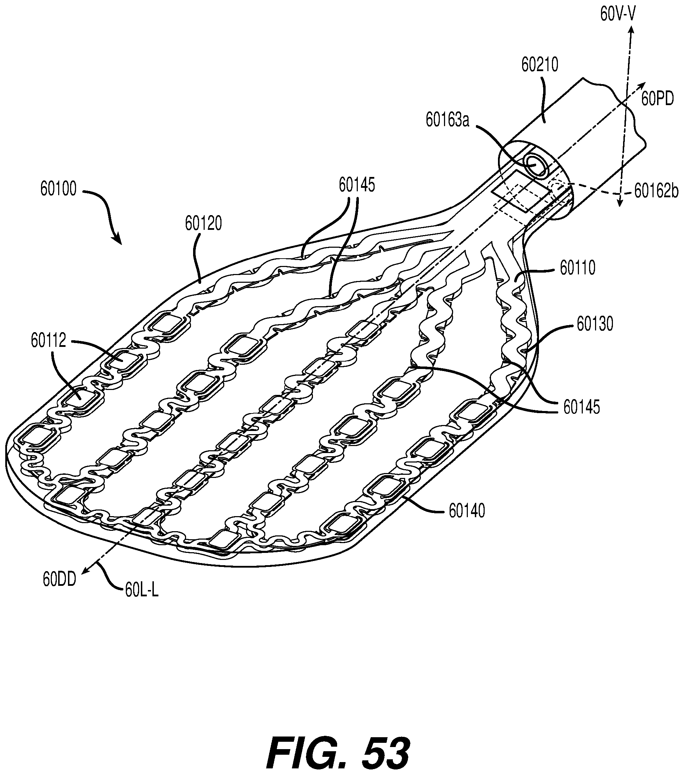

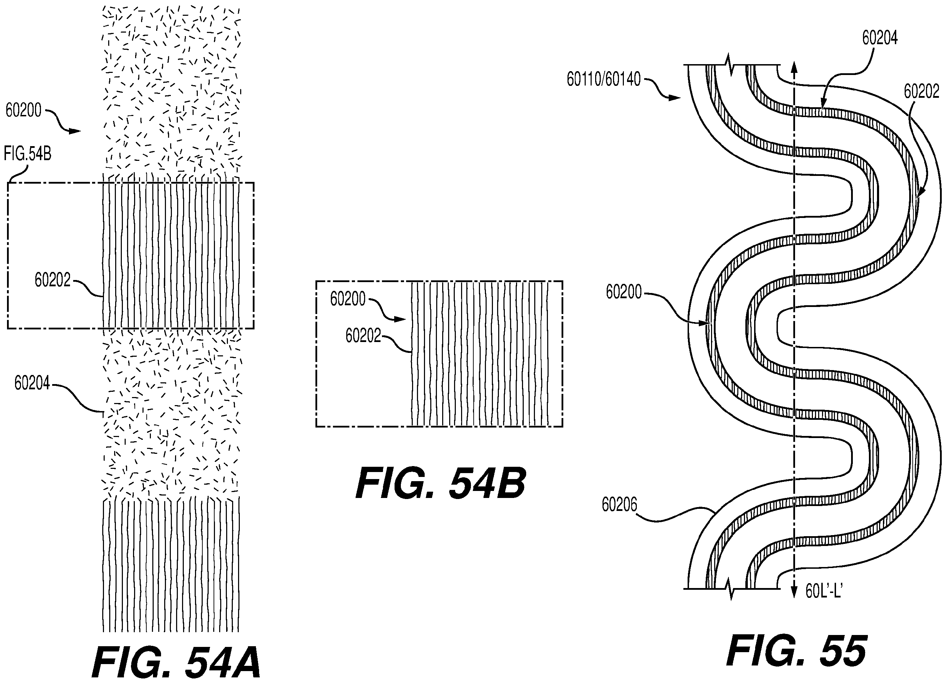

FIG. 53 provides an end effector 60100 in accordance with an embodiment of the present disclosure in order to achieve enhanced end effector properties, such as desired stiffness, electrode contact with target anatomy, and conformity of the end effector disclosed herein to flat, curved, irregular and/or nonplanar tissue surfaces found in the target anatomy. The end effector 60100 can include a flexible circuit 60110 including a plurality of electrodes 60112, each electrode of the plurality of electrodes 60112 including a contact surface 60112c. As used herein, the term "flexible circuit" includes the components of an electrical trace (e.g., electrical trace 60200) positioned on a substrate (e.g., substrate 60206). Additional information regarding example electrical traces and substrates is provided below with respect to FIGs. 54-56. The end effector 60100 can further include a second flexible circuit 60140 having a second plurality of electrodes (positioned below the electrodes 60112). The second flexible circuit 60140 can be substantially similar to the first flexible circuit 60110, and each electrode of the second plurality of electrodes can have a second contact surface. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The first flexible circuit 60110 and/or second flexible circuit 60140 (referred to collectively as flexible circuit(s) 60110, 60140) can be disposed on or within an insulative material 60120. The insulative material 60120 can be contiguous to the contact surfaces 60112c so that only the contact surfaces 60112c of at least a portion of the plurality of electrodes 60112 are exposed to the ambient environment. As used herein, the term "contact surface" includes the portion of an electrode having a generally flat surface and the edge or edges which immediately surround said flat surface. Electrodes 60112 may have a slightly rounded, radiused, or chamfered edge that contacts tissue, along with the generally flat surface, when the end effector 60100 is placed against tissue. As used herein, "ambient environment" refers to the external environment such as the organ in which the end effector 60100 is deployed or in the operating theater prior to being deployed in the biological organ.

It is noted that not all the electrodes 60112 on the end effector 60100 described herein must be exposed through the insulative material 60120, as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 60163a on one side and 60162b on the other side in fluid communication with an irrigation line not shown disposed in a catheter shaft. Instead of an irrigation line separate from the catheter shaft, a lumen can be formed via extrusion of the catheter shaft to provide for a lumen channel. It is noted that port 60163a or 60162b can be configured to have a sufficient flow diverter characteristic for irrigation fluid to cover the mapping electrodes during irrigation flow so as to prevent or reduce thrombus formations.

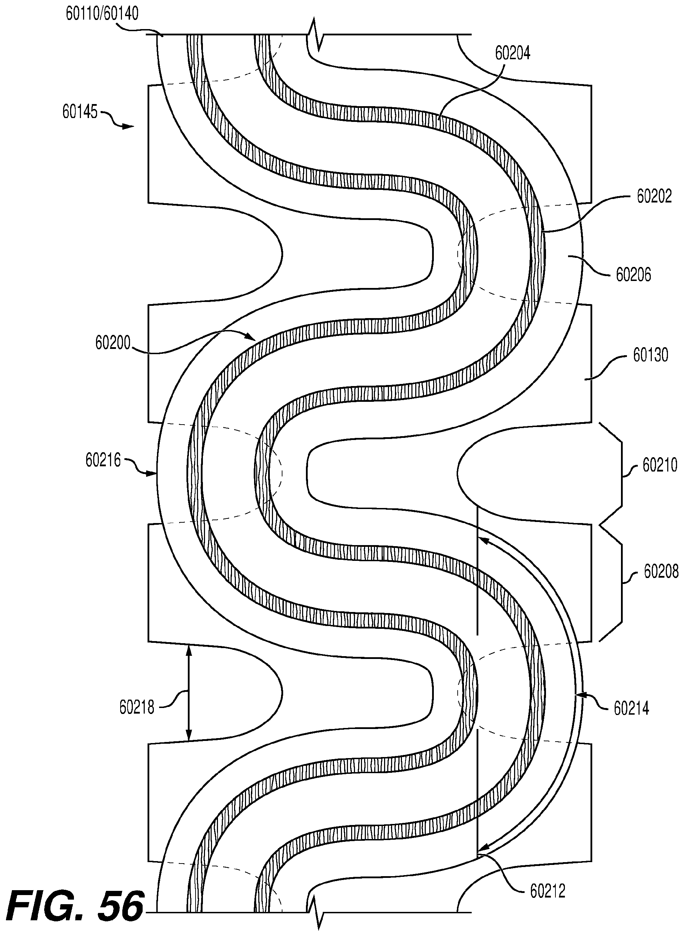

The end effector 60100 further includes a framework 60130 to support the flexible circuit(s) 60110, 60140. The framework 60130 can be a flexible material that allows the end effector 60100 to traverse vasculature. It is contemplated that the framework 60130 is a shape-memory material such as copper-aluminum-nickel or preferably nickel-titanium (NiTi/Nitinol) alloys, and/or alloys including of zinc, copper, and gold. Additional information about example frameworks 60130 is provided below with respect to FIG. 56. The framework 60130 can be contiguous to the insulative material 60120 or inside the insulative material 60120. The flexible circuit(s) 60110, 60140 can be disposed directly on the framework 60130 with none, or very little, of the insulative material 60120 coming between the two. In other examples, the framework 60130 and the flexible circuit(s) 60110, 60140 cane spaced be apart along a vertical axis 60V-V on different planes. The framework 60130 can include one or more struts 60142 extending along a longitudinal axis 60L-L of the end effector 60100. Each strut 60145 can include individual strands of the electrical traces 60200 extending along the longitudinal axis 60L'-L' of each strut 60142.

FIG. 54A is a detailed view of an electrical trace 60200 showing sections of alternating grain lengths in their crystal structure, and FIG. 54B is a detailed view of rolled annealed metal, in accordance with the disclosed technology. The electrical traces 60200 in any embodiment described herein are electrical conductors that connect the one or more electrodes 60112 (see FIG. 53) to signal and/or current generators (e.g., PIU 6030 in FIG. 52) or receivers. The electrical traces 60200 can be positioned onto a substrate 60206, as will be described with reference to FIG. 55. The electrical trace 60200 can be, for example, thin film deposited via lithography and/or etching processes on the substrate 60604, or can simply be adhered or otherwise applied to the substrate 60604. The electrical traces 60200 comprise a rolled annealed metal, such as rolled annealed metals comprising copper or alloys thereof, for example copper, manganese, and nickel alloys. In a preferred embodiment, rolled annealed copper is used for the electrical trace 60200, as will be described below. Rolled annealed metals are preferred due to their flexibility. In terms of manufacturing, rolled annealed metals can start as electrodeposited metals with a more vertical crystalline grain structure. These electrodeposited metals are then subjected to the rolling and annealing process, which stretches and elongates the grain structure, increasing their flexibility and capacity for mechanical bending. Furthermore, the rolled structure forms individual grains (i.e., individual longer grains 60202 shown in FIG. 54B) that all run essentially parallel to the direction of the roll of the rolled annealed metal.

The alternating structure can be formed, for example, by annealing and/or rolling the metallic foil to a greater degree at a location of the longer grain sections 60202. FIG. 54A shows such an example wherein the crystal structures are alternating. In practice, however, manufacturing these alternating crystal structures may be difficult, and the alternating of crystal structures can be undesired as they may provide areas for breaking or other failure. Accordingly, the alternative design is to provide longer grain sections 60202 and shorter grain sections 60204 via the cutting process. FIG. 54B shows examples of an electrical trace 60200 that is rolled such that all longer grain section 60202 are positioned parallel to the direction of the roll; the direction of the roll can then be parallel to a direction of each individual strut 60145 (see FIG. 55). Instead of having differing grain structures throughout, the roll is substantial uniform along its length, and the electrical trace 60200 is formed to be parallel to the roll (i.e., the elongated grains) of the rolled annealed metal. The longer grain sections 60202 and shorter grain sections 60204 can therefore be created through the process of cutting out or forming the electrical traces 60200 from the rolled annealed metal. A sheet of rolled annealed metal can be provided with the elongated grains extending lengthwise. The serpentine shape of the electrical trace 60200 can then be cut into the long sheet of rolled annealed metal such that the sections of the serpentine shape in the bends will have longer grains (i.e., the longer grain sections 60202) because they are cut parallel to the roll of metal, and the sections of the serpentine shape intermediate to the bends will have shorter grains (i.e., the shorter grain sections 60204) because they are cut perpendicular to the roll of metal. As such, the alternating structure of longer grain sections 60202 at the bends 60212 and shorter grain sections 60204 outside of the bends 60212 will encourage and facilitate the bending of the electrical trace 60200 at the apexes 60216 (see FIG. 56). This example where the serpentine shape of the electrical trace 60200 is cut into the direction of the roll of the rolled annealed metal is shown in the examples provided in FIGs. 55 and 56. It will be appreciated that the electrical trace 60200 can be cut into the rolled annealed metal via lithography, masking and etching, laser cutting, or similar processes.

FIG. 55 it a top plan view of a flexible circuit 60110 having electrical traces 60200 as shown in FIG. 54B applied to a substrate 60206, in accordance with the disclosed technology. The substrate 60206 can be made primarily of polyimide. In other examples, the substrate 60206 can be made of any of biocompatible polyimides, glass-reinforced epoxy laminate materials, copper, or graphene, alone or in combination. The flexible circuit(s) 60110, 60140 described herein can have a serpentine structure, as shown, that enables a greater degree of bending for the first flexible circuit 60110. In other words, the serpentine layout can enable the flexible circuit(s) 60110, 60140 to flex more easily side-to-side in their plane (i.e., left/right in an axis transverse to the longitudinal axis 60L-L). Referring again to the lamellar structure of the electrical traces 60200, the rolled annealed metal therein has good bending resistance in the direction of the grains (i.e., along longitudinal axis 60L-L, see FIG. 52), but lesser bending resistance orthogonal to the direction of the grains. As such, the alternating grain structures described above can be aligned with the bends 60212 of the electrical trace 60200 (see bends labeled in FIG. 56). The longest continuous sections of grains are therefore positioned at a location of the plurality of bends 60212 in the serpentine structure, and the shorter grain sections 60204 are positioned outside of the bends (e.g., in the seconds of the serpentine pattern that run more perpendicularly to the longitudinal axis 60L'-L'). This orientation of the grains is to encourage bending at the apexes of the bends (see apexes 60214 in FIG. 56).

FIG. 56 it a top plan view of the flexible circuit 60110, 60140 of FIG. 55 applied to a support framework 60130, in accordance with the disclosed technology. To further encourage and facilitate bending at the bends 60212 of the electrical trace 60200, each strut 60145 of the end effector 60100 can include wide sections 60208 and narrow sections 60210 extending along the longitudinal axis 60L'-L' of the strut 60145. The terms "wide" and "narrow" refer to a broadness of the strut 60145 in a direction that runs transverse to the longitudinal axis 60L'-L' of the strut 60145. The narrow sections 60210, therefore, define cutouts 60218. The wide sections 60208 provide more support and therefore the strut 60145 will be encouraged to bend at the narrow sections 60210. Each bend 60212 includes an apex 60214, and the apexes 60214 of the plurality of bends 60212 extend into the cutouts 60218 such that they overhang at least a portion of the strut 60145. The overhang is labeled in FIG. 56 as overhang 60216. In view of the design, the apexes 60214 of the bends 60212 in the electrical trace 60200 are positioned at the narrow sections 60210, the aforementioned longer grains 60202 of the electrical trace 60200 are positioned at the bends 60212 in the narrow sections 60210, and the strut 60145 is be encouraged and facilitated to bend at the narrow sections 60210.

As will be appreciated, nothing requires all the features above to be combined, as any of the beneficial features described herein can be used alone or in combination with any of the other novel features. For example, the formation of electrical traces 60200 with the longer grain sections 60202/shorter grain sections 60204 described herein can be used in any flexible circuit that includes bends; struts 60145 with wide sections 60208 and narrow sections 60210 can be used in any flexible circuit that includes bends 60212, or even with any flexible circuit that does not have the serpentine bends 60212; and the example flexible circuit 60110, 60140 shown in FIG. 55 can be used on a framework 60130 that does not include the wide sections 60208 and narrow sections 60210.

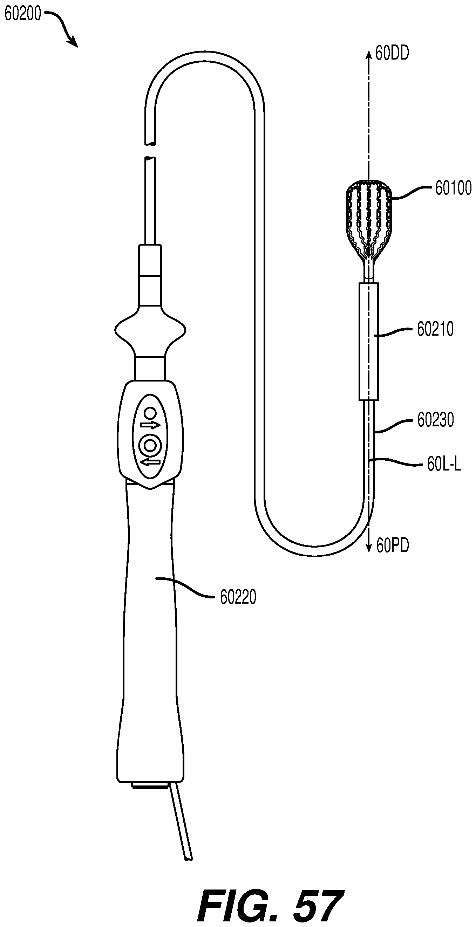

The present disclosure provides a catheter assembly 60300 as shown in FIG. 57 which can include a tubular member 60230 extending along a longitudinal axis 60L-L and configured to deliver end effector 60100 to an out of a sheath 60240. A physician 6024 can manipulate the catheter 60300 with handle 60220. Appropriate examples for catheter assembly 60300 and its subcomponents such as handle 60220, sheath 60240, tubular member 60230, and others not mentioned herein are described in US Patent publication No. 2021/0369339, which is incorporated herein by reference and included in the Appendix of Priority Application No. 63/615,947.

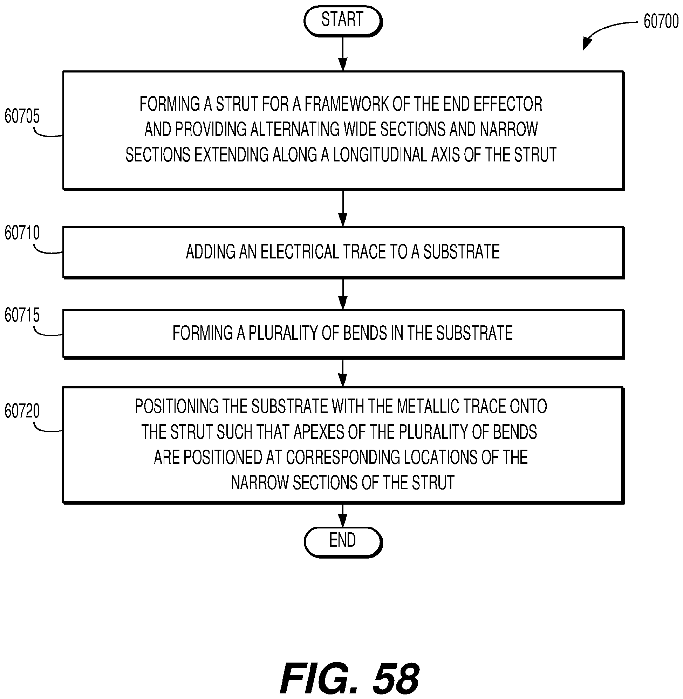

FIG. 58 is a flowchart showing a method 60700 of manufacturing an end effector 60100 for a medical catheter, in accordance with the disclosed technology. Method 60700 includes forming 60705 a strut 60145 for a framework 60130 of the end effector 60100 and providing alternating wide sections 60208 and narrow sections 60210 extending along a longitudinal axis 60L'-L' of the strut 60145. Method 60700 includes disposing 60710 an electrical trace 60200 on a substrate 60206. Method 60700 includes forming 60715 a plurality of bends 60212 in the substrate 60206 and the electrical trace 60200. Method 60700 includes aligning 60720 the substrate 60206 and the electrical trace 60200 with the strut 60145 such that apexes 60214 of the plurality of bends 60212 are positioned at corresponding locations of the narrow sections 60210 of the strut 60145. As mentioned above, the electrical trace 60200 can include a rolled annealed metal, preferably a rolled annealed copper or alloy of copper.

Method 60700 can end after step 60720, while in other examples additional steps can be performed according to the present disclosure. For example, method 60700 can include forming a serpentine shape from the rolled annealed metal by cutting the electrical trace along a direction of individual grains of the rolled annealed metal such that the individual grains run parallel to the longitudinal axis of the strut. The electrical trace can have longer grain sections and shorter grain sections. The longer grain sections 60202 can be positioned at a location of the plurality of bends 60212. The narrow sections 60210 define cutouts 60218, and the apexes 60214 of the plurality of bends 60212 extend into the cutouts 60218 such that they overhang (see overhang 60216 in FIG. 56) at least a portion of the first strut 60145.

The disclosed technology described herein can be further understood according to the following clauses:
Clause 1: An end effector comprising: an insulative material having a first outer surface and a second outer surface; a framework disposed in the insulative material; and a first flexible circuit comprising a first plurality of electrodes, the first flexible circuit disposed longitudinally along and at least partially within the insulative material and being positioned such that a first portion of the first flexible circuit is contiguous to a first plane and a second portion of the first flexible circuit is contiguous to a second plane, the first plane being a first distance from the framework and the second plane being a second distance from the framework, the second distance being less than the first distance.
Clause 2: The end effector of clause 1, each electrode of the first plurality of electrodes comprising a first contact surface positioned at the first outer surface.
Clause 3: The end effector of clause 1 or 2, the first outer surface comprises at a first height from the framework, and the first distance is equal to the first height.
Clause 4: The end effector of any one of the preceding clauses, the first flexible circuit comprising a first superficial planar section extending a first length within the first plane, the first superficial planar section extending parallel to the framework.
Clause 5: The end effector of clause 4, a first electrode of the first plurality of electrodes being disposed within the first superficial planar section.
Clause 6: The end effector of clause 4, a first electrode and a second electrode of the first plurality of electrodes being disposed within the first superficial planar section.
Clause 7: The end effector of any one of clauses 4 to 6, the first superficial planar section being positioned on the first outer surface.
Clause 8: The end effector of any one of the preceding clauses, the first flexible circuit comprising a first medial planar section extending a second length within the second plane, the first medial planar section extending parallel to the framework.
Clause 9: The end effector of clause 8 when dependent from any one of clauses 4 to 7 further comprising a plurality of medial planar sections and a plurality of superficial planar sections.
Clause 10: The end effector of any one of the preceding clauses, the second distance being approximately zero such that the second portion of the first flexible circuit contacts the framework.
Clause 11: The end effector of any one of the preceding clauses, the second portion of the first flexible circuit being positioned at portions of the end effector configured to be bent when in use.
Clause 12: The end effector of any one of the preceding clauses, the first portion and the second portion being connected by an angled section.
Clause 13: The end effector of clause 12, an angle of the angled section changing as the first portion bends or moves with respect to the second portion.
Clause 14: The end effector of any one of the preceding clauses, the second outer surface comprises a second height from the framework, the end effector further comprising: a second flexible circuit comprising a second plurality of electrodes, the second flexible circuit disposed longitudinally along and at least partially within the insulative material and being positioned such that a third portion of the second flexible circuit is contiguous to a third plane and a fourth portion of the second flexible circuit is contiguous to a fourth plane, the third plane being a third distance from the framework and the fourth plane being a fourth distance from the framework, the fourth distance being shorter than the third distance.
Clause 15: The end effector of clause 14, each electrode of the second plurality of electrodes comprising a second contact surface positioned on the second outer surface.
Clause 16: The end effector of clause 14 or 15, the third distance being equal to the second height.
Clause 17: The end effector of any one of clauses 14 to 16, the second flexible circuit comprising a second superficial planar section extending a second length within the third plane, the second superficial planar section extending parallel to the framework.
Clause 18: The end effector of clause 17, a third electrode of the second plurality of electrodes being disposed within the second superficial planar section.
Clause 19: The end effector of clause 17, a third electrode and fourth electrode of the first plurality of electrodes being disposed within the second superficial planar section.
Clause 20: The end effector of any one of clauses 17 to 19, the second superficial planar section being positioned on the second outer surface.
Clause 21: The end effector of any one of clause 14 to 20, the first flexible circuit comprising a second medial planar section extending a second length within the second plane, the second medial planar section extending parallel to the framework.
Clause 22: The end effector of any one of the preceding clauses further comprising a first support layer positioned within the insulative material between the framework and the first flexible circuit.
Clause 23: The end effector of clause 22, the first support layer comprising a polymer film.
Clause 24: The end effector of clause 22, the first support layer comprising a mesh polymer.
Clause 25: The end effector of any one of clauses 22 to 24, the insulative material comprising at least one of thermoplastic polyurethane (TPU), silicone, or siloxane.
Clause 26: The end effector of any one of clauses 22 to 25, the first support layer comprising at least one of polyamide or ethylene Tetrafluoroethylene (ETFE).
Clause 27: The end effector of clause 1, the first flexible circuit having a corrugate profile disposed in the insulative material.
Clause 28: An end effector comprising: an insulative material having a first outer surface and a second outer surface; a framework disposed in the insulative material; a first flexible circuit comprising a first plurality of electrodes, the first flexible circuit disposed on or within the insulative material and each electrode of the first plurality of electrodes comprising a first contact surface, the insulative material being contiguous to the first contact surfaces so that only the first contact surfaces of at least a portion of the first plurality of electrodes are exposed to an ambient environment; and a first support layer positioned within the insulative material between the framework and the first flexible circuit.
Clause 29: The end effector of clause 28, the first support layer comprising a polymer film.
Clause 30: The end effector of clause 28 or 29, the first support layer comprising a mesh polymer.
Clause 31: The end effector of any one of clauses 28 to 30, the insulative material comprising at least one of thermoplastic polyurethane (TPU), silicone, or siloxane.
Clause 32: The end effector of any one of clauses 28 to 31, the first support layer comprising at least one of polyamide or ethylene Tetrafluoroethylene (ETFE).
Clause 33: The end effector of any one of clauses 28 to 32 further comprising: a second flexible circuit comprising a second plurality of electrodes, the second flexible circuit disposed on or within the insulative material and each electrode of the second plurality of electrodes comprising a second contact surface, the insulative material being contiguous to the second contact surfaces so that only the second contact surfaces of at least a portion of the second plurality of electrodes are exposed to the ambient environment.
Clause 34: The end effector of clause 33 further comprising a second support layer positioned within the insulative material between the framework and the second flexible circuit.
Clause 35: The end effector of clause 34, the second support layer comprising a polymer film.
Clause 36: The end effector of clause 34, the second support layer comprising a mesh polymer.
Clause 37: The end effector of any one of clauses 34 to 36, the second support layer comprising at least one of polyamide or ethylene Tetrafluoroethylene (ETFE).
Clause 38: The end effector of any one of clauses 28 to 37, the first flexible circuit is disposed longitudinally along and at least partially within the insulative material and is positioned such that a first portion of the first flexible circuit is contiguous to a first plane and a second portion of the first flexible circuit is contiguous to a second plane, the first plane being a first distance from the framework and the second plane being a second distance from the framework, the second distance being shorter than the first distance.
Clause 39: The end effector of clause 38, the first outer surface comprises at a first height from the framework, and the first distance is equal to the first height.
Clause 40: The end effector of clause 38 or 39, the first flexible circuit comprising a first superficial planar section extending a first length within the first plane, the first superficial planar section extending parallel to the framework.
Clause 41: The end effector of clause 40, a first electrode of the first plurality of electrodes being disposed within the first superficial planar section.
Clause 42: The end effector of clause 40, a first electrode and a second electrode of the first plurality of electrodes being disposed within the first superficial planar section.
Clause 43: The end effector of any one of clauses 40 to 42, the first superficial planar section being positioned on the first outer surface.
Clause 44: The end effector of any one of clauses 38 to 43, the first flexible circuit comprising a first medial planar section extending a second length within the second plane, the first medial planar section extending parallel to the framework.
Clause 45: The end effector of clause 44 when dependent from any one of clauses 39 to 42 further comprising a plurality of medial planar section and a plurality of superficial planar sections.
Clause 46: The end effector of any one of clauses 38 to 45, the second distance being approximately zero such that the second portion of the first flexible circuit contacts the framework.
Clause 47: The end effector of any one of clauses 38 to 46, the second portion of the first flexible circuit being positioned at bending portions of the end effector.
Clause 48: The end effector of any one of clauses 38 to 47, the first portion and the second portion being connected by an angled section.
Clause 49: The end effector of clause 48, an angle of the angled section changing as the first portion bends or moves with respect to the second portion.
Clause 50: The end effector of any one of clauses 38 to 49, the second outer surface comprises a second height from the framework, the end effector further comprising: a second flexible circuit comprising a second plurality of electrodes, the second flexible circuit disposed longitudinally along and at least partially within the insulative material and being positioned such that a third portion of the second flexible circuit is contiguous to a third plane and a fourth portion of the second flexible circuit is contiguous to a fourth plane, the third plane being a third distance from the framework and the fourth plane being a fourth distance from the framework, the fourth distance being shorter than the third distance.
Clause 51: The end effector of clause 50, each electrode of the second plurality of electrodes comprising a second contact surface positioned on the second outer surface.
Clause 52: The end effector of clause 50 or 51, the third distance being equal to the second height.
Clause 53: The end effector of any one of clauses 50 to 52, the second flexible circuit comprising a second superficial planar section extending a second length within the third plane, the second superficial planar section extending parallel to the framework.
Clause 54: The end effector of clause 53, a third electrode of the second plurality of electrodes being disposed within the second superficial planar section.
Clause 55: The end effector of clause 53, a third electrode and fourth electrode of the first plurality of electrodes being disposed within the second superficial planar section.
Clause 56: The end effector of any one of clauses 53 to 55, the second superficial planar section being positioned on the second outer surface.
Clause 57: The end effector of any one of clauses 50 to 56, the first flexible circuit comprising a second medial planar section extending a second length within the second plane, the second medial planar section extending parallel to the framework.
Clause 58: An end effector comprising: an insulative material having a first outer surface and a second outer surface; a framework disposed in the insulative material; and a first flexible circuit having a corrugate profile disposed in the insulative material, the first flexible circuit having a first portion contiguous to the insulative material and exposed to ambient environment, the first flexible circuit being spaced apart from the framework.
Clause 59: The end effector of clause 58 further comprising a second flexible circuit having a corrugate profile disposed in the insulative material, the second flexible circuit having a second portion contiguous to the insulative material and exposed to ambient environment, the second flexible circuit being spaced apart from the framework.
Clause 60: The end effector of clause 59, the first portion of the first flexible circuit comprising a first plurality of electrodes, the second portion of the second flexible circuit comprising a second plurality of electrodes.
Clause 61: The end effector of clause 60, the first plurality of electrodes are aligned across from the second plurality of electrodes longitudinally along a length of the end effector.
Clause 62: The end effector of clause 60, a first position of the first plurality of electrodes is offset from a second position the second plurality of electrodes longitudinally along a length of the end effector.
Clause 63: The end effector of clause 58 further comprising a first support layer positioned within the insulative material between the framework and the first flexible circuit.
Clause 64: The end effector of clause 63, the first support layer comprising a polymer film.
Clause 65: The end effector of clause 63, the first support layer comprising a mesh polymer.
Clause 66: The end effector of any one of clauses 63 to 65, the insulative material comprising at least one of thermoplastic polyurethane (TPU), silicone, or siloxane.
Clause 67: The end effector of any one of clauses 63 to 66, the first support layer comprising at least one of polyamide or ethylene Tetrafluoroethylene (ETFE).
Clause 68: An end effector for a catheter comprising: a first flexible circuit extending along a longitudinal axis from a proximal portion to a distal portion of the end effector; and a second flexible circuit extending along the longitudinal axis from the proximal portion to the distal portion of the end effector, the second flexible circuit being substantially coplanar to the first flexible circuit in the distal portion of the end effector and disposed on top of the first flexible circuit in the proximal portion of the end effector.
Clause 69: The end effector of Clause 68, the first flexible circuit comprises one or more first electrodes disposed on a distal portion of the first flexible circuit.
Clause 70: The end effector of Clause 69, a proximal portion of the first flexible circuit comprises one or more first electrical contacts corresponding to the one or more first electrodes.
Clause 71: The end effector of Clause 70, the first flexible circuit comprises one or more first traces connecting the one or more first electrical contacts to the one or more first electrodes.
Clause 72: The end effector of any one of Clauses 69 to 71, the one or more first electrodes comprise a plurality of pairs of first electrodes disposed on the distal portion of the first flexible circuit.
Clause 73: The end effector of Clause 72, each first electrode of each pair of first electrodes is spaced apart a first predetermined longitudinal distance and each pair of first electrodes is spaced apart from adjacent pairs of first electrodes a second predetermined longitudinal distance, the second predetermined longitudinal distance being greater than the first predetermined longitudinal distance.
Clause 74: The end effector of Clause 73, the first predetermined longitudinal distance is about 100 microns.
Clause 75: The end effector of any one of Clauses 69-74, each of the one or more first electrodes comprises a length of about 500 microns and a width of approximately 500 microns.
Clause 76: The end effector of any one of Clauses 69-75, the second flexible circuit comprises one or more second electrodes disposed on a distal portion of the second flexible circuit.
Clause 77: The end effector of Clause 76, a proximal portion of the second flexible circuit comprises one or more second electrical contacts corresponding to the one or more second electrodes.
Clause 78: The end effector of Clause 77, the second flexible circuit comprises one or more second traces connecting the one or more second electrical contacts to the one or more second electrodes.
Clause 79: The end effector of any one of Clauses 76 to 78, the one or more second electrodes comprise a plurality of pairs of second electrodes disposed on the distal portion of the second flexible circuit.
Clause 80: The end effector of any one of Clauses 75 to 79, the end effector further comprises a frame, and the distal portion of the first flexible circuit is disposed on the frame and the distal portion of the second flexible circuit is disposed on the frame, and a proximal portion of the first flexible circuit is disposed on the frame and a proximal portion of the second flexible circuit is disposed on a portion of the proximal portion of the first flexible circuit.
Clause 81: The end effector of Clause 80, the frame comprising a first, a second, a third, and a fourth spine; the distal portion of the first flexible circuit comprising a first loop disposed over the first spine and the second spine; and the distal portion of the second flexible circuit comprising a second loop disposed over the third spine and the fourth spine.
Clause 82: The end effector of Clause 81, the frame further comprising at least a partial gap between the first, the second, the third, and the fourth spines.
Clause 83: The end effector of any one of Clauses 80 to 82, the frame comprises nitinol.
Clause 84: The end effector of any one of Clauses 68-83, the distal portion of the end effector comprises a width of about 9 millimeters.
Clause 85: The end effector of any one of Clauses 68-84, the distal portion of the end effector comprises a width of about 20 millimeters.
Clause 86: An end effector for a catheter comprising: a frame comprising extending along a longitudinal axis, the frame having a first side and a second side; a first flexible circuit disposed on the first side of the frame and extending along the longitudinal axis from a proximal portion to a distal portion of the frame; and a second flexible circuit extending along the longitudinal axis from the proximal portion to the distal portion of the frame, the second flexible circuit disposed on the first side of the frame at the distal portion of the frame and disposed on top of the first flexible circuit at the proximal portion of the frame.
Clause 87: The end effector of Clause 86, the first flexible circuit comprises one or more first electrodes disposed on a distal portion of the first flexible circuit, and the second flexible circuit comprises one or more second electrodes disposed on a distal portion of the second flexible circuit.
Clause 88: The end effector of Clause 87, one or more first electrodes and the one or more second electrodes are arranged in pairs of electrodes, each pair of electrodes is spaced apart a first predetermined longitudinal distance and each pair of electrodes is spaced apart from adjacent pairs of first electrodes a second predetermined longitudinal distance, the second predetermined longitudinal distance being greater than the first predetermined longitudinal distance.
Clause 89: The end effector of Clause 88, the first predetermined longitudinal distance is about 100 microns.
Clause 90: The end effector of any one of Clauses 86 to 89, further comprising: a third flexible circuit disposed on the second side of the frame and extending along the longitudinal axis from the proximal portion to the distal portion of the frame; a fourth flexible circuit extending along the longitudinal axis from the proximal portion to the distal portion of the frame, the fourth flexible circuit disposed on the second side of the frame at the distal portion of the frame and disposed on top of the third flexible circuit at the proximal portion of the frame.
Clause 91: The end effector of Clause 90, the third flexible circuit comprises one or more third electrodes disposed on a distal portion of the third flexible circuit, and the fourth flexible circuit comprises one or more fourth electrodes disposed on a distal portion of the second flexible circuit.
Clause 92: An end effector for a catheter comprising: a first flexible circuit extending along a longitudinal axis from a proximal portion to a distal portion of the end effector; and a second flexible circuit extending along the longitudinal axis from the proximal portion to the distal portion of the end effector, the second flexible circuit being substantially coplanar to the first flexible circuit in the distal portion of the end effector and disposed on top of the first flexible circuit in the proximal portion of the end effector.
Clause 93: The end effector of Clause 92, the first flexible circuit including a first set of electrodes disposed on one side of the first flexible circuit and a second set of electrodes on an opposite side of the first flexible circuit, the second flexible circuit including a first set of electrodes disposed on one side of the second flexible circuit and a second set of electrodes disposed on an opposite side of the second flexible circuit.
Clause 94: The end effector of Clause 93, further comprising a substrate disposed between the first and second flexible circuits at the proximal portion and coplanar with the first and second flexible circuits at the distal portion.
Clause 95: An end effector for a catheter, the end effector comprising: an insulative material; a framework disposed in the insulative material, the framework being substantially planar along a longitudinal axis; and location sensing loops spaced apart from the framework and coupled to the insulative material, the location sensing loops comprising: a center loop disposed over the longitudinal axis over an area near a distal portion of the insulative material; and a pair of side loops disposed generally symmetrical about the longitudinal axis, each side loop extending from a proximal portion to a distal portion of the insulative material along the longitudinal axis.
Clause 96: The end effector of Clause 95, the pair of side loops comprising a first side loop and a second side loop.
Clause 97: An end effector for a catheter, the end effector comprising: an insulative material; a framework disposed in the insulative material, the framework being substantially planar along a longitudinal axis; and location sensing loops disposed generally parallel to the framework and separated from the framework by the insulative material, the location sensing loops comprising: a center loop extending along the longitudinal axis and comprising a cumulative center loop surface area; a first side loop extending along the longitudinal axis and comprising a first cumulative side loop surface area; and a second side loop extending along the longitudinal axis and comprising a cumulative second side loop surface area, the cumulative center loop surface area, the cumulative first side loop surface area, and the cumulative second side loop surface area each being within a range of about one-hundred to three-hundred millimeters squared.
Clause 98: The end effector of Clause 97, the center loop comprising one or more center loop coils, each center loop coil defining a center loop surface area, the first side loop comprising one or more first side loop coils, each first side loop coil defining a first side loop surface area, and the second side loop comprising one or more second side loop coils, and each second side loop coil defining a second side loop surface area, wherein the cumulative center loop surface area is a sum of the center loop surface areas of the one or more center loop coils, the cumulative first side loop surface area is a sum of the first side loop surface areas of the one or more first side loop coils, and the cumulative second side loop surface area is a sum of the second side loop surface areas of the one or more second side loop coils.
Clause 99: The end effector of any one of Clauses 95-98, the center loop comprising: a first leg defining a distal end of the center loop; a second leg extending from the first leg in a proximal direction of the end effector; a third leg extending from the second leg in a distal direction of the end effector; a fourth leg extending from the third leg in the proximal direction of the end effector; and a fifth leg extending from the fourth leg in the distal direction of the end effector and connecting with the first leg.
Clause 100: The end effector of Clause 99, the first leg extending arcuately, and the second leg, the third leg, the fourth leg, and the fifth leg extending substantially linearly.
Clause 101: The end effector of Clause 100, the second leg, the third leg, the fourth leg, and the fifth leg being angled relative the longitudinal axis.
Clause 102: The end effector of any one of Clauses 95-101, the center loop comprising a plurality of center loop joint sections.
Clause 103: The end effector of any one of Clauses 96-102, the first side loop comprising: a sixth leg defining a distal end of the first side loop; a seventh leg extending from the sixth leg in a proximal direction of the end effector; an eighth leg extending from the seventh leg in the proximal direction of the end effector; and a ninth leg extending from the eighth leg in a distal direction of the end effector and connecting with the sixth leg.
Clause 104: The end effector of Clause 103, the seventh leg and the ninth leg extending substantially parallel with the longitudinal axis.
Clause 105: The end effector of Clause 103, the ninth leg comprising: a proximal section that is non-parallel with the longitudinal axis; and a distal section that is substantially parallel with the longitudinal axis.
Clause 106: The end effector of Clause 105, the proximal section bowing away from the longitudinal axis.
Clause 107: The end effector of any one of Clauses 103-106, the sixth leg and the eighth leg extending arcuately.
Clause 108: The end effector of any one of Clauses 96-107, the first side loop comprising a plurality of first side loop joint sections.
Clause 109: The end effector of any one of Clauses 96-108, the second side loop comprising: a tenth leg defining a distal end of the second side loop; an eleventh leg extending from the tenth leg in a proximal direction of the end effector; a twelfth leg extending from the eleventh leg in the proximal direction of the end effector; and a thirteenth leg extending from the twelfth leg in a distal direction of the end effector and connecting with the tenth leg.
Clause 110: The end effector of Clause 109, the eleventh leg and the thirteenth leg extending substantially parallel with the longitudinal axis.
Clause 111: The end effector of Clause 109, the thirteenth leg comprising: a proximal section that is non-parallel with the longitudinal axis; and a distal section that is substantially parallel with the longitudinal axis.
Clause 112: The end effector of Clause 111, the proximal section bowing away from the longitudinal axis.
Clause 113: The end effector of any one of Clauses 109-112, the tenth leg and the third second loop section extending arcuately.
Clause 114: The end effector of any one of Clauses 96-113, the second side loop comprising a plurality of second side loop joint sections.
Clause 115: The end effector of any one of Clauses 95-114, the center loop being symmetrical relative to the longitudinal axis.
Clause 116: The end effector of any one of Clauses 96-115, the first side loop and the second side loop being laterally offset from the longitudinal axis.
Clause 117: The end effector of any one of Clauses 96-116, the second side loop being a mirror image of the first side loop relative to the longitudinal axis.
Clause 118: The end effector of Clause 98, each center loop surface area being at least approximately 69 millimeters squared.
Clause 119: The end effector of Clause 98 or 118, each first side loop surface area being at least approximately 59 millimeters squared.
Clause 120: The end effector of any one of Clauses 98 and 118-119, each second side loop surface area being at least approximately 59 millimeters squared.
Clause 121: The end effector of any one of Clauses 96-120, the center loop, the first side loop, the second side loop, and the framework being stacked along a vertical axis that is substantially orthogonal to the longitudinal axis.
Clause 122: The end effector of any one of Clauses 96-121, the insulative material comprising: a first non-conductive flexible layer, the center loop, the first side loop, and the second side loop being separated from the framework by the first non-conductive flexible layer.
Clause 123: The end effector of any one of Clauses 96-121, the insulative material comprising: a first non-conductive flexible layer; and a second non-conductive flexible layer, the center loop being separated from the framework by the first non-conductive flexible layer, and the first side loop and the second side loop being separated from the framework by the second non-conductive flexible layer.
Clause 124: The end effector of any one of Clauses 96-123, the framework extending within a first plane, the middle loop extending within a second plane, the first side loop extending within a third plane, and the second side loop extending within a fourth plane.
Clause 125: The end effector of Clause 124, first plane being parallel with the second plane, the third plane, and the fourth plane.
Clause 126: The end effector of any one of Clauses 124-125, the third plane and the fourth plane being co-planar.
Clause 127: The end effector of any one of Clauses 124-126, the second plane being co-planar with the third plane and the fourth plane.
Clause 128: The end effector of any one of Clauses 124-126, the second plane being on a first side of the first plane along a vertical axis, and the second plane and the third plane being on a second, opposite side of the first plane along the vertical axis.
Clause 129: The end effector of any one of Clauses 97-128, the end effector having an overall length of approximately 20-25 millimeters and an overall width of approximately 9-12 millimeters.
Clause 130: The end effector of any one of Clauses 95-129, further comprising at least one flexible circuit disposed generally parallel to the framework and separated from the framework by the insulative material, the flexible circuit including a plurality of electrodes disposed on the flexible circuit.
Clause 131: A framework for an end effector of a medical device, the framework comprising: a base configured to connect with an elongated shaft of the medical device, the base extending along a longitudinal axis towards in a distal direction; and a first spine loop extending from the base along the longitudinal axis, the first spine loop comprising: a curvilinear first segment connected to the base and extending therefrom in a distal direction along the longitudinal axis; a second segment having an arcuate configuration connected to the curvilinear first segment and extending therefrom; a third segment connected to the second segment and extending therefrom in a proximal direction along the longitudinal axis; a curvilinear fourth segment connected to the base and extending therefrom in the distal direction along the longitudinal axis; an arcuate fifth segment connected to the fourth segment and extending therefrom; a sixth segment connected to the fifth segment and extending therefrom in the proximal direction along the longitudinal axis; and an arcuate seventh segment connecting the third segment and the sixth segment.
Clause 132: The framework of Clause 131, the arcuate seventh segment being connected to the base.
Clause 133: The framework of Clause 132, the first spine loop defining two finger shaped openings, and the base comprising: a first section that is configured to connect with the elongated shaft; and a second section that extends within the finger shaped opening to connect with the seventh segment.
Clause 134: The framework of Clause 133, the second section of the base defining one or more apertures therein.
Clause 135: The framework of Clause 133, further comprising: a second spine loop connected to the first section of the base and extending therefrom along the longitudinal axis.
Clause 136: The framework of Clause 135, the second spine loop comprising a pair of terminal sections that connect to the first section of the base.
Clause 137: The framework of Clause 135, the second spine loop comprising a pair of terminal sections, one terminal section connecting to the first section of the base and the other terminal section connecting to the first segment of the first spine loop.
Clause 138: The framework of any one of Clauses 135-137, further comprising: a third spine loop connected to the first section of the base and extending therefrom along the longitudinal axis.
Clause 139: The framework of Clause 138, the third spine loop comprising a pair of terminal sections that connect to the first section of the base.
Clause 140: The framework of Clause 138, the third spine loop comprising a pair of terminal sections, one terminal section connecting to the first section of the base and the other terminal section connecting to the fourth segment of the first spine loop.
Clause 141: The framework of any one of Clauses 131-140, the first segment, the second segment, and the third segment being symmetrical with the fourth segment, the fifth segment, and the sixth segment relative to the longitudinal axis.
Clause 142: The framework of any one of Clauses 131-141, the longitudinal axis substantially bisecting the seventh segment.
Clause 143: The framework of any one of Clauses 131-142, the longitudinal axis being coaxial with a longitudinal center of the framework.
Clause 144: The framework of any one of Clauses 131-143, comprising a super elastic material.
Clause 145: A framework for an end effector of a medical device, the framework comprising: a base configured to connect with an elongated shaft of the medical device, the base extending along a longitudinal axis; a first spine loop extending from the base along the longitudinal axis, the first spine loop comprising: a first segment connected to the base and extending therefrom in a distal direction along the longitudinal axis; a second segment connected to the first segment and extending therefrom inwardly towards the longitudinal axis; and a third segment connected to the second segment and extending therefrom in the distal direction along the longitudinal axis; a second spine loop extending from the base along the longitudinal axis, the second spine loop comprising: a first segment connected to the base and extending therefrom in the distal direction along the longitudinal axis; a second segment connected to the first segment and extending therefrom inwardly towards the longitudinal axis; and a third segment connected to the second segment and extending therefrom in the distal direction along the longitudinal axis; a third spine loop connecting the third segment of the first spine loop and the third segment of the second spine loop; and a finger shaped opening defined by the base, the first spine loop, the second spine loop, and the third spine loop.
Clause 146: The framework of Clause 145, the third spine loop comprising an undulating form.
Clause 147: The framework of any one of Clauses 145-146, the third segment of the first spine loop and the third segment of the second spine loop extending approximately parallel to the longitudinal axis.
Clause 148: The framework of any one of Clauses 145-147, the longitudinal axis bisecting the third segment.
Clause 149: The framework of any one of Clauses 145-148, the longitudinal axis being coaxial with a longitudinal center of the framework.
Clause 150: The framework of any one of Clauses 145-149, comprising a super elastic material.
Clause 151: An end effector for a medical device, the end effector comprising: a framework comprising: a base configured to connect with an elongated shaft of the medical device, the base extending along a longitudinal axis; a first spine loop extending from the base along the longitudinal axis; a second spine loop extending from the base along the longitudinal axis; a third spine loop connecting the first spine loop and the second spine loop; and a finger shaped opening defined by the base, the first spine loop, the second spine loop, and the third spine loop; an insulative material disposed on the framework; and a flexible circuit comprising: a first section vertically spaced from the framework along a vertical axis by the insulative material; and a second section offset from the first section along the vertical axis and extending within the finger shaped opening.
Clause 152: The end effector of Clause 151, the first section of the flexible circuit being disposed parallel to a framework plane of the framework.
Clause 153: The end effector of any one of Clauses 151-152, the second section of the flexible circuit extending along the framework plane of the framework.
Clause 154: The end effector of any one of Clauses 152-153, the framework plane extending along a neutral plane of the end effector.
Clause 155: The end effector of any one of Clauses 151-154, the first section of the flexible circuit comprising a peripheral section is disposed directly above the first spine loop, the second spine loop, and the third spine loop.
Clause 156: The end effector of any one of Clauses 151-155, the second section of the flexible circuit comprising a peripheral section that runs along an inner surface of the first spine loop and an inner surface of the second spine loop.
Clause 157: The end effector of any one of Clauses 151-156, further comprising a transition section that connects the first section and the second section, the transition section being disposed proximal a connecting segment of the first spine loop and a connecting segment of the second spine loop along the longitudinal axis.
Clause 158: A framework for an end effector of a medical device, the framework extending along a longitudinal axis that is coaxial with a longitudinal center of the framework and comprising: a base configured to connect with an elongated shaft of the medical device and extending along the longitudinal axis; a first spine loop extending from the base on a first side of the longitudinal axis, the first spine loop comprising: a first distal end connected to the base at a first longitudinal location along the longitudinal axis; and a second distal end connected to the base at a second longitudinal location along the longitudinal axis; and a second spine loop extending from the base on a second side of the longitudinal axis, the second spine loop comprising: a first distal end connected to the base at a third longitudinal location along the longitudinal axis; and a second distal end connected to the base at a fourth longitudinal location along the longitudinal axis, the first longitudinal location, the second longitudinal location, the third longitudinal location, and the fourth longitudinal location being respectively disposed along the longitudinal axis such that the framework is asymmetric relative to the longitudinal axis.
Clause 159: The framework of Clause 158, the first spine loop and the base defining a first finger shaped opening, and the second spine loop and the base defining a second finger shaped opening, the first finger shaped opening having a smaller area than an area of the second finger shaped opening.
Clause 160: The framework of any one of Clauses 158-159, the base being symmetric about the longitudinal axis.
Clause 161: The framework of any one of Clauses 158-160, the base comprising: a first section that is configured to connect with the elongated shaft, the first distal end of the first spine loop and the first distal end of the second spine loop being connected to the first section; and a second section that extends from the first section, the second distal end of the first spine loop and the second distal end of the second spine loop being connected to the second section.
Clause 162: The framework of Clause 161, the second distal end of the first spine loop being connected to a proximal end of the second section, and the second distal end of the second spine loop being connected to a distal end of the second section.
Clause 163: The framework of Clause 161, the second distal end of the first spine loop being connected to a distal end of the second section, and the second distal end of the second spine loop being connected to the distal end of the second section.
Clause 164: The framework of any one of Clauses 158-162, the first longitudinal location and the third longitudinal location being the same.
Clause 165: The framework of any one of Clauses 158-162 and 164, the second longitudinal location and the fourth longitudinal location being spaced by a predetermined distance along the longitudinal axis.
Clause 166: The framework of any one of Clauses 158-161 and 163, the first longitudinal location and the third longitudinal location being spaced by a predetermined distance along the longitudinal axis.
Clause 167: The framework of any one of Clauses 158-161, 163, and 166, the second longitudinal location and the fourth longitudinal location being the same.
Clause 168: The framework of any one of Clauses 158-167, the base, the first spine loop, and the second spine loop being a monolithic member.
Clause 169: An end effector for a medical device, the end effector comprising: a framework that is substantially planar along a longitudinal axis; a flexible circuit vertically spaced from the framework along a vertical axis; and an insulative material within which the framework and the flexible circuit are disposed, the insulative material defining a first outer edge of the end effector, the first outer edge comprising a treatment configured to facilitate collapsing of the end effector into a sheath, the treatment comprising at least one of: a rounded cut extending along at least a portion of the first outer edge; a tapered cut extending along at least a portion of the first outer edge; a plurality of incised cuts defined along at least a portion of the first outer edge; or a lubricious coating extending along at least a portion of the first outer edge.
Clause 170: The end effector of Clause 169, the treatment comprising the rounded cut, and the insulative material further comprising a second outer edge comprising a second outer edge treatment configured to facilitate collapsing of the end effector into a sheath, the second outer edge treatment comprising at least one of: a rounded cut extending along at least a portion of the second outer edge; a tapered cut extending along at least a portion of the second outer edge; a plurality of incised cuts defined along at least a portion of the second outer edge; or a lubricious coating extending along at least a portion of the second outer edge.
Clause 171: The end effector of Clause 169, the treatment comprising the tapered cut, and the insulative material further comprising a second outer edge comprising a second outer edge treatment configured to facilitate collapsing of the end effector into a sheath, the second outer edge treatment comprising at least one of: a rounded cut extending along at least a portion of the second outer edge; a tapered cut extending along at least a portion of the second outer edge; a plurality of incised cuts defined along at least a portion of the second outer edge; or a lubricious coating extending along at least a portion of the second outer edge.
Clause 172: The end effector of any one of Clauses 169-171, the treatment comprising the plurality of incised cuts.
Clause 173: The end effector of any one of Clauses 169-172, the treatment comprising the lubricious coating.
Clause 174: A method of using a medical device, comprising: retracting, from a deployed configuration, an end effector into a sheath along a longitudinal axis, the end effector comprising a substantially planar shape in the deployed configuration; and collapsing the end effector into a retracted configuration such that outer edges of the end effector slip past one another, and the end effector comprises one of a substantially cylindrical shape or a spiral shape in the retracted configuration, with a first outer edge of the outer edges comprising a treatment comprising at least one of: a rounded cut extending along at least a portion of the first outer edge; a tapered cut extending along at least a portion of the first outer edge; a plurality of incised cuts defined along at least a portion of the first outer edge; or a lubricious coating extending along at least a portion of the first outer edge.
Clause 175: A method of manufacturing an end effector for a medical device, the method comprising: forming a framework that is substantially planar along a longitudinal axis; disposing a flexible circuit over the framework; heating an insulative material; reflowing the insulative material such that the insulative material envelopes the framework and the flexible circuit; and treating an outer edge of the insulative material such that the outer edge comprises at least one of a reduced stiffness or a reduced coefficient of friction relative to a coefficient of friction or a stiffness of the outer edge prior to the treatment.
Clause 176: An end effector comprising a plurality of planar framework extending along a plane, each of the framework having a first planar surface and an opposite second planar surface, each of the first and second planar surfaces includes at least one electrode pair, the at least one electrode pair being configured to contact cardiovascular tissue, each electrode pair including first and second electrodes spaced apart along a longitudinal axis from each other by a gap area located between the electrodes, the gap area having a gap distance with respect to the longitudinal axis such that: (i) a length of one of the electrodes along the longitudinal axis is equal to or greater than the gap distance; and (ii) a ratio of an area defined by the gap area to one electrode area is equal to or less than one, the gap distance being determined by a product of the one electrode area up to one millimeter squared and a conversion factor of about 1.25 mm⁻¹ or less.
Clause 177. An end effector, the end effector comprising: a framework defining a neutral plane; a first insulative material disposed on at least one side of the framework; and a first flexible circuit disposed in the first insulative material and spaced apart from the framework along a vertical axis orthogonal to the neutral plane, the first flexible circuit comprising a planar transition zone located proximate a proximal end of the first flexible circuit, the first flexible circuit transitioning in the planar transition zone from a first plane closer to the neutral plane to a second plane farther from the neutral plane.
Clause 178: The end effector of Clause 177, the first flexible circuit further comprises a first plurality of electrodes being positioned on a portion of the first flexible circuit that is contiguous to the second plane and distal to the planar transition zone.
Clause 179: The end effector of Clause 177 or 178, the proximal end of the first flexible circuit comprises a tail extending proximally from the planar transition zone, the tail transitioning within the planar transition zone from the first plane to a proximal plane farther from the neutral plane.
Clause 180: The end effector of Clause 179, the proximal plane and the second plane are coplanar.
Clause 181: The end effector of any one of Clauses 178 to 180, at least a portion of the first plurality of electrodes extending from a surface defined by the first insulative material.
Clause 182: The end effector of any one of Clauses 177-181, further comprising: a second insulative material disposed on at least one side of the framework; and a second flexible circuit disposed in the second insulative material, the second flexible circuit spaced apart from the framework along a vertical axis and transitioning within the planar transition zone from a third plane closer to the neutral plane to a fourth plane farther from the neutral plane.
Clause 183: The end effector of Clause 182, the second flexible circuit comprising a second plurality of electrodes.
Clause 184: The end effector of Clause 183, at least a portion of the second plurality of electrodes extending from a surface defined by the second insulative material.
Clause 185: The end effector of Clause 183 or 184, the second plurality of electrodes being positioned on a portion of the second flexible circuit that is within the fourth plane and distal to the planar transition zone.
Clause 186: The end effector of Clause 182, the second flexible circuit further comprising an electromagnetic planar coil.
Clause 187: The end effector of Clause 182, the second insulative material is disposed on an opposite side of the framework from the first insulative material.
Clause 188: The end effector of Clause 187, the electromagnetic coil of the second flexible circuit comprising a distal loop and two side loops positioned proximate a surface defined by the second insulative material.
Clause 189: The end effector of Clause 187, the proximal end of the second flexible circuit comprising a tail extending proximally from the planar transition zone and away from the electromagnetic coil, the tail transitioning within the planar transition zone from the third plane to a proximal plane farther from the neutral plane.
Clause 190: The end effector of Clause 189, the proximal plane and the fourth plane are coplanar.
Clause 191: The end effector of any of the preceding clauses that depend from Clause 178, two adjacent electrodes of the first plurality of electrodes being connected by a serpentine connection.
Clause 192: The end effector of Clause 191, the serpentine connection extending entirely within the second plane.
Clause 193: The end effector of Clause 178, the first insulative material comprises: a distal insulative material sheet positioned between the framework and the first plurality of electrodes; and an intermediate insulative material sheet positioned within the planar transition zone such that at least a portion of the first flexible circuit is positioned between the intermediate insulative material sheet and the framework.
Clause 194: The end effector of Clause 193, the proximal end of the first flexible circuit comprises a tail extending proximally from the planar transition zone, and the first insulative material comprises a proximal insulative material sheet positioned between the framework and the tail.
Clause 195: The end effector of Clause 193 or 194 further comprising: a second insulative material disposed on at least one side of the framework; and a second flexible circuit comprising a second plurality of electrodes and disposed in the second insulative material, the second flexible circuit transitioning within the planar transition zone from a third plane closer to the neutral plane to a fourth plane farther from the neutral plane.
Clause 196: The end effector of Clause 195, the second insulative material comprises: a second distal insulative material sheet positioned between the framework and the second plurality of electrodes; and a second intermediate insulative material sheet positioned within the planar transition zone such that at least a portion of the first flexible circuit is positioned between the second intermediate insulative material sheet and the framework.
Clause 197: The end effector of Clause 196, a proximal end of the second flexible circuit comprises a second tail extending proximally from the planar transition zone and away from the second plurality of electrodes, and the second insulative material comprises a second proximal insulative material sheet positioned between the framework and the second tail.
Clause 198: The end effector of Clause 195, the second insulative material is disposed on an opposite side of the framework from the first insulative material.
Clause 199: The end effector of any one of Clauses 177-198, in which the first and second insulative material comprises a single insulative material.
Clause 200: The end effector of any one of Clauses 178 to 180, at least a portion of the first plurality of electrodes is recessed into a surface defined by the first insulative material.
Clause 201: An end effector comprising: a framework comprising a first strut extending along a longitudinal axis; and a flexible circuit aligned with the first strut and comprising a substrate supporting an electrical trace, the flexible circuit comprising a plurality of bends such that the flexible circuit comprises a serpentine shape as it extends along the longitudinal axis of the strut, the first strut of the framework comprising alternating wide sections and narrow sections extending along the longitudinal axis of the strut, the narrow sections being positioned proximate respective apexes of the plurality of bends.
Clause 202: The end effector of Clause 201, the narrow sections defining cutouts, and the apexes of the plurality of bends extending into the cutouts such that they overhang at least a portion of the first strut.
Clause 203: The end effector of Clause 201 or 202, the first strut comprising a first plurality of electrodes positioned between separated sections of the plurality of bends.
Clause 204: The end effector of any of the preceding Clauses, the electrical trace comprising rolled annealed metal.
Clause 205: The end effector of Clause 205, the rolled annealed metal being rolled annealed copper.
Clause 206: The end effector of Clauses 204 or 205, the serpentine shape being formed from the rolled annealed metal along a direction of individual grains of the rolled annealed metal such that the individual grains run parallel to the longitudinal axis of the strut.
Clause 207: The end effector of and one of Clauses 204 to 206, the electrical trace having alternating longer grain sections and shorter grain sections.
Clause 208: The end effector of Clause 207, the longer grain sections being positioned at a location of the plurality of bends.
Clause 209: The end effector of Clause 208, the electrical trace transitioning to the shorter grain sections intermediate to the plurality of bends.
Clause 210: The end effector of any one of Clauses 201-209, the first strut being one of a plurality of struts on the framework, the flexible circuit comprising electrical traces extending along each of the plurality of struts, and each of the electrical traces comprising the plurality of bends in the flexible circuit.
Clause 211: The end effector of Clause 210, each of the plurality of struts comprising the alternating wide sections and narrow sections extending along their respective longitudinal axes.
Clause 212: The end effector of any one of Clauses 201-211, in which the framework and the flexible circuit are spaced apart along a vertical axis on different planes.
Clause 213: An end effector comprising: a framework comprising a first strut extending along a longitudinal axis; and a flexible circuit aligned with the first strut and comprising a substrate supporting an electrical trace, the flexible circuit comprising a plurality of bends such that the flexible circuit has a serpentine shape as it extends along the longitudinal axis of the strut, the electrical trace comprises a rolled annealed metal, the serpentine shape being formed from the rolled annealed metal along a direction of individual grains of the rolled annealed metal such that the individual grains run parallel to the longitudinal axis of the strut.
Clause 214: The end effector of Clause 213, the rolled annealed metal being rolled annealed copper.
Clause 215: The end effector of Clause 213, the electrical trace comprising longer grain sections and shorter grain sections.
Clause 216: The end effector of Clause 215, the longer grain sections being positioned at a location of the plurality of bends.
Clause 217: The end effector of Clause 214, the electrical trace transitioning to the shorter grain sections intermediate to the plurality of bends.
Clause 218: The end effector of any one of Clauses 213 to 217, the first strut of the framework comprising alternating wide sections and narrow sections extending along the longitudinal axis of the strut, the narrow sections positioned proximate apexes of the plurality of bends.
Clause 219: The end effector of Clause 218, the narrow sections defining cutouts, and the apexes of the plurality of bends extend into the cutouts such that they overhang at least a portion of the first strut.
Clause 220: The end effector of Clause 218, the first strut being one of a plurality of struts on the framework, the flexible circuit comprising electrical traces extending along each of the plurality of struts, and each of the electrical traces extending along the plurality of struts comprising the plurality of bends.
Clause 221: The end effector of Clause 220, each of the plurality of struts comprising the alternating wide sections and narrow sections extending along their respective longitudinal axes.
Clause 222: The end effector of any one of Clauses 213 to 221, the first strut comprising a first plurality of electrodes positioned between separated sections of the plurality of bends.
Clause 223: The end effector of any one of Clauses 213 to 222, in which the framework and the flexible circuit are spaced apart along a vertical axis on different planes.
Clause 224: A method of manufacturing an end effector for a medical catheter, the method comprising: forming a strut for a framework of the end effector and providing alternating wide sections and narrow sections extending along a longitudinal axis of the strut, disposing an electrical trace on a substrate; forming a plurality of bends in the substrate and the electrical trace; and aligning the substrate and the electrical trace with the strut such that apexes of the plurality of bends are positioned at corresponding locations of the narrow sections of the strut.
Clause 225: The method of Clause 224, the electrical trace comprising a rolled annealed metal.
Clause 226: The method of Clause 225, the rolled annealed metal being rolled annealed copper.
Clause 227: The method of Clause 225 or 226 further comprising forming a serpentine shape from the rolled annealed metal by cutting the electrical trace along a direction of individual grains of the rolled annealed metal such that the individual grains run parallel to the longitudinal axis of the strut.
Clause 228: The method of Clause 227, the electrical trace having longer grain sections and shorter grain sections.
Clause 229: The method of Clause 228, the longer grain sections being positioned at a location of the plurality of bends.
Clause 230: The method of any one of Clauses 224 to 229, the narrow sections defining cutouts, and the apexes of the plurality of bends extend into the cutouts such that they overhang at least a portion of the first strut.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. An end effector for a catheter, the end effector comprising:
an insulative material;
a framework disposed in the insulative material, the framework being substantially planar along a longitudinal axis and having a first planar surface and a second planar surface opposite the first planar surface;
a plurality of generally planar electrodes aligned along each of the first planar surface and second planar surface such that each electrode is spaced apart from the first planar surface or the second planar surface; and
location sensing loops spaced apart from the framework and coupled to the insulative material, the location sensing loops comprising:
a center loop disposed over the longitudinal axis over an area near a distal portion of the insulative material; and
a pair of side loops disposed generally symmetrical about the longitudinal axis, each side loop extending from a proximal portion to a distal portion of the insulative material along the longitudinal axis.

2. The end effector of claim 1, the pair of side loops comprising a first side loop and a second side loop.

3. The end effector of claim 2, the first side loop comprising:
a sixth leg defining a distal end of the first side loop;
a seventh leg extending from the sixth leg in a proximal direction of the end effector;
an eighth leg extending from the seventh leg in the proximal direction of the end effector; and
a ninth leg extending from the eighth leg in a distal direction of the end effector and connecting with the sixth leg.

4. The end effector of claim 2, the first side loop comprising a plurality of first side loop joint sections.

5. The end effector of claim 2, the second side loop comprising:
a tenth leg defining a distal end of the second side loop;
an eleventh leg extending from the tenth leg in a proximal direction of the end effector;
a twelfth leg extending from the eleventh leg in the proximal direction of the end effector; and
a thirteenth leg extending from the twelfth leg in a distal direction of the end effector and connecting with the tenth leg.

6. The end effector of claim 2, the second side loop comprising a plurality of second side loop joint sections.

7. The end effector of claim 2, the first side loop and the second side loop being laterally offset from the longitudinal axis.

8. The end effector of claim 2, the second side loop being a mirror image of the first side loop relative to the longitudinal axis.

9. The end effector of claim 2, the center loop, the first side loop, the second side loop, and the framework being stacked along a vertical axis that is substantially orthogonal to the longitudinal axis.

10. The end effector of claim 2, the insulative material comprising:
a first non-conductive flexible layer,
the center loop, the first side loop, and the second side loop being separated from the framework by the first non-conductive flexible layer.

11. The end effector of claim 2, the insulative material comprising:
a first non-conductive flexible layer; and
a second non-conductive flexible layer,
the center loop being separated from the framework by the first non-conductive flexible layer, and the first side loop and the second side loop being separated from the framework by the second non-conductive flexible layer.

12. The end effector of claim 2, the framework extending in a first plane, the middle loop extending within a second plane, the first side loop extending in a third plane, and the second side loop extending within a fourth plane.

13. The end effector of claim 12, first plane being parallel with the second plane, the third plane, and the fourth plane.

14. The end effector of claim 12, the second plane being on a first side of the first plane along a vertical axis, and the second plane and the third plane being on a second, opposite side of the first plane along the vertical axis.

15. The end effector of claim 1, the center loop being symmetrical relative to the longitudinal axis.

16. The end effector of claim 1, the center loop comprising:
a first leg defining a distal end of the center loop;
a second leg extending from the first leg in a proximal direction of the end effector;
a third leg extending from the second leg in a distal direction of the end effector;
a fourth leg extending from the third leg in the proximal direction of the end effector; and
a fifth leg extending from the fourth leg in the distal direction of the end effector and connecting with the first leg.

17. The end effector of claim 1, the insulative material defining a first outer edge of the end effector, the first outer edge comprising a treatment configured to facilitate collapsing of the end effector into a sheath, the treatment comprising at least one of: a rounded cut extending along at least a portion of the first outer edge; a tapered cut extending along at least a portion of the first outer edge; a plurality of incised cuts defined along at least a portion of the first outer edge; or a lubricious coating extending along at least a portion of the first outer edge.

18. The end effector of claim 1, further comprising at least one flexible circuit disposed generally parallel to the framework and separated from the framework by the insulative material, the flexible circuit including a plurality of electrodes disposed on the flexible circuit.

19. An end effector for a catheter, the end effector comprising:
an insulative material;
a framework disposed in the insulative material, the framework being substantially planar along a longitudinal axis; and
location sensing loops disposed generally parallel to the framework and separated from the framework by the insulative material, the location sensing loops comprising:
a center loop extending along the longitudinal axis and comprising a cumulative center loop surface area;
a first side loop extending along the longitudinal axis and comprising a first cumulative side loop surface area; and
a second side loop extending along the longitudinal axis and comprising a cumulative second side loop surface area,
the cumulative center loop surface area, the cumulative first side loop surface area, and the cumulative second side loop surface area each being within a range of about one-hundred to about three-hundred millimeters squared.

20. The end effector of claim 19, the center loop comprising one or more center loop coils, each center loop coil defining a center loop surface area, the first side loop comprising one or more first side loop coils, each first side loop coil defining a first side loop surface area, and the second side loop comprising one or more second side loop coils, and each second side loop coil defining a second side loop surface area, wherein
the cumulative center loop surface area comprises a sum of the center loop surface areas of the one or more center loop coils,
the cumulative first side loop surface area comprises a sum of the first side loop surface areas of the one or more first side loop coils, and
the cumulative second side loop surface area comprises a sum of the second side loop surface areas of the one or more second side loop coils.
